# EUROPEAN PATENT SPECIFICATION

(11) **EP 1 002 091 B1**
(45) Date of publication and mention of the grant of the patent: **29.02.2012**
(21) Application number: 98931833.2
(22) Date of filing: 09.07.1998
(51) Int. Cl.: C12N 15/37, C07K 14/025, C12N 5/10, A61K 39/12

(54) **NUCLEIC ACID SEQUENCE AND METHOD FOR SELECTIVELY EXPRESSING A PROTEIN IN A TARGET CELL OR TISSUE**
NUKLEINSÄURESEQUENZ UND VERFAHREN ZUR SELEKTIVEN EXPRESSION EINES PROTEINS IN EINER ZIELZELLE ODER -GEWEBE
SEQUENCE D'ACIDES NUCLEIQUES ET PROCEDE POUR EXPRIMER, DE MANIERE SELECTIVE, UNE PROTEINE DANS UNE CELLULE OU UN TISSU CIBLE

(30) Priority: 09.07.1997 AU PO776597; 11.09.1997 AU PO946797
(43) Date of publication of application: 24.05.2000
(73) Proprietor: Coridon Pty Limited, Brisbane, QLD 4000 (AU)
(72) Inventor: FRAZER, Ian, St. Lucia, QLD 4067 (AU); ZHOU, Jian, Jindalee, QLD 4074 (AU)
(74) Representative: Williams, Aylsa
(86) International application number: PCT/AU1998/000530
(87) International publication number: WO 1999/002694

(56) References cited:
- WO-A-96/09378
- AU-A- 1 750 297
- AU-A- 3 509 995
- AU-A- 4 355 697
- AU-A- 7 446 796
- IE-A- 904 161
- HUA ZICHUN ET AL: "Enhancement of expression of human granulocyte-macrophage colony stimulating factor by argU gene product in Escherichia coli" BIOCHEMISTRY AND MOLECULAR BIOLOGY INTERNATIONAL, vol. 32, no. 3, 1994, pages 537-543, XP008033940
- KELLY S J ET AL: "EFFECT OF TRANSFER RNA FROM VARIOUS SOURCES ON PLACENTAL MESSENGER RNA TRANSLATION" MOLECULAR AND CELLULAR ENDOCRINOLOGY, vol. 29, no. 2, 1983, pages 181-195, XP002292169 ISSN: 0303-7207
- ZHOU JIAN ET AL: "Papillomavirus capsid protein expression level depends on the match between codon usage and tRNA availability" JOURNAL OF VIROLOGY, THE AMERICAN SOCIETY FOR MICROBIOLOGY, US, vol. 73, no. 6, June 1999 (1999-06), pages 4972-4982, XP002164427 ISSN: 0022-538X
- J. VIROL., Vol. 70, (1996), ZOLOTUKHIN et al., "A Humanized Green Fluorescent Protein cDNA Adapted for High Level Expression in Mammalian Cells", pp. 4646-4654. XP002030427

### SEQUENCE LISTING

<110> The University of Queensland
<120> NUCLEIC ACID SEQUENCE AND METHOD FOR SELECTIVELY EXPRESSING A PROTEIN IN A TARGET CELL OR TISSUE
<130> Selective Expression
<140> PCT/AU98/00530
   <141> 1998-07-09
<150> PO7765
   <151> 1997-07-09
<150> PO9467
   <151> 1997-09-11
<160> 34
<170> PatentIn Ver. 2.0
<210> 1
   <211> 1488
   <212> DNA
   <213> Bovine papillomavirus type 1
<220>
   <221> CDS
   <222> (1) .. (1488)
<220>
   <223> L1 open reading frame (wild-type)
<400> 1
   atg gcg ttg tgg caa caa ggc cag aag ctg tat ctc cct cca acc cct    48
   Met Ala Leu Trp Gln Gln Gly Gln Lys Leu Tyr Leu Pro Pro Thr Pro
    1               5                  10                  15
   gta agc aag gtg ctt tgc agt gaa acc tat gtg caa aga aaa agc att    96
   Val Ser Lys Val Leu Cys Ser Glu Thr Tyr Val Gln Arg Lys Ser Ile
               20                  25                  30
   ttt tat cat gca gaa acg gag cgc ctg cta act ata gga cat cca tat    144
   Phe Tyr His Ala Glu Thr Glu Arg Leu Leu Thr Ile Gly His Pro Tyr
           35                  40                  45
   tac cca gtg tct atc ggg gcc aaa act gtt cct aag gtc tct gca aat    192
   Tyr Pro Val Ser Ile Gly Ala Lys Thr Val Pro Lys Val Ser Ala Asn
       50                  55                  60
   cag tat agg gta ttt aaa ata caa cta cct gat ccc aat caa ttt gca    240
   Gln Tyr Arg Val Phe Lys Ile Gln Leu Pro Asp Pro Asn Gln Phe Ala
   65                  70                  75                  80
   cta cct gac agg act gtt cac aac cca agt aaa gag cgg ctg gtg tgg 288
   Leu Pro Asp Arg Thr Val His Asn Pro Ser Lys Glu Arg Leu Val Trp
   85 90 95
   cca gtc ata ggt gtg cag gtg tcc aga ggg cag cct ctt gga ggt act 336
   Pro Val Ile Gly Val Gln Val Ser Arg Gly Gln Pro Leu Gly Gly Thr
   100 105 110
   gta act ggg cac ccc act ttt aat gct ttg ctt gat gca gaa aat gtg 384
   Val Thr Gly His Pro Thr Phe Asn Ala Leu Leu Asp Ala Glu Asn Val
   115 120 125
   aat aga aaa gtc acc acc caa aca aca gat gac agg aaa caa aca ggc 432
   Asn Arg Lys Val Thr Thr Gln Thr Thr Asp Asp Arg Lys Gln Thr Gly
   130 135 140
   cta gat gct aag caa caa cag att ctg ttg cta ggc tgt acc cct gct 480
   Leu Asp Ala Lys Gln Gln Gln Ile Leu Leu Leu Gly Cys Thr Pro Ala
   145 150 155 160
   gaa ggg gaa tat tgg aca aca gcc cgt cca tgt gtt act gat cgt cta 528
   Glu Gly Glu Tyr Trp Thr Thr Ala Arg Pro Cys Val Thr Asp Arg Leu
   165 170 175
   gaa aat ggc gcc tgc cct cct ctt gaa tta aaa aac aag cac ata gaa 576
   Glu Asn Gly Ala Cys Pro Pro Leu Glu Leu Lys Asn Lys His Ile Glu
   180 185 190
   gat ggg gat atg atg gaa att ggg ttt ggt gca gcc aac ttc aaa gaa 624
   Asp Gly Asp Met Met Glu Ile Gly Phe Gly Ala Ala Asn Phe Lys Glu
   195 200 205
   att aat gca agt aaa tca gat cta cct ctt gac att caa aat gag atc 672
   Ile Asn Ala Ser Lys Ser Asp Leu Pro Leu Asp Ile Gln Asn Glu Ile
   210 215 220
   tgc ttg tac cca gac tac ctc aaa atg gct gag gac gct gct ggt aat 720
   Cys Leu Tyr Pro Asp Tyr Leu Lys Met Ala Glu Asp Ala Ala Gly Asn
   225 230 235 240
   agc atg ttc ttt ttt gca agg aaa gaa cag gtg tat gtt aga cac atc 768
   Ser Met Phe Phe Phe Ala Arg Lys Glu Gln Val Tyr Val Arg His Ile
   245 250 255
   tgg acc aga ggg ggc tcg gag aaa gaa gcc cct acc aca gat ttt tat 816
   Trp Thr Arg Gly Gly Ser Glu Lys Glu Ala Pro Thr Thr Asp Phe Tyr
   260 265 270
   tta aag aat aat aaa ggg gat gcc acc ctt aaa ata ccc agt gtg cat 864
   Leu Lys Asn Asn Lys Gly Asp Ala Thr Leu Lys Ile Pro Ser Val His
   275 280 285
   ttt ggt agt ccc agt ggc tca cta gtc tca act gat aat caa att ttt 912
   Phe Gly Ser Pro Ser Gly Ser Leu Val Ser Thr Asp Asn Gln Ile Phe
   290 295 300
   aat cgg ccc tac tgg cta ttc cgt gcc cag ggc atg aac aat gga att    960
   Asn Arg Pro Tyr Trp Leu Phe Arg Ala Gln Gly Met Asn Asn Gly Ile
   305                 310                 315                 320
   gca tgg aat aat tta ttg ttt tta aca gtg ggg gac aat aca cgt ggt    1008
   Ala Trp Asn Asn Leu Leu Phe Leu Thr Val Gly Asp Asn Thr Arg Gly
                   325                 330                 335
   act aat ctt acc ata agt gta gcc tca gat gga acc cca cta aca gag    1056
   Thr Asn Leu Thr Ile Ser Val Ala Ser Asp Gly Thr Pro Leu Thr Glu
               340                 345                 350
   tat gat agc tca aaa ttc aat gta tac cat aga cat atg gaa gaa tat    1104
   Tyr Asp Ser Ser Lys Phe Asn Val Tyr His Arg His Met Glu Glu Tyr
           355                 360                 365
   aag cta gcc ttt ata tta gag cta tgc tct gtg gaa atc aca gct caa    1152
   Lys Leu Ala Phe Ile Leu Glu Leu Cys Ser Val Glu Ile Thr Ala Gln
       370                 375                 380
   act gtg tca cat ctg caa gga ctt atg ccc tct gtg ctt gaa aat tgg    1200
   Thr Val Ser His Leu Gln Gly Leu Met Pro Ser Val Leu Glu Asn Trp
   385                 390                 395                 400
   gaa ata ggt gtg cag cct cct acc tca tcg ata tta gag gac acc tat    1248
   Glu Ile Gly Val Gln Pro Pro Thr Ser Ser Ile Leu Glu Asp Thr Tyr
                   405                 410                 415
   cgc tat ata gag tct cct gca act aaa tgt gca agc aat gta att cct    1296
   Arg Tyr Ile Glu Ser Pro Ala Thr Lys Cys Ala Ser Asn Val Ile Pro
               420                 425                 430
   gca aaa gaa gac cct tat gca ggg ttt aag ttt tgg aac ata gat ctt    1344
   Ala Lys Glu Asp Pro Tyr Ala Gly Phe Lys Phe Trp Asn Ile Asp Leu
           435                 440                 445
   aaa gaa aag ctt tct ttg gac tta gat caa ttt ccc ttg gga aga aga    1392
   Lys Glu Lys Leu Ser Leu Asp Leu Asp Gln Phe Pro Leu Gly Arg Arg
       450                 455                 460
   ttt tta gca cag caa ggg gca gga tgt tca act gtg aga aaa cga aga    1440
   Phe Leu Ala Gln Gln Gly Ala Gly Cys Ser Thr Val Arg Lys Arg Arg
   465                 470                 475                 480
   att agc caa aaa act tcc agt aag cct gca aaa aaa aaa aaa aaa taa    1488
   Ile Ser Gln Lys Thr Ser Ser Lys Pro Ala Lys Lys Lys Lys Lys
                   485                 490                 495
<210> 2
   <211> 495
   <212> PRT
   <213> Bovine papillomavirus type 1
<400> 2
   Met Ala Leu Trp Gln Gln Gly Gln Lys Leu Tyr Leu Pro Pro Thr Pro
   1               5                   10                  15
   Val Ser Lys Val Leu Cys Ser Glu Thr Tyr Val Gln Arg Lys Ser Ile
               20                  25                  30
   Phe Tyr His Ala Glu Thr Glu Arg Leu Leu Thr Ile Gly His Pro Tyr
           35                  40                  45
   Tyr Pro Val Ser Ile Gly Ala Lys Thr Val Pro Lys Val Ser Ala Asn
       50                  55                  60
   Gln Tyr Arg Val Phe Lys Ile Gln Leu Pro Asp Pro Asn Gln Phe Ala
   65                  70                  75                  80
   Leu Pro Asp Arg Thr Val His Asn Pro Ser Lys Glu Arg Leu Val Trp
                   85                  90                  95
   Pro Val Ile Gly Val Gln Val Ser Arg Gly Gln Pro Leu Gly Gly Thr
               100                 105                 110
   Val Thr Gly His Pro Thr Phe Asn Ala Leu Leu Asp Ala Glu Asn Val
           115                 120                 125
   Asn Arg Lys Val Thr Thr Gln Thr Thr Asp Asp Arg Lys Gln Thr Gly
       130                 135                 140
   Leu Asp Ala Lys Gln Gln Gln Ile Leu Leu Leu Gly Cys Thr Pro Ala
   145                 150                 155                 160
   Glu Gly Glu Tyr Trp Thr Thr Ala Arg Pro Cys Val Thr Asp Arg Leu
                   165                 170                 175
   Glu Asn Gly Ala Cys Pro Pro Leu Glu Leu Lys Asn Lys His Ile Glu
               180                 185                 190
   Asp Gly Asp Met Met Glu Ile Gly Phe Gly Ala Ala Asn Phe Lys Glu
           195                 200                 205
   Ile Asn Ala Ser Lys Ser Asp Leu Pro Leu Asp Ile Gln Asn Glu Ile
       210                 215                 220
   Cys Leu Tyr Pro Asp Tyr Leu Lys Met Ala Glu Asp Ala Ala Gly Asn
   225                 230                 235                 240
   Ser Met Phe Phe Phe Ala Arg Lys Glu Gln Val Tyr Val Arg His Ile
                   245                 250                 255
   Trp Thr Arg Gly Gly Ser Glu Lys Glu Ala Pro Thr Thr Asp Phe Tyr
               260                 265                 270
   Leu Lys Asn Asn Lys Gly Asp Ala Thr Leu Lys Ile Pro Ser Val His
           275                 280                 285
   Phe Gly Ser Pro Ser Gly Ser Leu Val Ser Thr Asp Asn Gln Ile Phe
       290                 295                 300
   Asn Arg Pro Tyr Trp Leu Phe Arg Ala Gln Gly Met Asn Asn Gly Ile
   305                 310                 315                 320
   Ala Trp Asn Asn Leu Leu Phe Leu Thr Val Gly Asp Asn Thr Arg Gly
                   325                 330                 335
   Thr Asn Leu Thr Ile Ser Val Ala Ser Asp Gly Thr Pro Leu Thr Glu
               340                 345                 350
   Tyr Asp Ser Ser Lys Phe Asn Val Tyr His Arg His Met Glu Glu Tyr
           355                 360                 365
   Lys Leu Ala Phe Ile Leu Glu Leu Cys Ser Val Glu Ile Thr Ala Gln
       370                 375                 380
   Thr Val Ser His Leu Gln Gly Leu Met Pro Ser Val Leu Glu Asn Trp
   385                 390                 395                 400
   Glu Ile Gly Val Gln Pro Pro Thr Ser Ser Ile Leu Glu Asp Thr Tyr
                   405                 410                 415
   Arg Tyr Ile Glu Ser Pro Ala Thr Lys Cys Ala Ser Asn Val Ile Pro
               420                 425                 430
   Ala Lys Glu Asp Pro Tyr Ala Gly Phe Lys Phe Trp Asn Ile Asp Leu
           435                 440                 445
   Lys Glu Lys Leu Ser Leu Asp Leu Asp Gln Phe Pro Leu Gly Arg Arg
       450                 455                 460
   Phe Leu Ala Gln Gln Gly Ala Gly Cys Ser Thr Val Arg Lys Arg Arg
   465                 470                 475                 480
   Ile Ser Gln Lys Thr Ser Ser Lys Pro Ala Lys Lys Lys Lys Lys
                   485                 490                 495
<210> 3
   <211> 1488
   <212> DNA
   <213> Artificial Sequence
<220>
   <221> CDS
   <222> (1) .. (1488)
<220>
   <223> Description of Artificial Sequence: Bovine papillomavirus type 1 L1 open reading frame (humanized)
<220>
   <223> Wild-type codons replaced with synonymous codons used at relatively high frequency by human genes
<400> 3
   atg gcc ctg tgg cag cag ggc cag aag ctg tac ctg ccc cct acc ccc     48
   Met Ala Leu Trp Gln Gln Gly Gln Lys Leu Tyr Leu Pro Pro Thr Pro
     1               5                  10                  15
   gtg agc aag gtg ctt tgc agt gaa acc tat gtg caa aga aaa agc att    96
   Val Ser Lys Val Leu Cys Ser Glu Thr Tyr Val Gln Arg Lys Ser Ile
               20                  25                  30
   ttt tat cat gca gaa acg gag cgc ctg ctg acc atc gga cac ccc tat    144
   Phe Tyr His Ala Glu Thr Glu Arg Leu Leu Thr Ile Gly His Pro Tyr
           35                  40                  45
   tac ccc gtg tcc atc ggg gcc aag act gtg cct aag gtg tcc gcc aat    192
   Tyr Pro Val Ser Ile Gly Ala Lys Thr Val Pro Lys Val Ser Ala Asn
       50                  55                  60
   cag tat agg gtg ttc aaa atc caa ctg cct gat ccc aat caa ttt gca    240
   Gln Tyr Arg Val Phe Lys Ile Gln Leu Pro Asp Pro Asn Gln Phe Ala
   65                  70                  75                  80
   ctg cct gac agg acc gtg cac aac ccc agc aaa gag cgg ctg gtg tgg    288
   Leu Pro Asp Arg Thr Val His Asn Pro Ser Lys Glu Arg Leu Val Trp
                   85                  90                  95
   cca gtg atc ggc gtg cag gtg tcc aga ggc cag cct ctg ggc ggc acc    336
   Pro Val Ile Gly Val Gln Val Ser Arg Gly Gln Pro Leu Gly Gly Thr
               100                 105                 110
   gtg act ggg cac ccc act ttt aat gct ttg ctt gat gca gaa aat gtg    384
   Val Thr Gly His Pro Thr Phe Asn Ala Leu Leu Asp Ala Glu Asn Val
           115                 120                 125
   aat aga aaa gtc acc acc cag acc acc gac gac agg aaa cag aca ggc    432
   Asn Arg Lys Val Thr Thr Gln Thr Thr Asp Asp Arg Lys Gln Thr Gly
       130                 135                 140
   ctg gat gcc aag cag cag cag atc ctg ctg ctg ggc tgt acc cct gct    480
   Leu Asp Ala Lys Gln Gln Gln Ile Leu Leu Leu Gly Cys Thr Pro Ala
   145                 150                 155                 160
   gaa ggg gaa tat tgg aca aca gcc cgt cca tgt gtg acc gac cgt cta    528
   Glu Gly Glu Tyr Trp Thr Thr Ala Arg Pro Cys Val Thr Asp Arg Leu
                   165                 170                 175
   gaa aac ggc gcc tgc cct cct ctg gag ctg aaa aac aag cac atc gaa    576
   Glu Asn Gly Ala Cys Pro Pro Leu Glu Leu Lys Asn Lys His Ile Glu
               180                 185                 190
   gat ggg gat atg atg gaa att ggg ttt ggt gca gcc aac ttc aaa gaa    624
   Asp Gly Asp Met Met Glu Ile Gly Phe Gly Ala Ala Asn Phe Lys Glu
           195                 200                 205
   att aat gca agt aaa tca gat cta cct ctg gac atc caa aat gag atc    672
   Ile Asn Ala Ser Lys Ser Asp Leu Pro Leu Asp Ile Gln Asn Glu Ile
       210                 215                 220
   tgc ctg tac ccc gac tac ctg aaa atg gct gag gac gcc gcc ggc aac    720
   Cys Leu Tyr Pro Asp Tyr Leu Lys Met Ala Glu Asp Ala Ala Gly Asn
   225                 230                 235                 240
   agc atg ttc ttc ttc gcc agg aag gag cag gtg tac gtg aga cac atc    768
   Ser Met Phe Phe Phe Ala Arg Lys Glu Gln Val Tyr Val Arg His Ile
                   245                 250                 255
   tgg acc aga ggc ggc tcc gag aaa gaa gcc cct acc aca gat ttt tat    816
   Trp Thr Arg Gly Gly Ser Glu Lys Glu Ala Pro Thr Thr Asp Phe Tyr
               260                 265                 270
   ttg aag aac aac aag ggc gac gcc acc ctg aag atc ccc agc gtg cac    864
   Leu Lys Asn Asn Lys Gly Asp Ala Thr Leu Lys Ile Pro Ser Val His
           275                 280                 285
   ttc ggc agc ccc agc ggc tca cta gtg tcc acc gac aac cag atc ttc    912
   Phe Gly Ser Pro Ser Gly Ser Leu Val Ser Thr Asp Asn Gln Ile Phe
       290                 295                 300
   aac cgg ccc tac tgg ctg ttc cgc gcc cag ggc atg aac aat gga att    960
   Asn Arg Pro Tyr Trp Leu Phe Arg Ala Gln Gly Met Asn Asn Gly Ile
   305                 310                 315                 320
   gcc tgg aac aac ctg ctg ttc ctg acc gtg ggc gac aac aca cgt ggc    1008
   Ala Trp Asn Asn Leu Leu Phe Leu Thr Val Gly Asp Asn Thr Arg Gly
                   325                 330                 335
   acc aac ctg acc atc agc gtg gcc tcc gat gga acc cca ctg acc gag    1056
   Thr Asn Leu Thr Ile Ser Val Ala Ser Asp Gly Thr Pro Leu Thr Glu
               340                 345                 350
   tat gat agc tcg aaa ttc aac gtg tac cac aga cac atg gag gag tat    1104
   Tyr Asp Ser Ser Lys Phe Asn Val Tyr His Arg His Met Glu Glu Tyr
           355                 360                 365
   aag cta gcc ttc atc ctg gag ctg tgc tcc gtg gag atc acc gcc cag    1152
   Lys Leu Ala Phe Ile Leu Glu Leu Cys Ser Val Glu Ile Thr Ala Gln
       370                 375                 380
   acc gtg tcc cat ctg caa gga ctg atg ccc tcc gtg ctg gag aat tgg    1200
   Thr Val Ser His Leu Gln Gly Leu Met Pro Ser Val Leu Glu Asn Trp
   385                 390                 395                 400
   gag atc ggc gtg cag ccc ccc acc tca tcg atc ttg gag gac acc tac    1248
   Glu Ile Gly Val Gln Pro Pro Thr Ser Ser Ile Leu Glu Asp Thr Tyr
                   405                 410                 415
   cgc tac atc gag tcc ccc gcc acc aag tgt gcc agc aac gtg att cct    1296
   Arg Tyr Ile Glu Ser Pro Ala Thr Lys Cys Ala Ser Asn Val Ile Pro
               420                 425                 430
   gca aaa gaa gac cct tat gca ggg ttt aag ttc tgg aac atc gac ctg    1344
   Ala Lys Glu Asp Pro Tyr Ala Gly Phe Lys Phe Trp Asn Ile Asp Leu
           435                 440                 445
   aag gag aag ctg tct ctg gac ctg gat cag ttc ccc ttg ggc aga aga    1392
   Lys Glu Lys Leu Ser Leu Asp Leu Asp Gln Phe Pro Leu Gly Arg Arg
       450                 455                 460
   ttt ctg gcc cag cag ggg gcc ggc tgt tcc acc gtg aga aaa cgc agg    1440
   Phe Leu Ala Gln Gln Gly Ala Gly Cys Ser Thr Val Arg Lys Arg Arg
   465                 470                 475                 480
   atc agc cag aag acc tcc agc aag ccc gcc aag aag aag aaa aag taa    1488
   Ile Ser Gln Lys Thr Ser Ser Lys Pro Ala Lys Lys Lys Lys Lys
                   485                 490                 495
<210> 4
   <211> 495
   <212> PRT
   <213> Artificial Sequence
<400> 4
   Met Ala Leu Trp Gln Gln Gly Gln Lys Leu Tyr Leu Pro Pro Thr Pro
   1               5                   10                  15
   Val Ser Lys Val Leu Cys Ser Glu Thr Tyr Val Gln Arg Lys Ser Ile
               20                  25                  30
   Phe Tyr His Ala Glu Thr Glu Arg Leu Leu Thr Ile Gly His Pro Tyr
           35                  40                  45
   Tyr Pro Val Ser Ile Gly Ala Lys Thr Val Pro Lys Val Ser Ala Asn
       50                  55                  60
   Gln Tyr Arg Val Phe Lys Ile Gln Leu Pro Asp Pro Asn Gln Phe Ala
   65                  70                  75                  80
   Leu Pro Asp Arg Thr Val His Asn Pro Ser Lys Glu Arg Leu Val Trp
                   85                  90                  95
   Pro Val Ile Gly Val Gln Val Ser Arg Gly Gln Pro Leu Gly Gly Thr
               100                 105                 110
   Val Thr Gly His Pro Thr Phe Asn Ala Leu Leu Asp Ala Glu Asn Val
           115                 120                 125
   Asn Arg Lys Val Thr Thr Gln Thr Thr Asp Asp Arg Lys Gln Thr Gly
       130                 135                 140
   Leu Asp Ala Lys Gln Gln Gln Ile Leu Leu Leu Gly Cys Thr Pro Ala
   145                 150                 155                 160
   Glu Gly Glu Tyr Trp Thr Thr Ala Arg Pro Cys Val Thr Asp Arg Leu
                   165                 170                 175
   Glu Asn Gly Ala Cys Pro Pro Leu Glu Leu Lys Asn Lys His Ile Glu
               180                 185                 190
   Asp Gly Asp Met Met Glu Ile Gly Phe Gly Ala Ala Asn Phe Lys Glu
           195                 200                 205
   Ile Asn Ala Ser Lys Ser Asp Leu Pro Leu Asp Ile Gln Asn Glu Ile
       210                 215                 220
   Cys Leu Tyr Pro Asp Tyr Leu Lys Met Ala Glu Asp Ala Ala Gly Asn
   225                 230                 235                 240
   Ser Met Phe Phe Phe Ala Arg Lys Glu Gln Val Tyr Val Arg His Ile
                   245                 250                 255
   Trp Thr Arg Gly Gly Ser Glu Lys Glu Ala Pro Thr Thr Asp Phe Tyr
               260                 265                 270
   Leu Lys Asn Asn Lys Gly Asp Ala Thr Leu Lys Ile Pro Ser Val His
           275                 280                 285
   Phe Gly Ser Pro Ser Gly Ser Leu Val Ser Thr Asp Asn Gln Ile Phe
       290                 295                 300
   Asn Arg Pro Tyr Trp Leu Phe Arg Ala Gln Gly Met Asn Asn Gly Ile
   305                 310                 315                 320
   Ala Trp Asn Asn Leu Leu Phe Leu Thr Val Gly Asp Asn Thr Arg Gly
                   325                 330                 335
   Thr Asn Leu Thr Ile Ser Val Ala Ser Asp Gly Thr Pro Leu Thr Glu
               340                 345                 350
   Tyr Asp Ser Ser Lys Phe Asn Val Tyr His Arg His Met Glu Glu Tyr
           355                 360                 365
   Lys Leu Ala Phe Ile Leu Glu Leu Cys Ser Val Glu Ile Thr Ala Gln
       370                 375                 380
   Thr Val Ser His Leu Gln Gly Leu Met Pro Ser Val Leu Glu Asn Trp
   385                 390                 395                 400
   Glu Ile Gly Val Gln Pro Pro Thr Ser Ser Ile Leu Glu Asp Thr Tyr
                   405                 410                 415
   Arg Tyr Ile Glu Ser Pro Ala Thr Lys Cys Ala Ser Asn Val Ile Pro
               420                 425                 430
   Ala Lys Glu Asp Pro Tyr Ala Gly Phe Lys Phe Trp Asn Ile Asp Leu
           435                 440                 445
   Lys Glu Lys Leu Ser Leu Asp Leu Asp Gln Phe Pro Leu Gly Arg Arg
       450                 455                 460
   Phe Leu Ala Gln Gln Gly Ala Gly Cys Ser Thr Val Arg Lys Arg Arg
   465                 470                 475                 480
   Ile Ser Gln Lys Thr Ser Ser Lys Pro Ala Lys Lys Lys Lys Lys
                   485                 490                 495
<210> 5
   <211> 1410
   <212> DNA
   <213> Bovine papillomavirus type 1
<220>
   <221> CDS
   <222> (1)..(1410)
<220>
   <223> L2 open reading frame (wild-type)
<400> 5
   atg agt gca cga aaa aga gta aaa cgt gcc agt gcc tat gac ctg tac    48
   Met Ser Ala Arg Lys Arg Val Lys Arg Ala Ser Ala Tyr Asp Leu Tyr
    1               5                  10                  15
   agg acc tgc aag caa gcg ggc aca tgt cca cca gat gtg ata cga aag    96
   Arg Thr Cys Lys Gln Ala Gly Thr Cys Pro Pro Asp Val Ile Arg Lys
               20                  25                  30
   gta gaa gga gat act ata gca gat aaa att ttg aaa ttt ggg ggt ctt   144
   Val Glu Gly Asp Thr Ile Ala Asp Lys Ile Leu Lys Phe Gly Gly Leu
           35                  40                  45
   gca atc tac tta gga ggg cta gga ata gga aca tgg tct act gga agg   192
   Ala Ile Tyr Leu Gly Gly Leu Gly Ile Gly Thr Trp Ser Thr Gly Arg
       50                  55                  60
   gtg gcc gca ggt gga tca cca agg tac aca cca ctc cga aca gca ggg   240
   Val Ala Ala Gly Gly Ser Pro Arg Tyr Thr Pro Leu Arg Thr Ala Gly
   65                  70                  75                  80
   tcc aca tca tcg ctt gca tca ata gga tcc aga gct gta aca gca ggg   288
   Ser Thr Ser Ser Leu Ala Ser Ile Gly Ser Arg Ala Val Thr Ala Gly
                   85                  90                  95
   acc cgc ccc agt ata ggt gcg ggc att cct tta gac acc ctt gaa act   336
   Thr Arg Pro Ser Ile Gly Ala Gly Ile Pro Leu Asp Thr Leu Glu Thr
               100                 105                 110
   ctt ggg gcc ttg cgt cca ggg gtg tat gag gac act gtg cta cca gag   384
   Leu Gly Ala Leu Arg Pro Gly Val Tyr Glu Asp Thr Val Leu Pro Glu
           115                 120                 125
   gcc cct gca ata gtc act cct gat gct gtt cct gca gat tca ggg ctt   432
   Ala Pro Ala Ile Val Thr Pro Asp Ala Val Pro Ala Asp Ser Gly Leu
       130                 135                 140
   gat gcc ctg tcc ata ggt aca gac tcg tcc acg gag acc ctc att act   480
   Asp Ala Leu Ser Ile Gly Thr Asp Ser Ser Thr Glu Thr Leu Ile Thr
   145                 150                 155                 160
   ctg cta gag cct gag ggt ccc gag gac ata gcg gtt ctt gag ctg caa   528
   Leu Leu Glu Pro Glu Gly Pro Glu Asp Ile Ala Val Leu Glu Leu Gln
                   165                 170                 175
   ccc ctg gac cgt cca act tgg caa gta agc aat gct gtt cat cag tcc   576
   Pro Leu Asp Arg Pro Thr Trp Gln Val Ser Asn Ala Val His Gln Ser
               180                 185                 190
   tct gca tac cac gcc cct ctg cag ctg caa tcg tcc att gca gaa aca     624
   Ser Ala Tyr His Ala Pro Leu Gln Leu Gln Ser Ser Ile Ala Glu Thr
           195                 200                 205
   tct ggt tta gaa aat att ttt gta gga ggc tcg ggt tta ggg gat aca     672
   Ser Gly Leu Glu Asn Ile Phe Val Gly Gly Ser Gly Leu Gly Asp Thr
       210                 215                 220
   gga gga gaa aac att gaa ctg aca tac ttc ggg tcc cca cga aca agc     720
   Gly Gly Glu Asn Ile Glu Leu Thr Tyr Phe Gly Ser Pro Arg Thr Ser
   225                 230                 235                 240
   acg ccc cgc agt att gcc tct aaa tca cgt ggc att tta aac tgg ttc     768
   Thr Pro Arg Ser Ile Ala Ser Lys Ser Arg Gly Ile Leu Asn Trp Phe
                   245                 250                 255
   agt aaa cgg tac tac aca cag gtg ccc acg gaa gat cct gaa gtg ttt     816
   Ser Lys Arg Tyr Tyr Thr Gln Val Pro Thr Glu Asp Pro Glu Val Phe
               260                 265                 270
   tca tcc caa aca ttt gca aac cca ctg tat gaa gca gaa cca gct gtg     864
   Ser Ser Gln Thr Phe Ala Asn Pro Leu Tyr Glu Ala Glu Pro Ala Val
           275                 280                 285
   ctt aag gga cct agt gga cgt gtt gga ctc agt cag gtt tat aaa cct     912
   Leu Lys Gly Pro Ser Gly Arg Val Gly Leu Ser Gln Val Tyr Lys Pro
       290                 295                 300
   gat aca ctt aca aca cgt agc ggg aca gag gtg gga cca cag cta cat     960
   Asp Thr Leu Thr Thr Arg Ser Gly Thr Glu Val Gly Pro Gln Leu His
   305                 310                 315                 320
   gtc agg tac tca ttg agt act ata cat gaa gat gta gaa gca atc ccc     1008
   Val Arg Tyr Ser Leu Ser Thr Ile His Glu Asp Val Glu Ala Ile Pro
                   325                 330                 335
   tac aca gtt gat gaa aat aca cag gga ctt gca ttc gta ccc ttg cat     1056
   Tyr Thr Val Asp Glu Asn Thr Gln Gly Leu Ala Phe Val Pro Leu His
               340                 345                 350
   gaa gag caa gca ggt ttt gag gag ata gaa tta gat gat ttt agt gag     1104
   Glu Glu Gln Ala Gly Phe Glu Glu Ile Glu Leu Asp Asp Phe Ser Glu
           355                 360                 365
   aca cat aga ctg cta cct cag aac acc tct tct aca cct gtt ggt agt     1152
   Thr His Arg Leu Leu Pro Gln Asn Thr Ser Ser Thr Pro Val Gly Ser
       370                 375                 380
   ggt gta cga aga agc ctc att cca act cga gaa ttt agt gca aca cgg     1200
   Gly Val Arg Arg Ser Leu Ile Pro Thr Arg Glu Phe Ser Ala Thr Arg
   385                 390                 395                 400
   cct aca ggt gtt gta acc tat ggc tca cct gac act tac tct gct agc     1248
   Pro Thr Gly Val Val Thr Tyr Gly Ser Pro Asp Thr Tyr Ser Ala Ser
                   405                 410                 415
   cca gtt act gac cct gat tct acc tct cct agt cta gtt atc gat gac    1296
   Pro Val Thr Asp Pro Asp Ser Thr Ser Pro Ser Leu Val Ile Asp Asp
               420                 425                 430
   act act act aca cca atc att ata att gat ggg cac aca gtt gat ttg    1344
   Thr Thr Thr Thr Pro Ile Ile Ile Ile Asp Gly His Thr Val Asp Leu
           435                 440                 445
   tac agc agt aac tac acc ttg cat ccc tcc ttg ttg agg aaa cga aaa    1392
   Tyr Ser Ser Asn Tyr Thr Leu His Pro Ser Leu Leu Arg Lys Arg Lys
       450                 455                 460
   aaa cgg aaa cat gcc taa                                            1410
   Lys Arg Lys His Ala
   465                 470
<210> 6
   <211> 469
   <212> PRT
   <213> Bovine papillomavirus type 1
<400> 6
   Met Ser Ala Arg Lys Arg Val Lys Arg Ala Ser Ala Tyr Asp Leu Tyr
   1               5                   10                  15
   Arg Thr Cys Lys Gln Ala Gly Thr Cys Pro Pro Asp Val Ile Arg Lys
               20                  25                  30
   Val Glu Gly Asp Thr Ile Ala Asp Lys Ile Leu Lys Phe Gly Gly Leu
           35                  40                  45
   Ala Ile Tyr Leu Gly Gly Leu Gly Ile Gly Thr Trp Ser Thr Gly Arg
       50                  55                  60
   Val Ala Ala Gly Gly Ser Pro Arg Tyr Thr Pro Leu Arg Thr Ala Gly
   65                  70                  75                  80
   Ser Thr Ser Ser Leu Ala Ser Ile Gly Ser Arg Ala Val Thr Ala Gly
                   85                  90                  95
   Thr Arg Pro Ser Ile Gly Ala Gly Ile Pro Leu Asp Thr Leu Glu Thr
               100                 105                 110
   Leu Gly Ala Leu Arg Pro Gly Val Tyr Glu Asp Thr Val Leu Pro Glu
           115                 120                 125
   Ala Pro Ala Ile Val Thr Pro Asp Ala Val Pro Ala Asp Ser Gly Leu
       130                 135                 140
   Asp Ala Leu Ser Ile Gly Thr Asp Ser Ser Thr Glu Thr Leu Ile Thr
   145                 150                 155                 160
   Leu Leu Glu Pro Glu Gly Pro Glu Asp Ile Ala Val Leu Glu Leu Gln
                   165                 170                 175
   Pro Leu Asp Arg Pro Thr Trp Gln Val Ser Asn Ala Val His Gln Ser
               180                 185                 190
   Ser Ala Tyr His Ala Pro Leu Gln Leu Gln Ser Ser Ile Ala Glu Thr
           195                 200                 205
   Ser Gly Leu Glu Asn Ile Phe Val Gly Gly Ser Gly Leu Gly Asp Thr
       210                 215                 220
   Gly Gly Glu Asn Ile Glu Leu Thr Tyr Phe Gly Ser Pro Arg Thr Ser
   225                 230                 235                 240
   Thr Pro Arg Ser Ile Ala Ser Lys Ser Arg Gly Ile Leu Asn Trp Phe
                   245                 250                 255
   Ser Lys Arg Tyr Tyr Thr Gln Val Pro Thr Glu Asp Pro Glu Val Phe
               260                 265                 270
   Ser Ser Gln Thr Phe Ala Asn Pro Leu Tyr Glu Ala Glu Pro Ala Val
           275                 280                 285
   Leu Lys Gly Pro Ser Gly Arg Val Gly Leu Ser Gln Val Tyr Lys Pro
       290                 295                 300
   Asp Thr Leu Thr Thr Arg Ser Gly Thr Glu Val Gly Pro Gln Leu His
   305                 310                 315                 320
   Val Arg Tyr Ser Leu Ser Thr Ile His Glu Asp Val Glu Ala Ile Pro
                   325                 330                 335
   Tyr Thr Val Asp Glu Asn Thr Gln Gly Leu Ala Phe Val Pro Leu His
               340                 345                 350
   Glu Glu Gln Ala Gly Phe Glu Glu Ile Glu Leu Asp Asp Phe Ser Glu
           355                 360                 365
   Thr His Arg Leu Leu Pro Gln Asn Thr Ser Ser Thr Pro Val Gly Ser
       370                 375                 380
   Gly Val Arg Arg Ser Leu Ile Pro Thr Arg Glu Phe Ser Ala Thr Arg
   385                 390                 395                 400
   Pro Thr Gly Val Val Thr Tyr Gly Ser Pro Asp Thr Tyr Ser Ala Ser
                   405                 410                 415
   Pro Val Thr Asp Pro Asp Ser Thr Ser Pro Ser Leu Val Ile Asp Asp
               420                 425                 430
   Thr Thr Thr Thr Pro Ile Ile Ile Ile Asp Gly His Thr Val Asp Leu
           435                 440                 445
   Tyr Ser Ser Asn Tyr Thr Leu His Pro Ser Leu Leu Arg Lys Arg Lys
       450                 455                 460
   Lys Arg Lys His Ala
   465
<210> 7
   <211> 1410
   <212> DNA
   <213> Artificial Sequence
<220>
   <221> CDS
   <222> (1)..(1410)
<220>
   <223> Description of Artificial Sequence: Bovine papillomavirus type 1 L2 open reading frame (humanized)
<220>
   <223> Wild-type codons replaced with synonymous codons used at relatively high frequency by human genes
<400> 7
   atg agc gcc cgc aag aga gtg aag cgc gcc agc gcc tac gac ctg tac    48
   Met Ser Ala Arg Lys Arg Val Lys Arg Ala Ser Ala Tyr Asp Leu Tyr
    1               5                  10                  15
   agg acc tgc aag cag gcc ggc aca tgt cca cca gat gtg atc cga aag    96
   Arg Thr Cys Lys Gln Ala Gly Thr Cys Pro Pro Asp Val Ile Arg Lys
               20                  25                  30
   gtg gag ggc gac acc atc gcc gac aag atc ctg aag ttc ggc ggc ctg   144
   Val Glu Gly Asp Thr Ile Ala Asp Lys Ile Leu Lys Phe Gly Gly Leu
           35                  40                  45
   gcc atc tac ctg ggc ggc ctg ggc atc gga aca tgg tct acc ggc agg   192
   Ala Ile Tyr Leu Gly Gly Leu Gly Ile Gly Thr Trp Ser Thr Gly Arg
       50                  55                  60
   gtg gcc gcc ggc ggc tca cca agg tac acc cca ctg cgc acc gcc ggc   240
   Val Ala Ala Gly Gly Ser Pro Arg Tyr Thr Pro Leu Arg Thr Ala Gly
   65                  70                  75                  80
   tcc acc tcc tcc ctg gcc tcc atc gga tcc aga gcc gtg acc gcc ggg   288
   Ser Thr Ser Ser Leu Ala Ser Ile Gly Ser Arg Ala Val Thr Ala Gly
                   85                  90                  95
   acc cgc ccc tcc atc ggc gcg ggc atc cct ctg gac acc ctg gaa act   336
   Thr Arg Pro Ser Ile Gly Ala Gly Ile Pro Leu Asp Thr Leu Glu Thr
               100                 105                 110
   ctt ggg gcc ctg cgc cct ggc gtg tac gag gac acc gtg ctg ccc gaa   384
   Leu Gly Ala Leu Arg Pro Gly Val Tyr Glu Asp Thr Val Leu Pro Glu
           115                 120                 125
   gcc cct gcc atc gtg acc cct gac gcc gtg cct gca gac tcc ggc ctg   432
   Ala Pro Ala Ile Val Thr Pro Asp Ala Val Pro Ala Asp Ser Gly Leu
       130                 135                 140
   gac gcc ctg tcc atc ggc aca gac tcc tcc acc gag acc ctg atc acc    480
   Asp Ala Leu Ser Ile Gly Thr Asp Ser Ser Thr Glu Thr Leu Ile Thr
   145                 150                 155                 160
   ctg ctg gag cct gag ggc ccc gaa gac ata gcc gtg ctg gaa ctc cag    528
   Leu Leu Glu Pro Glu Gly Pro Glu Asp Ile Ala Val Leu Glu Leu Gln
                   165                 170                 175
   ccc ctg gac cgc cca acc tgg cag gtg agc aat gct gtg cac cag tcc    576
   Pro Leu Asp Arg Pro Thr Trp Gln Val Ser Asn Ala Val His Gln Ser
               180                 185                 190
   tct gcc tac cac gcc cct ctc cag ctg caa tcc tcc atc gcc gag aca    624
   Ser Ala Tyr His Ala Pro Leu Gln Leu Gln Ser Ser Ile Ala Glu Thr
           195                 200                 205
   tct ggt tta gaa aat att ttt gta gga ggc tcg ggt tta ggg gat acc    672
   Ser Gly Leu Glu Asn Ile Phe Val Gly Gly Ser Gly Leu Gly Asp Thr
       210                 215                 220
   ggc ggc gag aac atc gag ctg acc tac ttc ggc tcc ccc cgc acc agc    720
   Gly Gly Glu Asn Ile Glu Leu Thr Tyr Phe Gly Ser Pro Arg Thr Ser
   225                 230                 235                 240
   acc ccc cgc tcc atc gcc tcc aag tcc cgc ggc atc ctg aac tgg ttc    768
   Thr Pro Arg Ser Ile Ala Ser Lys Ser Arg Gly Ile Leu Asn Trp Phe
                   245                 250                 255
   agc aag cgg tac tac acc cag gtg ccc acc gaa gat ccc gaa gtg ttc    816
   Ser Lys Arg Tyr Tyr Thr Gln Val Pro Thr Glu Asp Pro Glu Val Phe
               260                 265                 270
   tcc tcc cag acc ttc gcc aac ccc ctg tac gag gcc gag ccc gcc gtg    864
   Ser Ser Gln Thr Phe Ala Asn Pro Leu Tyr Glu Ala Glu Pro Ala Val
           275                 280                 285
   ctg aag ggc cct agc ggc cgc gtg ggc ctg tcc cag gtg tac aag cct    912
   Leu Lys Gly Pro Ser Gly Arg Val Gly Leu Ser Gln Val Tyr Lys Pro
       290                 295                 300
   gat acc ctg acc aca cgt agc ggc aca gag gtg ggc ccc cag ctg cat    960
   Asp Thr Leu Thr Thr Arg Ser Gly Thr Glu Val Gly Pro Gln Leu His
   305                 310                 315                 320
   gtg agg tac tcc ctg tcc acc atc cat gag gat gtg gag gct atc ccc    1008
   Val Arg Tyr Ser Leu Ser Thr Ile His Glu Asp Val Glu Ala Ile Pro
                   325                 330                 335
   tac acc gtg gat gag aac acc cag ggc ctg gcc ttc gtg ccc ctg cat    1056
   Tyr Thr Val Asp Glu Asn Thr Gln Gly Leu Ala Phe Val Pro Leu His
               340                 345                 350
   gag gag cag gcc ggc ttc gag gag atc gag ctc gac gat ttc agc gag    1104
   Glu Glu Gln Ala Gly Phe Glu Glu Ile Glu Leu Asp Asp Phe Ser Glu
           355                 360                 365
   acc cat cgc ctg ctg ccc cag aac acc tcc tcc acc ccc gtg ggc agc    1152
   Thr His Arg Leu Leu Pro Gln Asn Thr Ser Ser Thr Pro Val Gly Ser
       370                 375                 380
   ggc gtg cgc aga agc ctg atc cct acc cga gag ttc agc gcc acc cgg    1200
   Gly Val Arg Arg Ser Leu Ile Pro Thr Arg Glu Phe Ser Ala Thr Arg
   385                 390                 395                 400
   cct acc ggc gtg gtg acc tac ggc tcc ccc gac acc tac tcc gct agc    1248
   Pro Thr Gly Val Val Thr Tyr Gly Ser Pro Asp Thr Tyr Ser Ala Ser
                   405                 410                 415
   ccc gtg acc gac cct gat tct acc tct cct agc ctg gtg atc gac gac    1296
   Pro Val Thr Asp Pro Asp Ser Thr Ser Pro Ser Leu Val Ile Asp Asp
               420                 425                 430
   acc acc acc acc ccc atc atc atc atc gac ggc cac aca gtg gat ctg    1344
   Thr Thr Thr Thr Pro Ile Ile Ile Ile Asp Gly His Thr Val Asp Leu
           435                 440                 445
   tac agc agc aac tac acc ctg cat ccc tcc ctg ctg agg aag cgc aag    1392
   Tyr Ser Ser Asn Tyr Thr Leu His Pro Ser Leu Leu Arg Lys Arg Lys
       450                 455                 460
   aag cgc aag cat gcc taa                                            1410
   Lys Arg Lys His Ala
   465                 470
<210> 8
   <211> 469
   <212> PRT
   <213> Artificial Sequence
<400> 8
   Met Ser Ala Arg Lys Arg Val Lys Arg Ala Ser Ala Tyr Asp Leu Tyr
   1               5                   10                  15
   Arg Thr Cys Lys Gln Ala Gly Thr Cys Pro Pro Asp Val Ile Arg Lys
               20                  25                  30
   Val Glu Gly Asp Thr Ile Ala Asp Lys Ile Leu Lys Phe Gly Gly Leu
           35                  40                  45
   Ala Ile Tyr Leu Gly Gly Leu Gly Ile Gly Thr Trp Ser Thr Gly Arg
       50                  55                  60
   Val Ala Ala Gly Gly Ser Pro Arg Tyr Thr Pro Leu Arg Thr Ala Gly
   65                  70                  75                  80
   Ser Thr Ser Ser Leu Ala Ser Ile Gly Ser Arg Ala Val Thr Ala Gly
                   85                  90                  95
   Thr Arg Pro Ser Ile Gly Ala Gly Ile Pro Leu Asp Thr Leu Glu Thr
               100                 105                 110
   Leu Gly Ala Leu Arg Pro Gly Val Tyr Glu Asp Thr Val Leu Pro Glu
           115                 120                 125
   Ala Pro Ala Ile Val Thr Pro Asp Ala Val Pro Ala Asp Ser Gly Leu
       130                 135                 140
   Asp Ala Leu Ser Ile Gly Thr Asp Ser Ser Thr Glu Thr Leu Ile Thr
   145                 150                 155                 160
   Leu Leu Glu Pro Glu Gly Pro Glu Asp Ile Ala Val Leu Glu Leu Gln
                   165                 170                 175
   Pro Leu Asp Arg Pro Thr Trp Gln Val Ser Asn Ala Val His Gln Ser
               180                 185                 190
   Ser Ala Tyr His Ala Pro Leu Gln Leu Gln Ser Ser Ile Ala Glu Thr
           195                 200                 205
   Ser Gly Leu Glu Asn Ile Phe Val Gly Gly Ser Gly Leu Gly Asp Thr
       210                 215                 220
   Gly Gly Glu Asn Ile Glu Leu Thr Tyr Phe Gly Ser Pro Arg Thr Ser
   225                 230                 235                 240
   Thr Pro Arg Ser Ile Ala Ser Lys Ser Arg Gly Ile Leu Asn Trp Phe
                   245                 250                 255
   Ser Lys Arg Tyr Tyr Thr Gln Val Pro Thr Glu Asp Pro Glu Val Phe
               260                 265                 270
   Ser Ser Gln Thr Phe Ala Asn Pro Leu Tyr Glu Ala Glu Pro Ala Val
           275                 280                 285
   Leu Lys Gly Pro Ser Gly Arg Val Gly Leu Ser Gln Val Tyr Lys Pro
       290                 295                 300
   Asp Thr Leu Thr Thr Arg Ser Gly Thr Glu Val Gly Pro Gln Leu His
   305                 310                 315                 320
   Val Arg Tyr Ser Leu Ser Thr Ile His Glu Asp Val Glu Ala Ile Pro
                   325                 330                 335
   Tyr Thr Val Asp Glu Asn Thr Gln Gly Leu Ala Phe Val Pro Leu His
               340                 345                 350
   Glu Glu Gln Ala Gly Phe Glu Glu Ile Glu Leu Asp Asp Phe Ser Glu
           355                 360                 365
   Thr His Arg Leu Leu Pro Gln Asn Thr Ser Ser Thr Pro Val Gly Ser
       370                 375                 380
   Gly Val Arg Arg Ser Leu Ile Pro Thr Arg Glu Phe Ser Ala Thr Arg
   385                 390                 395                 400
   Pro Thr Gly Val Val Thr Tyr Gly Ser Pro Asp Thr Tyr Ser Ala Ser
                   405                 410                 415
   Pro Val Thr Asp Pro Asp Ser Thr Ser Pro Ser Leu Val Ile Asp Asp
               420                 425                 430
   Thr Thr Thr Thr Pro Ile Ile Ile Ile Asp Gly His Thr Val Asp Leu
           435                 440                 445
   Tyr Ser Ser Asn Tyr Thr Leu His Pro Ser Leu Leu Arg Lys Arg Lys
       450                 455                 460
   Lys Arg Lys His Ala
   465
<210> 9
   <211> 717
   <212> DNA
   <213> Artificial Sequence
<220>
   <223> Description of Artificial Sequence: Aequorea victoria gfp gene (humanized)
<220>
   <221> CDS
   <222> (1) .. (717)
<400> 9
   atg agc aag ggc gag gaa ctg ttc act ggc gtg gtc cca att ctc gtg    48
   Met Ser Lys Gly Glu Glu Leu Phe Thr Gly Val Val Pro Ile Leu Val
     1               5                  10                  15
   gaa ctg gat ggc gat gtg aat ggg cac aaa ttt tct gtc agc gga gag    96
   Glu Leu Asp Gly Asp Val Asn Gly His Lys Phe Ser Val Ser Gly Glu
                20                  25                  30
   ggt gaa ggt gat gcc aca tac gga aag ctc acc ctg aaa ttc atc tgc   144
   Gly Glu Gly Asp Ala Thr Tyr Gly Lys Leu Thr Leu Lys Phe Ile Cys
            35                  40                  45
   acc act gga aag ctc cct gtg cca tgg cca aca ctg gtc act acc ttc   192
   Thr Thr Gly Lys Leu Pro Val Pro Trp Pro Thr Leu Val Thr Thr Phe
        50                  55                  60
   tct tat ggc gtg cag tgc ttt tcc aga tac cca gac cat atg aag cag   240
   Ser Tyr Gly Val Gln Cys Phe Ser Arg Tyr Pro Asp His Met Lys Gln
    65                  70                  75                  80
   cat gac ttt ttc aag agc gcc atg ccc gag ggc tat gtg cag gag aga   288
   His Asp Phe Phe Lys Ser Ala Met Pro Glu Gly Tyr Val Gln Glu Arg
                    85                  90                  95
   acc atc ttt ttc aaa gat gac ggg aac tac aag acc cgc gct gaa gtc   336
   Thr Ile Phe Phe Lys Asp Asp Gly Asn Tyr Lys Thr Arg Ala Glu Val
               100                 105                 110
   aag ttc gaa ggt gac acc ctg gtg aat aga atc gag ctg aag ggc att   384
   Lys Phe Glu Gly Asp Thr Leu Val Asn Arg Ile Glu Leu Lys Gly Ile
               115                     120                     125
   gac ttt aag gag gat gga aac att ctc ggc cac aag ctg gaa tac aac      432
   Asp Phe Lys Glu Asp Gly Asn Ile Leu Gly His Lys Leu Glu Tyr Asn
       130                     135                     140
   tat aac tcc cac aat gtg tac atc atg gcc gac aag caa aag aat ggc      480
   Tyr Asn Ser His Asn Val Tyr Ile Met Ala Asp Lys Gln Lys Asn Gly
   145                     150                     155                 160
   atc aag gtc aac ttc aag atc aga cac aac att gag gat gga tcc gtg      528
   Ile Lys Val Asn Phe Lys Ile Arg His Asn Ile Glu Asp Gly Ser Val
                   165                     170                     175
   cag ctg gcc gac cat tat caa cag aac act cca atc ggc gac ggc cct      576
   Gln Leu Ala Asp His Tyr Gln Gln Asn Thr Pro Ile Gly Asp Gly Pro
               180                     185                     190
   gtg ctc ctc cca gac aac cat tac ctg tcc acc cag tct gcc ctg tct      624
   Val Leu Leu Pro Asp Asn His Tyr Leu Ser Thr Gln Ser Ala Leu Ser
           195                     200                     205
   aaa gat ccc aac gaa aag aga gac cac atg gtc ctg ctg gag ttt gtg      672
   Lys Asp Pro Asn Glu Lys Arg Asp His Met Val Leu Leu Glu Phe Val
       210                     215                     220
   acc gct gct ggg atc aca cat ggc atg gac gag ctg tac aag tga          717
   Thr Ala Ala Gly Ile Thr His Gly Met Asp Glu Leu Tyr Lys
   225                     230                     235
<210> 10
   <211> 238
   <212> PRT
   <213> Artificial Sequence
<400> 10
   Met Ser Lys Gly Glu Glu Leu Phe Thr Gly Val Val Pro Ile Leu Val
   1               5                   10                  15
   Glu Leu Asp Gly Asp Val Asn Gly His Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Arg His Asn Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 11
   <211> 717
   <212> DNA
   <213> Artificial Sequence
<220>
   <221> CDS
   <222> (1)..(717)
<220>
   <223> Description of Artificial Sequence: Synthetic gfp gene (Papillomavirusized)
<220>
   <223> Codons of humanized gfp gene replaced with synonymous codons used at relatively high frequency by papillomavirus genes
<400> 11
   atg agt aaa ggg gaa gaa cta ttt aca ggg gtg gtg cct ata cta gtg    48
   Met Ser Lys Gly Glu Glu Leu Phe Thr Gly Val Val Pro Ile Leu Val
     1               5                  10                  15
   gaa cta gat ggg gat gtg aat ggg cac aaa ttt tct gtc agt ggg gaa    96
   Glu Leu Asp Gly Asp Val Asn Gly His Lys Phe Ser Val Ser Gly Glu
                20                  25                  30
   ggg gaa ggg gat gca aca tat ggg aaa cta aca cta aaa ttt ata tgc   144
   Gly Glu Gly Asp Ala Thr Tyr Gly Lys Leu Thr Leu Lys Phe Ile Cys
            35                  40                  45
   aca aca ggg aaa cta cct gtg cca tgg cct aca cta gtg aca aca ttt    192
   Thr Thr Gly Lys Leu Pro Val Pro Trp Pro Thr Leu Val Thr Thr Phe
        50                  55                  60
   agt tat ggg gtg caa tgc ttt agt aga tat cct gat cat atg aaa caa    240
   Ser Tyr Gly Val Gln Cys Phe Ser Arg Tyr Pro Asp His Met Lys Gln
    65                  70                  75                  80
   cat gat ttt ttt aaa agt gca atg ccc gag ggg tat gtg caa gaa aga    288
   His Asp Phe Phe Lys Ser Ala Met Pro Glu Gly Tyr Val Gln Glu Arg
                    85                  90                  95
   aca ata ttt ttt aaa gat gat ggg aat tat aaa aca aga gca gaa gtc    336
   Thr Ile Phe Phe Lys Asp Asp Gly Asn Tyr Lys Thr Arg Ala Glu Val
               100                 105                 110
   aaa ttt gaa ggg gat aca cta gtg aat aga ata gag ctc aaa ggg ata    384
   Lys Phe Glu Gly Asp Thr Leu Val Asn Arg Ile Glu Leu Lys Gly Ile
           115                 120                 125
   gat ttt aaa gaa gat ggg aat ata cta ggg cat aaa cta gaa tat aat    432
   Asp Phe Lys Glu Asp Gly Asn Ile Leu Gly His Lys Leu Glu Tyr Asn
       130                 135                 140
   tat aat agt cat aat gtg tat ata atg gca gat aaa caa aaa aat ggg    480
   Tyr Asn Ser His Asn Val Tyr Ile Met Ala Asp Lys Gln Lys Asn Gly
   145                 150                 155                 160
   ata aaa gtg aat ttt aaa ata ata aga cat ata gaa gat gga tcc gtg    528
   Ile Lys Val Asn Phe Lys Ile Ile Arg His Ile Glu Asp Gly Ser Val
                   165                 170                 175
   caa cta gca gat cat tat caa caa aat aca cct ata ggg gat ggg cct    576
   Gln Leu Ala Asp His Tyr Gln Gln Asn Thr Pro Ile Gly Asp Gly Pro
               180                 185                 190
   gtg cta cta cct gat aac cat tat cta agt aca caa agt gca cta agt    624
   Val Leu Leu Pro Asp Asn His Tyr Leu Ser Thr Gln Ser Ala Leu Ser
           195                 200                 205
   aaa gat cct aat gaa aaa aga gat cat atg gtg cta ctc gag ttt gtg    672
   Lys Asp Pro Asn Glu Lys Arg Asp His Met Val Leu Leu Glu Phe Val
       210                 215                 220
   aca gca gca ggg ata aca cat ggg atg gat gaa cta tat aaa tga        717
   Thr Ala Ala Gly Ile Thr His Gly Met Asp Glu Leu Tyr Lys
   225                 230                 235
<210> 12
   <211> 238
   <212> PRT
   <213> Artificial Sequence
<400> 12
   Met Ser Lys Gly Glu Glu Leu Phe Thr Gly Val Val Pro Ile Leu Val
    1               5                  10                  15
   Glu Leu Asp Gly Asp Val Asn Gly His Lys Phe Ser Val Ser Gly Glu
                   20                  25                  30
   Gly Glu Gly Asp Ala Thr Tyr Gly Lys Leu Thr Leu Lys Phe Ile Cys
               35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Phe Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Tyr Asn Ser His Asn Val Tyr Ile Met Ala Asp Lys Gln Lys Asn Gly
   145                 150                 155                 160
   Ile Lys Val Asn Phe Lys Ile Ile Arg His Ile Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 13
   <211> 17
   <212> DNA
   <213> Artificial Sequence
<220>
   <223> Description of Artificial Sequence: Oligonucleotide specific for Ala(GCA)
<400> 13
   taaggactgt aagactt 17
<210> 14
   <211> 17
   <212> DNA
   <213> Artificial Sequence
<220>
   <223> Description of Artificial Sequence: Oligonucleotide specific for Arg(CGA)
<400> 14
   cgagccagcc aggagtc 17
<210> 15 <211> 17

   <212> DNA
   <213> Artificial Sequence
<220>
   <223> Description of Artificial Sequence: Oligonucleotide specific for Asn(AAC)
<400> 15
   ctagattggc aggaatt 17
<210> 16
   <211> 17
   <212> DNA
   <213> Artificial Sequence
<220>
   <223> Description of Artificial Sequence: Oligonucleotide specific for Asp(GAC)
<400> 16
   taagatatat agattat 17
<210> 17
   <211> 17
   <212> DNA
   <213> Artificial Sequence
<220>
   <223> Description of Artificial Sequence: Oligonucleotide specific for Cys(TGC)
<400> 17
   aagtcttagt agagatt 17
<210> 18
   <211> 17
   <212> DNA
   <213> Artificial Sequence
<220>
   <223> Description of Artificial Sequence: Oligonucleotide specific for Glu(GAA)
<400> 18
   tatttctaca cagcatt 17
<210> 19
   <211> 17
   <212> DNA
   <213> Artificial Sequence
<220>
   <223> Description of Artificial Sequence: Oligonucleotide specific for Gln(CAA)
<400> 19
   ctaggacaat aggaatt 17
<210> 20
   <211> 17
   <212> DNA
   <213> Artificial Sequence
<220>
   <223> Description of Artificial Sequence: Oligonucleotide specific for Gly(GGA)
<400> 20
   tactctcttc tgggttt 17
<210> 21
   <211> 17
   <212> DNA
   <213> Artificial Sequence
<220>
   <223> Description of Artificial Sequence: Oligonucleotide specific for His(CAC)
<400> 21
   tgccgtgact cggattc 17
<210> 22
   <211> 17
   <212> DNA
   <213> Artificial Sequence
<220>
   <223> Description of Artificial Sequence: Oligonucleotide specific for Ile(ATC)
<400> 22
   tagaaataag agggctt 17
<210> 23
   <211> 17
   <212> DNA
   <213> Artificial Sequence
<220>
   <223> Description of Artificial Sequence: Oligonucleotide specific for Leu(CTA)
<400> 23
   tacttttatt tggattt 17
<210> 24
   <211> 17
   <212> DNA
   <213> Artificial Sequence
<220>
   <223> Description of Artificial Sequence: Oligonucleotide specific for Leu(CTT)
<400> 24
   tattagggag aggattt 17
<210> 25
   <211> 17
   <212> DNA
   <213> Artificial Sequence
<220>
   <223> Description of Artificial Sequence: Oligonucleotide specific for Lys(AAA)
<400> 25
   tcactatgga gatttta 17
<210> 26
   <211> 17
   <212> DNA
   <213> Artificial Sequence
<220>
   <223> Description of Artificial Sequence: Oligonucleotide specific for Lys(AAG)
<400> 26
   cgcccaacgt ggggctc 17
<210> 27
   <211> 17
   <212> DNA
   <213> Artificial Sequence
<220>
   <223> Description of Artificial Sequence: Oligonucleotide specific for Met (elong)
<400> 27
   tagtacggga aggattt 17
<210> 28
   <211> 17
   <212> DNA
   <213> Artificial Sequence
<220>
   <223> Description of Artificial Sequence: Oligonucleotide specific for Phe(TTC)
<400> 28
   tgtttatggg atacaat 17
<210> 29
   <211> 17
   <212> DNA
   <213> Artificial Sequence
<220>
   <223> Description of Artificial Sequence: Oligonucleotide specific for Pro(CCA)
<400> 29
   tcaagaagaa ggagcta 17
<210> 30
   <211> 17
<212> DNA
   <213> Artificial Sequence
<220>
   <223> Description of Artificial Sequence: Oligonucleotide specific for Pro(CCI)
<400> 30
   gggctcgtcc gggattt 17
<210> 31
   <211> 17
   <212> DNA
   <213> Artificial Sequence
<220>
   <223> Description of Artificial Sequence: Oligonucleotide specific for Ser(AGC)
<400> 31
   ataagaaagg aagatcg 17
<210> 32
   <211> 17
   <212> DNA
   <213> Artificial Sequence
<220>
   <223> Description of Artificial Sequence: Oligonucleotide specific for Thr(ACA)
<400> 32
   tgtcttgaga agagaag 17
<210> 33
   <211> 17
   <212> DNA
   <213> Artificial Sequence
<220>
   <223> Description of Artificial Sequence: Oligonucleotide specific for Tyr(TAC)
<400> 33
   tggtaaaaag aggattt 17
<210> 34
   <211> 17
   <212> DNA
   <213> Artificial Sequence
<220>
   <223> Description of Artificial Sequence: Oligonucleotide specific for Val(GTA)
<400> 34
   tcagagtgtt cattggt 17

## Description

### FIELD OF THE INVENTION

THIS INVENTION relates generally to gene therapy. More particularly, the present invention relates to a synthetic nucleic acid sequence and to a method for selectively expressing a protein in a target cell or tissue in which at least one existing codon of a parent nucleic acid sequence encoding the protein has been replaced with a synonymous codon. The invention also relates to production of virus particles using one or more synthetic nucleic acid sequences and the method according to the invention.

### BACKGROUND OF THE INVENTION

While gene therapy is of great clinical interest for treatment of gene defects, this therapy has not entered into mainstream clinical practice because selective delivery of genes to target tissues has proven extremely difficult. Currently, viral vectors are used, particularly retroviruses and adenovirus, which are to some extent selective. However, many vector systems are by their nature unable to produce stable integrants and some also invoke immune responses thereby preventing effective treatment. Alternatively, "naked" DNA is packaged in liposomes or other similar delivery systems. A major problem to be overcome is that such gene delivery systems themselves are not tissue selective, whereas selective targeting of genes to particular tissues would be desirable for many disorders (*e.g.*, cancer therapy). While use of tissue specific promoters to target gene therapy has been effective in some animal models it has proven less so in man, and selective tissue specific promoters are not available for a wide range of tissues.

The current invention has arisen unexpectedly from recent investigations exploring why papillomavirus (PV) late gene expression is restricted to differentiated keratinocytes. In this regard, it is known that PV late genes L1 and L2 are only expressed in non-dividing differentiated keratinocytes (KCs). Many investigators including the present inventors have been unable to detect significant PV L1 and L2 protein expression when these genes are transduced or transfected into undifferentiated cultured cells, using a range of conventional constitutive viral promoters including retroviral long terminal repeats (LTRs) and the strong constitutive promoters of CMV and SV40.

PV L1 mRNA can however be efficiently translated *in vitro* using rabbit reticulocyte cell lysate, suggesting that there are no cellular inhibitors in the lysate interfering with translation of L1. The major difference between the *in vitro* and *in vivo* translation systems is that L1 comprises the dominant L1 mRNA in *in vitro* translation reactions, while it constitutes a minor fraction among the cellular mRNAs in intact cells.

*In vivo*, PV late proteins are not produced in undifferentiated KC. However, they are expressed in large quantity in highly differentiated KC. The mechanism of this tight control of late gene expression has been poorly understood, and searches by many groups for KC specific PV gene transcriptional control proteins have been unrewarding.

Blockage to translation of L1 mRNA *in vivo* has been attributed to sequences within the L1 ORF (Tan et al. 1995, J. Virol. 69 5607-5620; Tan and Schwartz, 1995, J. Virol. 69 2932-2945). By using a Rev and Rev-responsive element of HIV, such inhibition could be overcome (Tan *et al.* 1995, *supra*). Accordingly, the inventors examined whether removal of putative "inhibitory sequences" in the L1 ORF would allow production of L1 protein in undifferentiated cells. Deletion mutagenesis of BPV L1 to remove putative inhibitory sequences and expression of resultant deletion mutants in CV-1 cells revealed surprisingly that despite expression of L1 mRNA, L1 protein could not be detected.

In view of the foregoing, it has been difficult hitherto to understand how papillomaviruses produce large amounts of L1 protein in the late stage of their life cycle using this apparently "untranslatable" gene.

Surprisingly, however, it has now been discovered that PV L1 protein can be produced at substantially enhanced levels in an undifferentiated host cell by replacing existing codons of a native L1 gene with synonymous codons used at relatively high frequency by genes of the undifferentiated host cell compared to the existing codons. It has also been found unexpectedly that there are substantial differences in the relative abundance of particular isoaccepting transfer RNAs (tRNAs) in different cells or tissues and this plays a pivotal role in protein expression from a gene with a given codon usage or composition. This discovery has been reduced to practice in synthetic nucleic acid sequences and generic methods, which utilize codon alteration as a means for targeting expression of a protein to particular cells or tissues or alternatively, to cells in a specific state of differentiation.
IE904161 discloses a method for the preparation of recombinant HIU-I particles from host cells by expressing polynucleotides which encode the envelope and viralcore structure polypeptides within the host cells.
Zichun Hua et al (1994) Biochemistry and Molecular Biology International Vol. 32, No. 3, pages 537-543, discloses expression of hGM-CSF in E.coli and the improvement of this resulting from providing the agU gene encoding a minor tRNA.

### OBJECT OF THE INVENTION

It is therefore an object of the present invention to provide a synthetic nucleic acid sequence and a method for selectively expressing a protein in a target cell or tissue which sequence and method ameliorate at least some of the disadvantages associated with the prior art.

### SUMMARY OF THE INVENTION

Accordingly, in one aspect of the invention, there is provided a method of constructing a synthetic polynucleotide that encodes a polypeptide the method comprising selecting a first codon of a parent polynucleotide for replacement with a synonymous codon wherein the synonymous codon is selected on the basis that it exhibits a higher translational efficiency in a first cell of a mammal than in a second cell of the mammal; and replacing the first codon with the synonymous codon to construct the synthetic polynucleotide.

Preferably, the step of selecting is further characterized by the steps of:-
(a) measuring relative abundance of different iso-tRNAs in said target cell or tissue, and in said one or more other cells or tissues; and
(b) identifying said at least one existing codon and said synonymous codon based on said measurement, wherein said synonymous codon corresponds to an iso-tRNA which, when compared to an iso-tRNA corresponding to the existing codon, is in higher abundance in said target cell or tissue relative to said one or more other cells or tissues.

Preferably, the step of selecting is further characterized in that said synonymous codon corresponds to an iso-tRNA which, when compared to an iso-tRNA corresponding to the at least one existing codon, is in higher abundance in the target cell or tissue relative to a precursor cell or tissue of the target cell or tissue.

Alternatively, the step of selecting is further characterized in that said synonymous codon corresponds to an iso-tRNA which, when compared to an iso-tRNA corresponding to the at least one existing codon, is in higher abundance in the target cell or tissue relative to a cell or tissue derived from said target cell or tissue.

Advantageously, the step of selecting is further characterized in that the corresponding iso-tRNA in said target cell or tissue is at a level which is at least 110%, preferably at least 200%, more preferably at least 500%, and most preferably at least 1000%, of that expressed in said one or more other cells or tissues.

Alternatively, the method further includes the step of selecting said synonymous codon from the group consisting of (1) a codon used at relatively high frequency by genes, preferably highly expressed genes, of the target cell or tissue, (2) a codon used at relatively high frequency by genes, preferably highly expressed genes, of said one or more other cells or tissues, (3) a codon used at relatively high frequency by genes, preferably highly expressed genes, of the mammal, (4) a codon used at relatively low frequency by genes of the target cell or tissue, (5) a codon used at relatively low frequency by genes of said one or more other cells or tissues, (6) a codon used at relatively low frequency by genes of the mammal, (7) a codon used at relatively high frequency by genes of another organism, and (8) a codon used at relatively low frequency by genes of another organism.

In a preferred embodiment, the method further includes the step of selecting the at least one existing codon and the synonymous codon such that said protein is expressed from said synthetic polynucleotide in said target cell or tissue at a level which, is at least 110%, preferably at least 200%, more preferably at least 500%, and most preferably at least 1000%, of that expressed from said parent polynucleotide in said target cell or tissue.

In another aspect, the invention provides a synthetic polynucleotide constructed according to any one of the above methods.

Preferably, said synonymous codon(s) are selected from the group consisting of gca (Ala), cuu (Leu) and cua (Leu), and said target cell is a differentiated cell.

More preferably, said synonymous codon (s) are selected from the group consisting of gca (Ala), cuu (Leu) and cua (Leu), and said target cell is a differentiated keratinocyte.

In yet another aspect, the invention resides in a method for selectively expressing a protein in a first cell of a mammal, said method including the steps of:-
(a) selecting a first codon of a parent polynucleotide encoding said protein for replacement with a synonymous codon, wherein the synonymous codon is selected on the basis that it exhibits a higher translational efficiency in the first cell than in a second cell of the mammal;
   - replacing the first codon with the synonymous codon to contruct a synthetic polynucleotide; and
   - introducing the synthetic polynucleotide into a cell selected from the group consisting of the first cell and a precursor of the first cell the synthetic polynucleotide being operably linked to one or more regulatory nucleotide sequences; and
(c) selectively expressing said protein in said target cell or tissue but not in said one or more other cells or tissues.

Preferably, the method further includes, prior to step (a):
(i) measuring relative abundance of different iso-tRNAs in said target cell, and in said one or more other cells or tissues; and
(ii) identifying said at least one existing codon and said synonymous codon based on said measurement, wherein said synonymous codon corresponds to an iso-tRNA which, when compared to an iso-tRNA corresponding to the existing codon, is in higher abundance in said target cell relative to said one or more other cells.

Suitably, step (ii) above is further characterized in that said synonymous codon corresponds to an iso-tRNA which, when compared to an iso-tRNA corresponding to the at least one existing codon, is in higher abundance in the target cell relative to a precursor cell.

Alternatively, step (ii) above is further characterized in that said synonymous codon corresponds to an iso-tRNA which, when compared to an iso-tRNA corresponding to the at least one existing codon, is in higher abundance in the target cell relative to a cell.

Alternatively, the method further includes, prior to step (a), identifying said at least one existing codon and said synonymous codon based on respective relative frequencies of particular codons used by genes selected from the group consisting of (I) genes of the target cell or tissue, (II) genes of the or each other cell or tissue, (III) genes of the mammal, and (IV) genes of another organism.

In yet another aspect, the invention provides a method for expressing a polypeptide from a first polynucleotide in a mammalian cell including the step of selecting an iso-tRNA on the basis that it is normally in lower abundance in the cell than other iso-tRNAs and corresponds to a codon of the first polynucleotide; introducing into the cell a second polynucleotide that encodes the selected iso-tRNA and that is operably linked to a regulatory polynucleotide and expressing the second polynucleotide in the cell, whereby the polypeptide is expressed in the cell.

In a further aspect, the invention extends to a method for producing a virus particle in a cycling eukaryotic cell, said virus particle comprising at least one protein necessary for assembly of said virus particle, wherein said at least one protein is not expressed in said cell from a parent polynucleotide at a level sufficient to permit virus assembly therein, said method including the steps of:-
providing a comparison of translational efficiencies of individual codons in the cell;
Selecting from the comparison a first codon of the parent polynucleotide for replacement with a synonymous codon, wherein the synonymous codon is selected on the basis that is exhibits a higher translational efficiency in the cell than the first codon; and
replacing the first codon with the synonymous codon to construct a synthetic polynucleotide having enhanced translational kinetics compared to said parent polynucleotide such that said at least one protein is expressible from said synthetic polynucleotide in said cell at a level sufficient to permit virus assembly therein;
introducing into said cell or a precursor cell thereof said synthetic polynucleotide operably linked to one or more regulatory nucleotide sequences; and
whereby said at least one protein is expressed and the virus particle is produced.

In yet a further aspect of the invention, there is provided a method for producing a virus particle in a cycling cell, said virus particle comprising at least one protein necessary for assembly of said virus particle, wherein said at least one protein is not expressed in said cell from a parent polynucleotide at a level sufficient to permit virus assembly therein, said method including the step of;
providing a comparison of translational efficiencies of individual codons in the cell;
selecting from the comparison a first codon of the first polynucleotide that exhibits a lower translational efficiency in the cell than a synonymous codon;
introducing into said cell a second polynucleotide encoding an isoaccepting transfer RNA specific for said at least one codon; and
expressing the second polynucleotide in the cell, whereby the virus particle is produced in the cycling cell.

### BRIEF DESCRITPION OF THE DRAWINGS

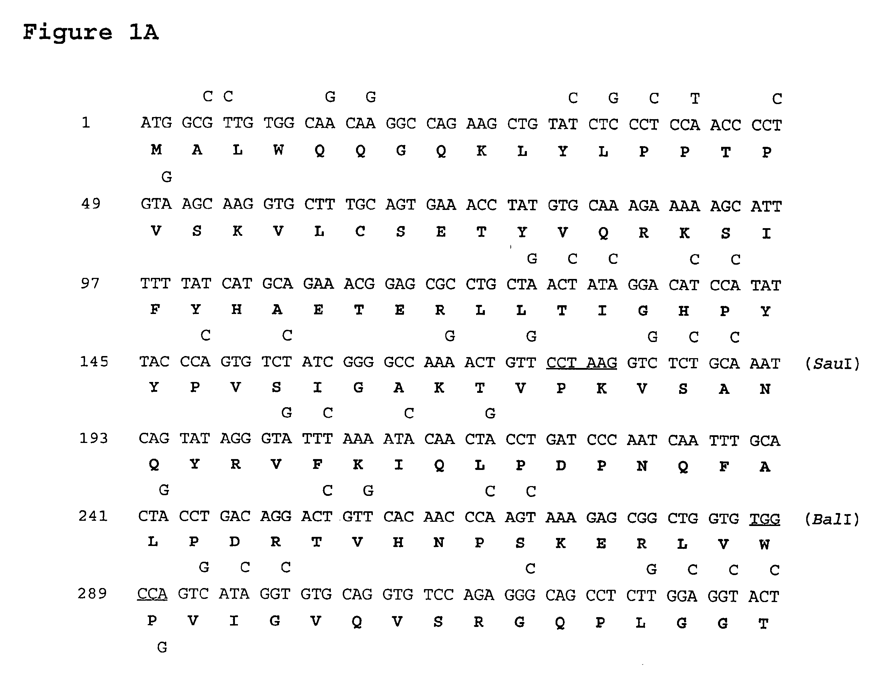

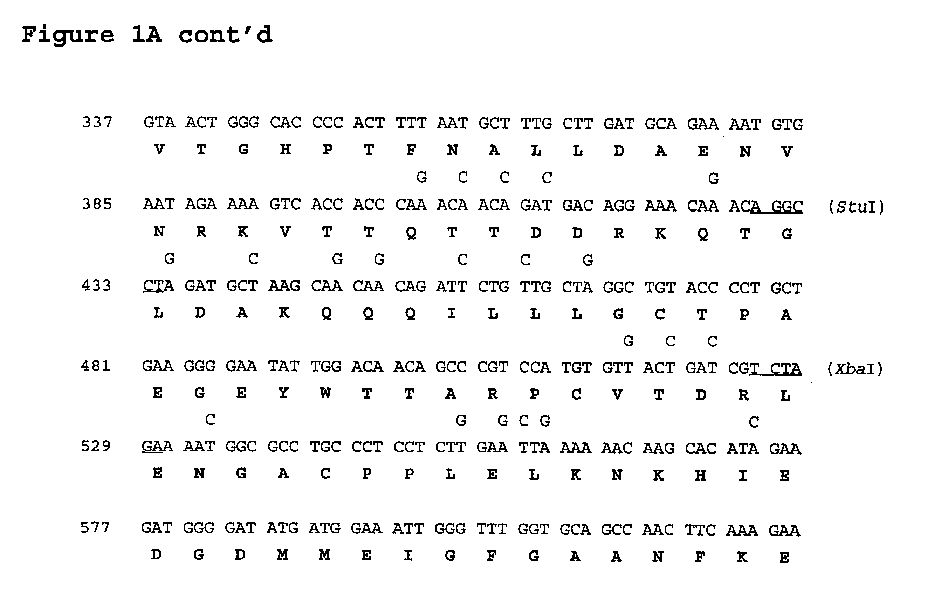

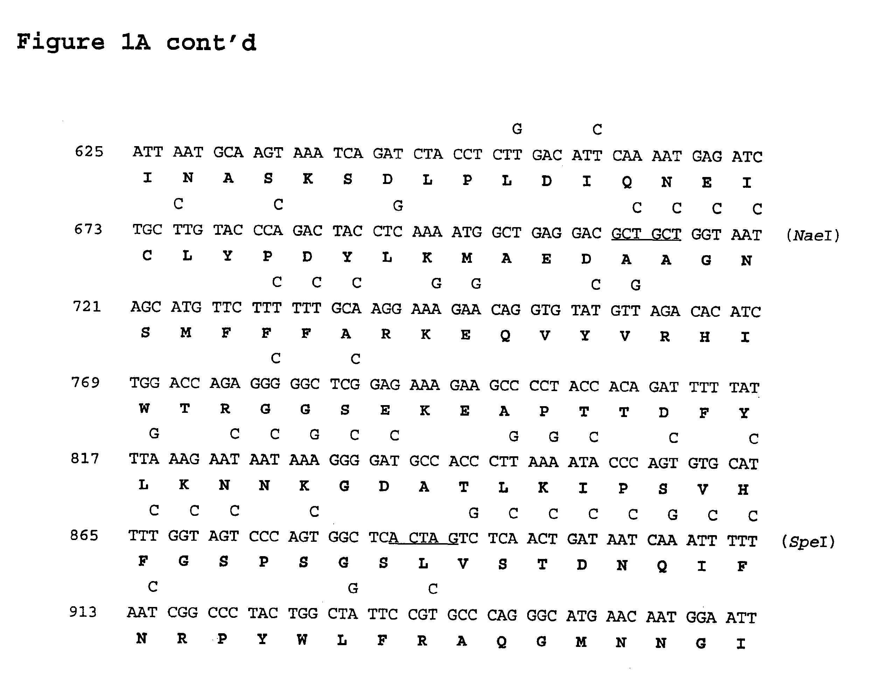

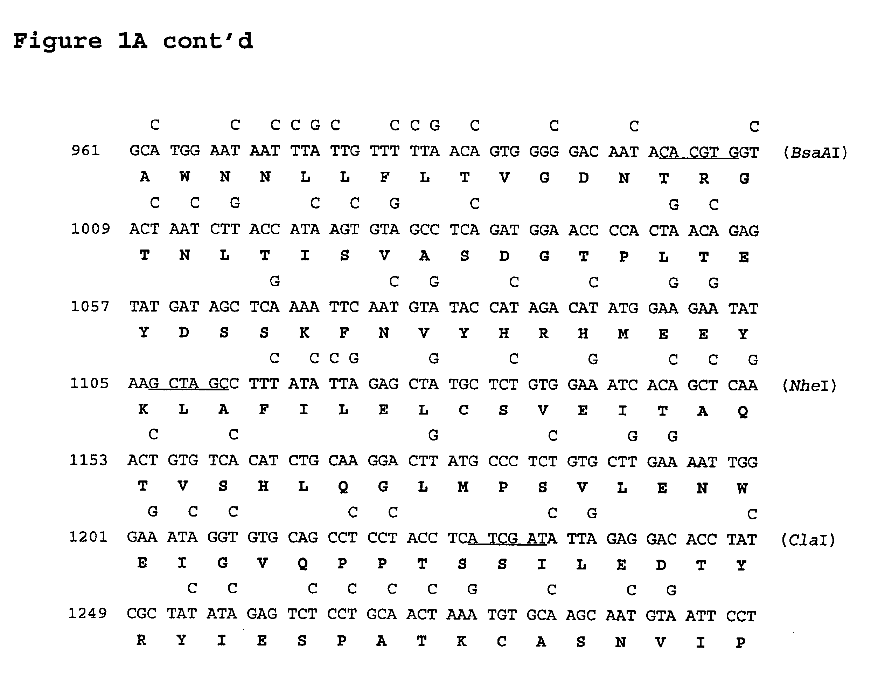

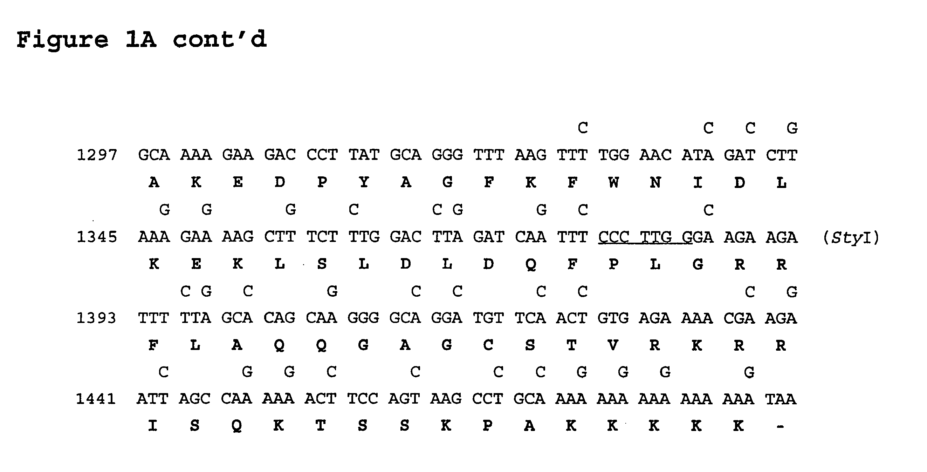

Figure 1A depicts the nucleotide sequence (SEQ ID NO:1) and deduced amino acid sequence (SEQ ID NO:2) of BPV1 L1. Amino acids (in single letter code) are presented below the second nucleotide of each codon. Mutations introduced into the genes are indicated above the corresponding nucleotides of the original sequence. Horizontal lines indicate the sites and enzymes used for cloning. This replacement of nucleotides resulted in a nucleic acid sequence encoding BPV-1 L1 polypeptide with an amino acid sequences identical to the wild type, but having synonymous codons that are frequently used by mammalian genes.

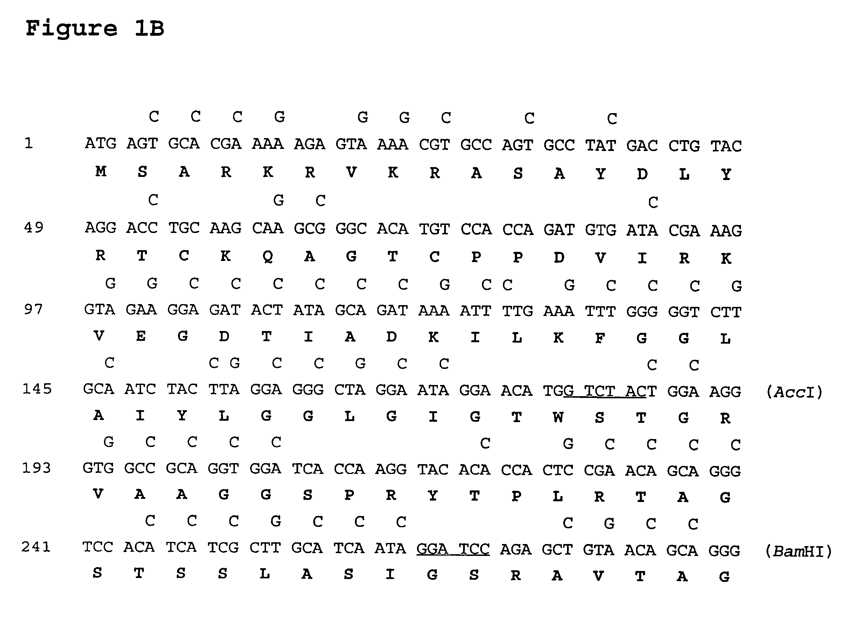

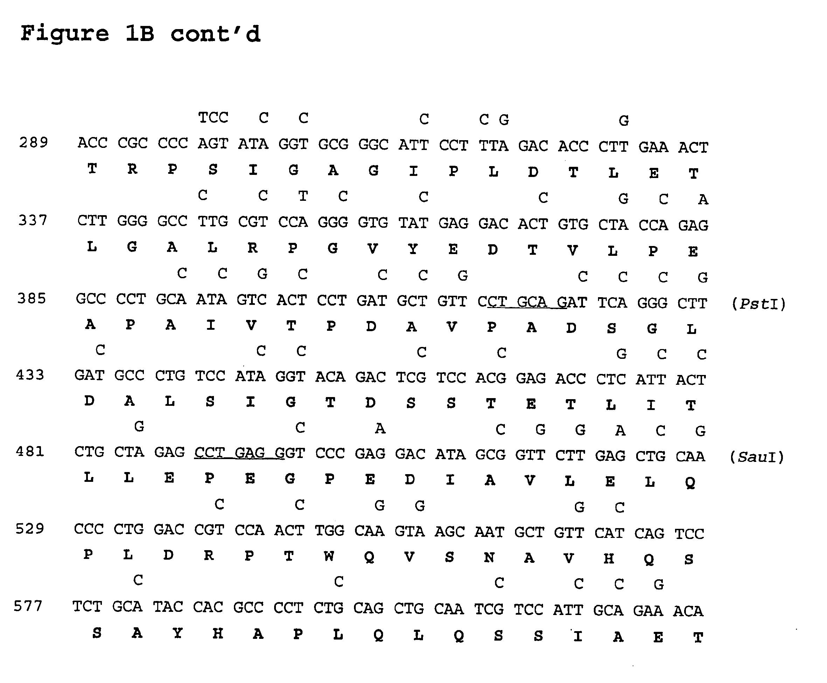

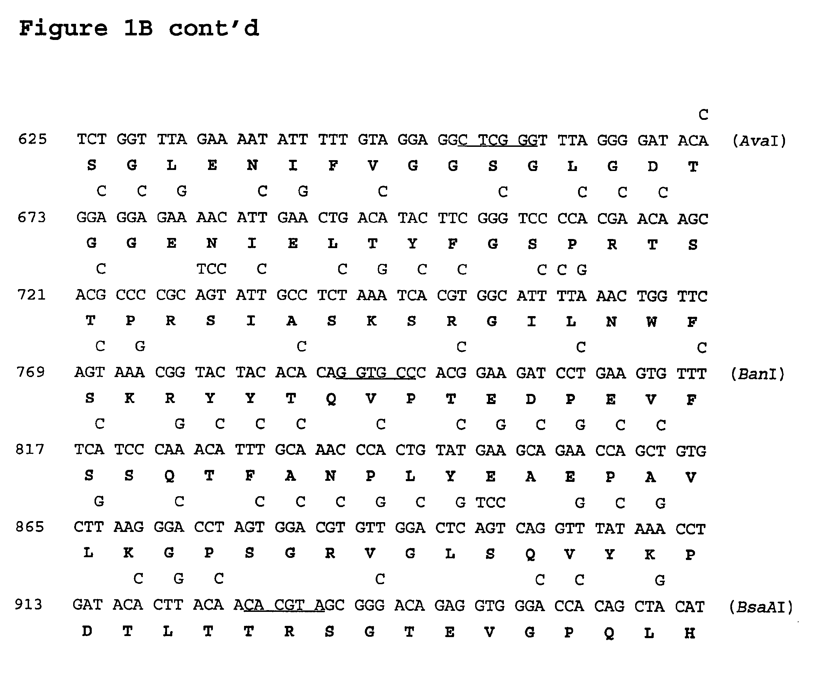

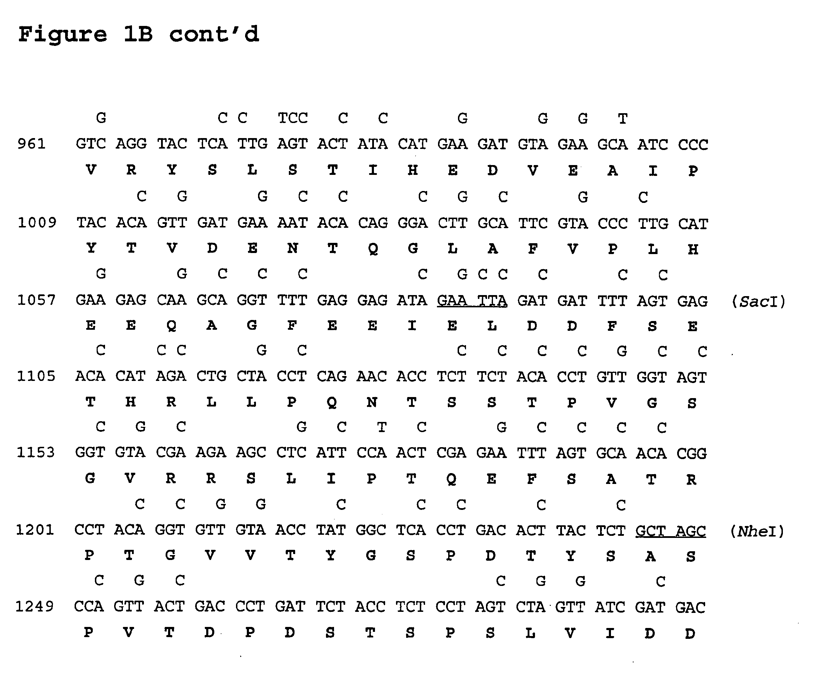

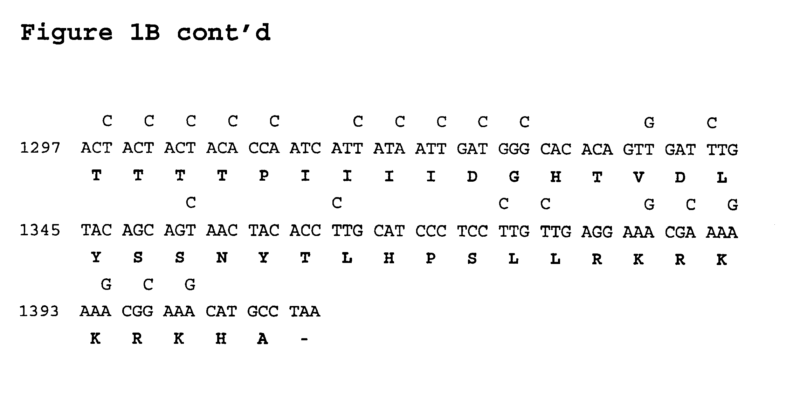

Figure 1B shows the nucleotide sequence (SEQ ID NO:5) and deduced amino acid sequence (SEQ ID NO:6) relating to BPV1 L2 ORF. Amino acids (in single letter code) are presented below the second nucleotide of each codon. Mutations introduced into the genes are indicated above the corresponding nucleotides of the original sequence. Horizontal lines indicate the sites and enzymes used for cloning. This replacement of nucleotides resulted in a nucleic acid sequence encoding BPV-1 L2 polypeptide with an amino acid sequences identical to the wild type, but having synonymous codons that are frequently used by mammalian genes.

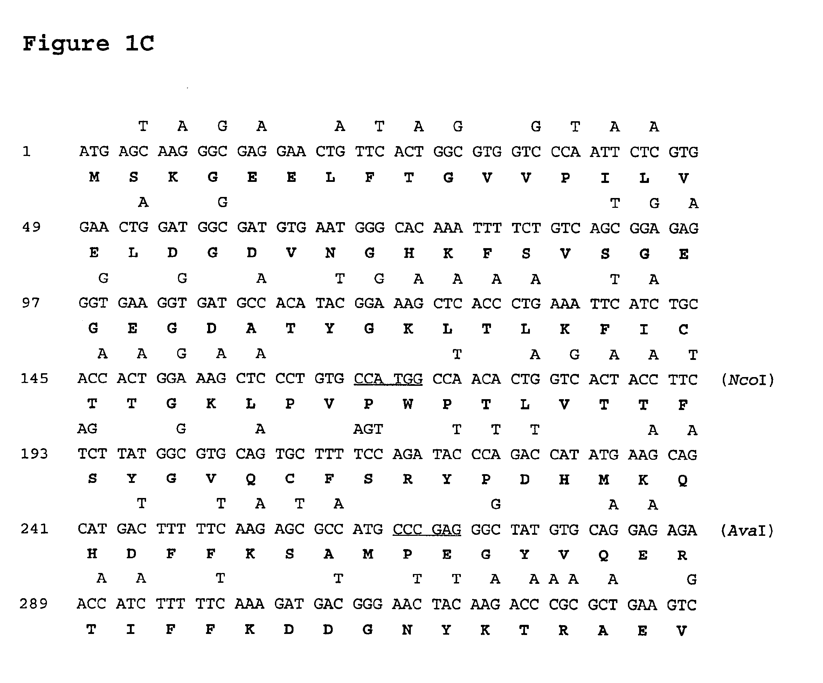

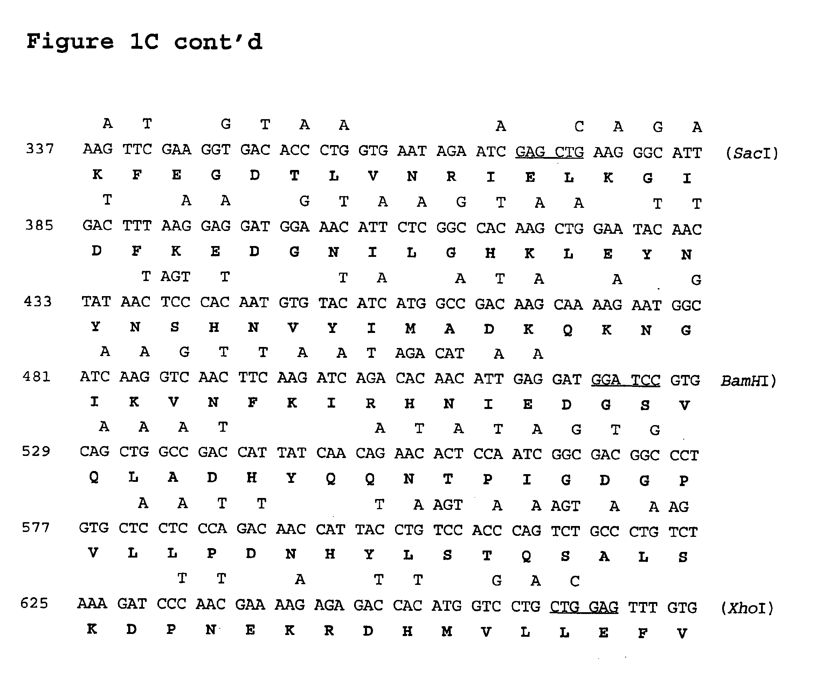

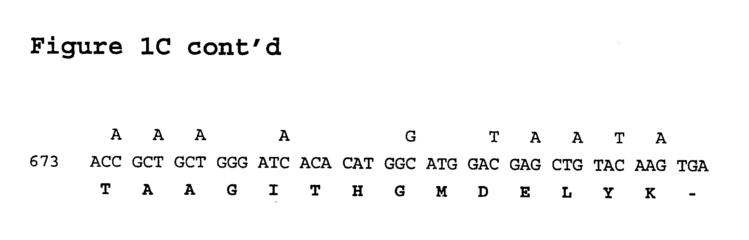

Figure 1C depicts the nucleotide sequence (SEQ ID NO:9) and deduced amino acid sequence (SEQ ID NO:10) of green fluorescent protein (GFP). Amino acids (in single letter code) are presented below the second nucleotide of each codon. Mutations introduced into the genes are indicated above the corresponding nucleotides of the original sequence. Horizontal lines indicate the sites and enzymes used for cloning. This replacement of nucleotides resulted in a nucleic acid sequence encoding GFP polypeptide with an amino acid sequence identical to the native sequence modified for optimal expression in eukaryotic cells, but having synonymous codons that are frequently used by papillomavirus genes.

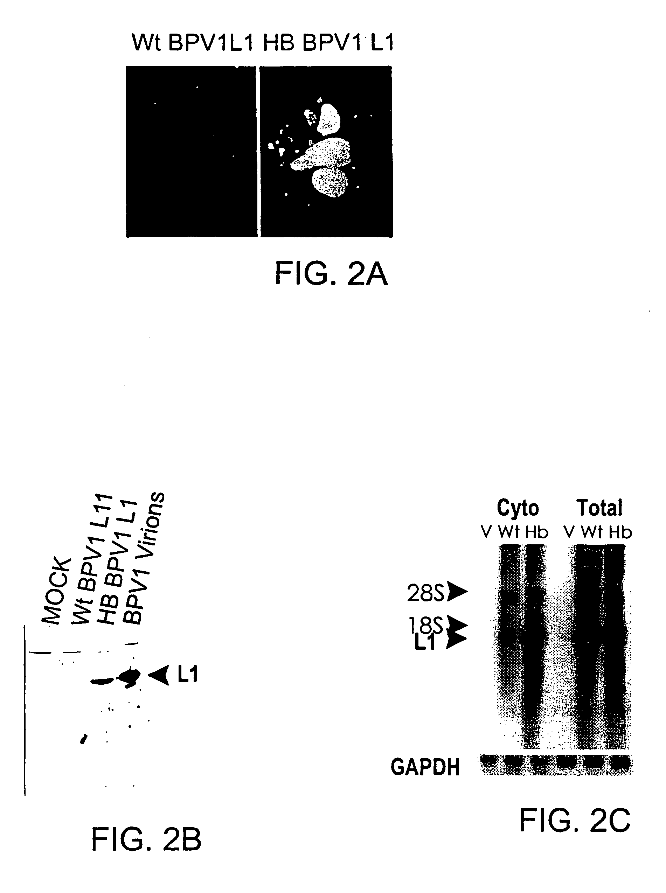

Figure 2A shows detection of L1 protein expressed from synthetic and wild type BPV1 L1 genes. Cos-1 cells were transfected with a synthetic L1 expression plasmid pCDNA/HBL1, and a wild type L1 expression plasmid pCDNA/BPVL1wt. The expression of L1 was detected by immunofluorescent staining. Cells were fixed after 36 hrs and incubated with rabbit anti-BPV1 L1 antiserum, followed by FITC-conjugated goat anti-rabbit IgG antibody.

Figure 2B shows detection by Western blot of L1 protein from Cos-1 cells transfected with pCDNA/HBL1 and pCDNA/BPVL1wt.

Figure 2C shows a Northern blot in which L1 mRNA extracted from transfected cells was probed with ³²P-labeled probes produced from wild type L1 sequence. The amount of mRNA loaded in respective lanes was examined by hybridization of the mRNA sample with a gapdh probe.

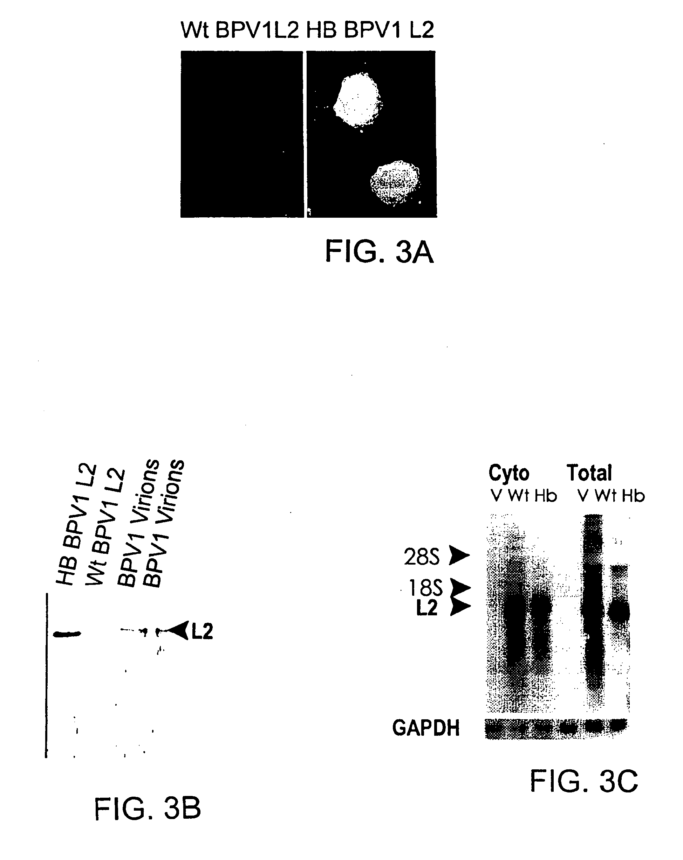

Figure 3A shows detection of L2 protein expressed from synthetic and wild type BPV1 L2 genes. Cos-1 cells were transfected with a synthetic L2 expression plasmid pCDNA/HBL2, and a wild type L2 expression plasmid pCDNA/BPVL2wt. The expression of L2 was detected by immunofluorescent staining. Cells were fixed after 36 hrs and incubated with rabbit anti-BPV1 L2 antiserum, followed by FITC-conjugated goat anti-rabbit IgG antibody.

Figure 3B shows detection by Western blot of L2 protein from Cos-1 cells transfected with pCDNA/HBL2 and pCDNA/BPVL2wt.

Figure 3C shows a Northern blot in which L2 mRNA extracted from transfected cells was probed with ³²P-labeled probes produced from wild type L2 sequence. The amount of mRNA loaded in respective lanes was examined by hybridization of the mRNA sample with a *gapdh* probe.

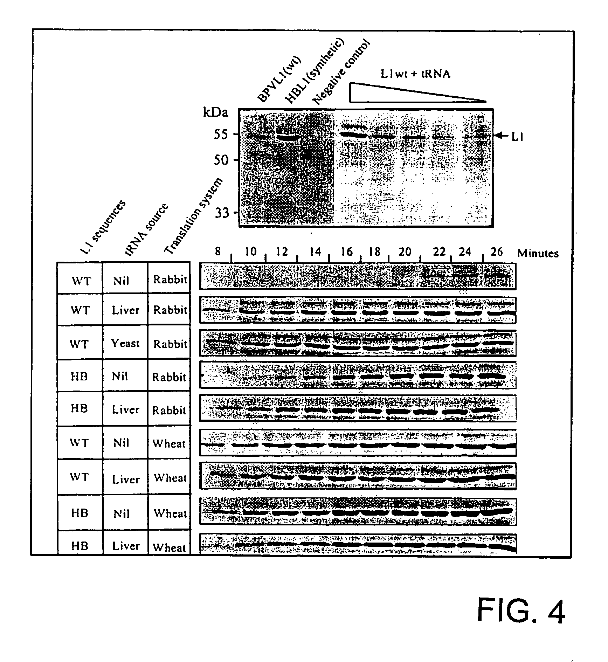

Figure 4 shows *in vitro* translation of BPVL1 sequences, wild type BPVL1 (wt) or synthetic L1 (HB) using rabbit reticulocyte lysate or wheat germ extract in the presence of ³⁵S-methionine. In the top panel, wt L1 or HB L1 plasmid DNA was added to the T7 DNA polymerase-coupled *in vitro* translation system. L1 protein was detected by Western blot analysis. In the bottom panel, the translation efficiency of wt L1 or HB L1 sequences in the presence or absence of tRNA was compared. Translation was carried out in rabbit reticulocyte lysate (rabbit) or wheat germ extract (wheat), and samples were collected every two minutes starting from minute 8. Left side of lower panel indicates if 10⁻⁵ M bovine liver or yeast tRNA was supplied.

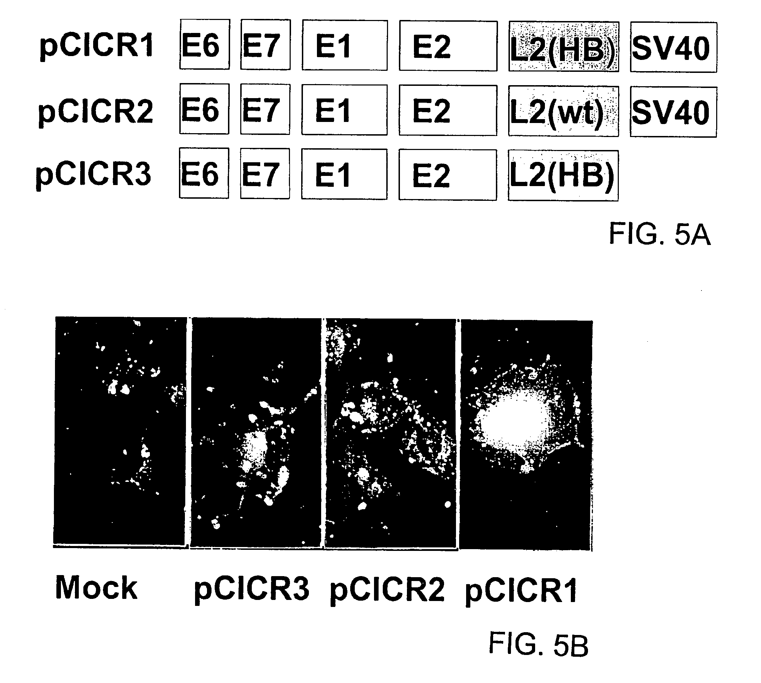

Figure 5A is a schematic representation of plasmids used to determine L2 expression from BPV cryptic promoter(s). The wild type L1 sequence and most of the wild type L2 sequence were deleted from the BPV1 genome by BamHI and *Hin*dIII digestion and the remaining BPV1 sequence (in yellow) was cloned into pUC18. Wild type or synthetic humanized L2 sequences (in red) were inserted into the *Bam*HI site of the BPV1 genome. The position of the inserted SV40 *ori* sequence (in white) is indicated. The plasmid in which modified L2 was used but without SV40 *ori* sequence was also used as a control. The plasmids were transfected into Cos-1 cells and the expression of L2 protein was determined using BPV1 L2-specific polyclonal antiserum followed by FITC-linked anti rabbit IgG.

Figure 5B shows expression of L2 protein from native papillomavirus promoter. The plasmids shown in Figure 5A were used to transfect Cos-1 cells and the expression of L2 protein was determined using BPV1 L2-specific polyclonal antiserum followed by FITC-linked anti rabbit IgG. A mock transfection in which the cells did not receive plasmid was used as control.

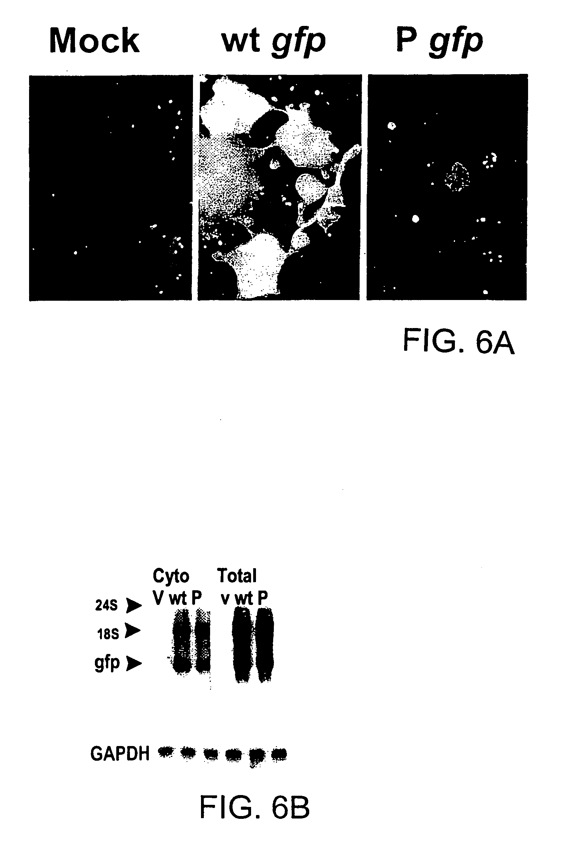

Figure 6 shows expression of GFP in Cos-1 cells transfected with wild-type *gfp* (wt) or a synthetic *gfp* gene carrying codons used at relatively high frequency by papillomavirus genes (p). The mRNA extracted from cells transfected with *gfp* or P *gfp* was probed with ³²P-labeled *gfp* probe and is shown on the right panel, using *gapdh* as a reference gene.

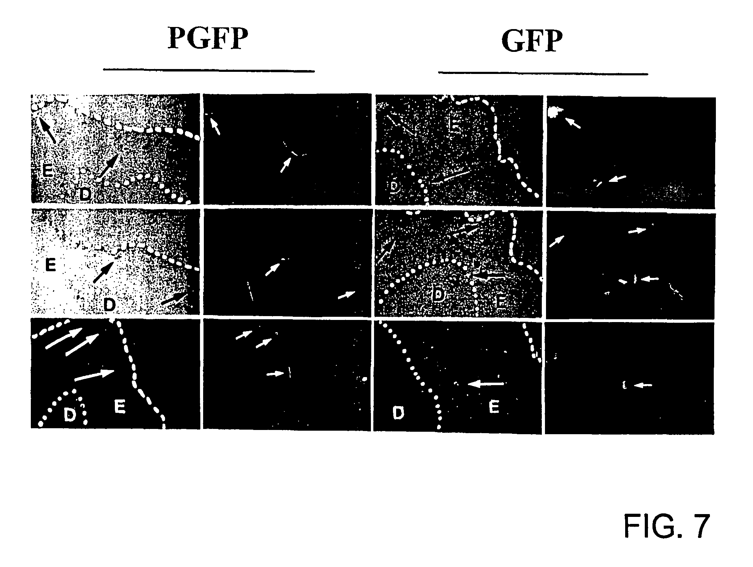

Figure 7 shows the expression pattern of GFP *in vivo* from wild-type *gfp* gene, or a synthetic gfp gene carrying codons used at relatively high frequency by papillomavirus genes. Using a gene gun, mice were shot with PGFP (left panel) and GFP (right panel) expression plasmids encoding GFP protein. A transverse section of the mouse skin section shows where the *gfp* gene is expressed. Bright-field photographs of the same section where dermis (D) epidermis (E) are highlighted are shown to identify the location of fluorescence in the epidermis. Arrows indicate fluorescent signals.

### DETAILED DESCRIPTION

The present invention arises from the unexpected discovery that the relative abundance of different isoaccepting transfer RNAs varies in different cells or tissues, or alternatively in cells or tissues in different states of differentiation or in different stages of the cell cycle, and that such differences may be exploited together with codon composition of a gene to regulate and direct expression of a protein to a particular cell or tissue, or alternatively to a cell or tissue in a specific state of differentiation or in a specific stage of the cell cycle. According to the present invention, this selective targeting is effected by replacing at least one existing codon of a parent nucleic acid sequence encoding the protein with a synonymous codon.

Replacement of synonymous codons for existing codons is not new *per se*. In this regard, we refer to International Application Publication No WO 96/09378 which utilizes such substitution to provide a method of expressing proteins of eukaryotic and viral origin at high levels in *in vitro* mammalian cell culture systems, the main thrust of the method being the harvesting of such proteins. In distinct contrast, the present invention utilizes substitution of one or more codons in a gene for targeting expression of the gene to particular cells or tissues with the ultimate aim of facilitating gene therapy as described herein.

The term "synonymous codon" as used herein refers to a codon having a different nucleotide sequence to an existing codon but encoding the same amino acid as the existing codon.

By "isoaccepting transfer RNA" is meant one or more transfer RNA molecules that differ in their anticodon structure but are specific for the same amino acid.

Throughout this specification, unless the context requires otherwise, the words "comprise", comprises" and "comprising" will be understood to imply the inclusion of a stated integer or group of integers but not the exclusion of any other integer or group of integers.

### Selection of synonymous codons

### Determination of relative abundance of different tRNA species in different cells

Advantageously, the synonymous codon corresponds to an iso-tRNA (iso-tRNA) which, when compared to an iso-tRNA corresponding to the at least one existing codon, is in higher abundance in the target cell or tissue relative to one or more other cells or tissues of the mammal.

Any method for determining the relative abundance of an iso-tRNA in two or more cells or tissues may be employed. For example, such method may include isolating two or more particular cells or tissues from a mammal, preparing an RNA extract from each cell or tissue which extract includes tRNA, and probing each extract respectively with different nucleic acid sequences each being specific for a particular iso-tRNA to determine the relative abundance of an iso-tRNA between the two or more cells or tissues.

Suitable methods for isolating particular cells or tissues are well known to those of skill in the art. For example, one can take advantage of one or more particular characteristics of a cell or tissue to specifically isolate the cell or tissue from a heterogeneous population. Such characteristics include, but are not limited to, anatomical location of a tissue, cell density, cell size, cell morphology, cellular metabolic activity, cell uptake of ions such as Ca²⁺, K⁺, and H⁺ ions, cell uptake of compounds such as stains, markers expressed on the cell surface, cytokine expression, protein fluorescence, and membrane potential. Suitable methods that may be used in this regard include surgical removal of tissue, flow cytometry techniques such as fluorescence-activated cell sorting (FACS), immunoaffinity separation (e.g., magnetic bead separation such as Dynabead™ separation), density separation (e.g., metrizamide, Percoll™, or Ficoll™ gradient centrifugation), and cell-type specific density separation (*e.g.*, Lymphoprep^{™}). For example, dividing cells or blast cells may be separated from non-dividing cells or resting cells according to cell size by FACS or metrizamide gradient separation.

Any suitable method for isolating total RNA from a cell or tissue may be used. Typical procedures contemplated by the invention are described in CURRENT PROTOCOLS IN MOLECULAR BIOLOGY (Ausubel, et al., eds) (John Wiley & Sons, Inc. 1997), at page 4.2.1 through page 4.2.7. Preferably, techniques which favor isolation of tRNA are employed as, for example, described in Brunngraber, E.F. (1962, Biochem. Biophys. Res. Commun. 8:1-3).

The probing of an RNA extract is suitably effected with different oligonucleotide sequences each being specific for a particular iso-tRNA. Of course it will be appreciated that for a given mammal, oligonucleotide sequences would need to be selected which hybridize specifically with particular iso-tRNA sequences expressed by the mammal. Such selection is well within the realm of one of ordinary skill in the art based a known iso-tRNA sequence. For example, in the case of a mouse, exemplary oligonucleotide sequences which may be used include those described in Gauss and Sprinzel (1983, Nucleic Acids Res. 11 (1)). In this respect, the oligonucleotide sequences may be selected from the group consisting of:
5'-TAAGGACTGTAAGACTT-3' (SEQ ID NO:13) for Ala^{GCA}
5'-CGAGCCAGCCAGGAGTC-3' (SEQ ID NO:14) for Arg^{CGA}
5'-CTAGATTGGCAGGAATT-3' (SEQ ID NO:15) for Asn^{AAC}
5'-TAAGATATATAGATTAT-3' (SEQ ID NO:16) for Asp^{GAC}
5'-AAGTCTTAGTAGAGATT-3' (SEQ ID NO:17) for Cys^{TGC}
5'-TATTTCTACACAGCATT-3' (SEQ ID NO:18) for Glu^{GAA}
5'-CTAGGACAATAGGAATT-3' (SEQ ID NO:19) for Gln^{CAA}
5'-TACTCTCTTCTGGGTTT-3' (SEQ ID NO:20) for Gly^{GGA}
5'-TGCCGTGACTCGGATTC-3' (SEQ ID NO:21) for His^{CAC}
5'-TAGAAATAAGAGGGCTT-3' (SEQ ID NO_{:}22) for Ile^{ATC}
5'-TACTTTTATTTGGATTT-3' (SEQ ID NO:23) for Leu^{CTA}
5'-TATTAGGGAGAGGATTT-3' (SEQ ID NO:24) for Leu^{CTT}
5'-TCACTATGGAGATTTTA-3' (SEQ ID NO:25) for Lys^{AAA}
5'-CGCCCAACGTGGGGCTC-3' (SEQ ID NO:26) for Lys^{AAG}
5'-TAGTACGGGAAGGATTT-3' (SEQ ID NO:27) for Met^{elong}
5'-TGTTTATGGGATACAAT-3' (SEQ ID NO:28) for Phe^{TTC}
5'-TCAAGAAGAAGGAGCTA-3' (SEQ ID NO:29) for Pro^{CCA}
5'-GGGCTCGTCCGGGATTT-3' (SEQ ID NO:30) for Pro^{CCI}
5'-ATAAGAAAGGAAGATCG-3' (SEQ ID NO:31) for Ser^{AGC}
5'-TGTCTTGAGAAGAGAAG-3' (SEQ ID NO:32) for Thr^{ACA}
5'-TGGTAAAAAGAGGATTT-3' (SEQ ID NO:33) for Tyr^{TAC}
5'-TCAGAGTGTTCATTGGT-3' (SEQ ID NO:34) for Val^{GTA}

Typically, the relative abundance of iso-tRNA species may be determined by blotting techniques that include a step whereby sample RNA or tRNA extract is immobilized on a matrix (preferably a synthetic membrane such as nitrocellulose), a hybridization step, and a detection step. Northern blotting may be used to identify an RNA sequence that is complementary to a nucleic acid probe. Alternatively, dot blotting and slot blotting can be used to identify complementary DNA/RNA or RNA/RNA nucleic acid sequences. Such techniques are well known by those skilled in the art, and have been described in Ausubel, et al (supra) at pages 2.9.1 through 2.9.20.

According to such methods, a sample of tRNA immobilized on a matrix is hybridized under stringent conditions to a complementary nucleotide sequence (such as those mentioned above) which is labeled, for example, radioactively, enzymatically or fluorochromatically.

"Stringency" as used herein, refers to the temperature and ionic strength conditions, and presence or absence of certain organic solvents, during hybridization. The higher the stringency, the higher will be the degree of complementarity between the immobilized nucleotide sequences (*i.e.*, iso-tRNA) and the labeled oligonucleotide sequence. For a discussion of typical stringent conditions that may be used, see CURRENT PROTOCOLS IN MOLECULAR BIOLOGY supra at pages 2.10.1 to 2.10.16, and Sambrook et al in MOLECULAR CLONING. A LABORATORY MANUAL (Cold Spring Harbor Press, 1989), at sections 1.101 to 1.104.

While stringent washes are typically carried out at temperatures from about 42°C to 68°C, one skilled in the art will appreciate that other temperatures may be suitable for stringent conditions. Maximum hybridization typically occurs at about 20° to 25° below the Tₘ for formation of a DNA-DNA hybrid. It is well known in the art that the Tₘ is the melting temperature, or temperature at which two complementary nucleic acid sequences dissociate. Methods for estimating Tₘ are well known in the art (see CURRENT PROTOCOLS IN MOLECULAR BIOLOGY supra at page 2.10.8). Maximum hybridization typically occurs at about 10° to 15° below the Tₘ for a DNA-RNA hybrid.

Other stringent conditions are well known in the art. A skilled addressee will recognize that various factors can be manipulated to optimize the specificity of the hybridization. Optimization of the stringency of the final washes can serve to ensure a high degree of hybridization.

Methods for detecting labeled nucleotide sequences hybridized to an immobilized nucleotide sequence are well known to practitioners in the art. Such methods include autoradiography, chemiluminescent, fluorescent and colorimetric detection.

Advantageously, the relative abundance of an iso-tRNA in two or more cells or tissues may be determined by comparing the respective levels of binding of a labeled nucleotide sequence specific for the iso-tRNA to equivalent amounts of immobilized RNA obtained from the two or more cells or tissues. Similar comparisons are suitably carried out to determine the respective relative abundance of other iso-tRNAs in the two or more cells or tissues. One of ordinary skill in the art will thereby be able to determine a relative tRNA abundance table (see for example TABLE 2) for different cells or tissues. From such comparisons, one or more synonymous codons may be selected such that the or each synonymous codon corresponds to an iso-tRNA which, when compared to an iso-tRNA corresponding to an existing codon of the parent nucleic acid sequence, is in higher abundance in the target cell or tissue relative to other cells or tissues of the mammal.

Advantageously, a synonymous codon is selected such that its corresponding iso-tRNA in the target cell or tissue is at a level which is at least 110%, preferably at least 200%, more preferably at least 500%, and most preferably at least 1000%, of that expressed in the or each other cell or tissue of the mammal.

Suitably, synonymous codons for selective expression of a protein in a differentiated cell, preferably a differentiated keratinocyte, are selected from the group consisting of gca (Ala), cuu (Leu) and cua (Leu).

Synonymous codons for selective expression of a protein in an undifferentiated cell, preferably an undifferentiated keratinocyte, are suitably selected from the group consisting of cga (Arg), cci (Pro) and aac (Asn).

### Analysis of codon usage

Alternatively, synonymous codons may be selected by analyzing the frequency at which codons are used by genes expressed in (i) particular cells or tissues, (ii) substantially all cells or tissues of the mammal, or (iii) an organism which may infect particular cells or tissues of the mammal.

Codon frequency tables as well as suitable methods for determining frequency of codon usage in an organism are described, for example, in an article by Sharp et al (1988, Nucleic Acids Res. 16 8207-8211).

The relative level of gene expression (*e.g*., detectable protein expression vs no detectable protein expression) can provide an indirect measure of the relative abundance of specific iso-tRNAs expressed in different cells or tissues. For example, a virus may be capable of propagating within a first cell or tissue (which may include a cell or tissue at a specific stage of differentiation) but may be substantially incapable of propagating in a second cell or tissue (which may include a cell or tissue at another stage of differentiation). Comparison of the pattern of codon usage by genes of the virus with the pattern of codon usage by genes expressed in the second cell or tissue may thus provide indirectly a set of synonymous codons which correspond to iso-tRNAs expressed at relatively high abundance in the first cell or tissue relative to the second cell or tissue and *vice versa*. Simultaneously, the above comparison may also provide indirectly a set of synonymous codons which correspond to iso-tRNAs expressed at relatively high abundance in the second cell or tissue relative to the first cell or tissue.

From the foregoing, a synonymous codon according to the invention may correspond to a codon including, but not limited to, (1) a codon used at relatively high frequency by genes, preferably highly expressed genes, of the target cell or tissue, (2) a codon used at relatively high frequency by genes, preferably highly expressed genes, of the or each other cell or tissue, (3) a codon used at relatively high frequency by genes, preferably highly expressed genes, of the mammal, (4) a codon used at relatively low frequency by genes of the target cell or tissue, (5) a codon used at relatively low frequency by genes of the or each other cell or tissue, (6) a codon used at relatively low frequency by genes of the mammal, (7) a codon used at relatively high frequency by genes of another organism, and (8) a codon used at relatively low frequency by genes of another organism.

For example, codons used at a relatively high frequency by genes, preferably highly expressed genes, of the mammal may be selected from the group consisting of: cuc (Leu), cuu, (Leu), cug (Leu), uua (Leu), uug (Leu); cgg (Arg), cgc (Arg), aga (Arg), agg (Arg); agu (Ser), agc (Ser), ucu (Ser), ucc (Ser), and uca (Ser). Alternatively, such codons may include auu (Ile), auc (Ile); guu (Val), guc (Val), gug (Val); acu (Thr), acc (Thr), aca (Thr); gcu (Ala), gcc (Ala), gca (Ala); cag (Glu); ggc (Gly), gga (Gly), ggg (Gly).

Codons used at a relatively low frequency by genes of the mammal are described, for example, in Sharp et al (1988, supra). Such codons may comprise cua (Leu) ; cga (Arg), cgu (Arg) ; ucg (Ser) . Alternatively, such codons may include aua (Ile); gua (Val); acg (Thr); gcg (Ala); caa (Glu); ggu (Gly).

### Construction of synthetic nucleic acid sequences

The step of replacing synonymous codons for existing codons may be effected by any suitable technique. For example, *in vitro* mutagenesis methods may be employed which are well known to those of skill in the art. Suitable mutagenesis methods are described for example in the relevant sections of Ausubel, *et al.* (*supra*) and of Sambrook, *et al.*, (supra). Alternatively, suitable methods for altering DNA are set forth, for example, in U.S. Patent Nos 4, 184, 917, 4,321,365 and 4, 351, 901. Instead of *in vitro* mutagenesis, the second nucleic acid sequence may be synthesized *de novo* using readily available machinery. Sequential synthesis of DNA is described, for example, in U.S. Patent No 4,293,652. However, it should be noted that the present invention is not dependent on and not directed to any one particular technique for replacing synonymous codons for existing codons.

It is not necessary to replace all the existing codons of the parent nucleic acid sequence with synonymous codons each corresponding to a iso-tRNA expressed in relatively high abundance in the target cell compared to other cells. Increased expression may be accomplished even with partial replacement. Preferably, the replacing step affects 5%, 10%, 15%, 20%, 25%, 30%, more preferably 35%, 40%, 50%, 60%, 70% or more of the existing codons of the parent nucleic acid sequence.

The parent nucleic acid sequence is preferably a natural gene. By "natural gene" is meant a gene that naturally encodes the protein. However, it is possible that the parent nucleic acid sequence encodes a protein that is not naturally-occurring but has been engineered using recombinant techniques.

The parent nucleic acid sequence need not be obtained from the mammal but may be obtained from any suitable source such as from a eukaryotic or prokaryotic organism. For example, the parent nucleic acid sequence may be obtained from another mammal or other animal. Alternatively, the parent nucleic acid sequence may be obtained from a pathogenic organism. In such a case, a natural host of the pathogenic organism is preferably a mammal. For example, the pathogenic organism may be a yeast, bacterium or virus.

For example, suitable proteins which may be used for selective expression in accordance with the invention include, but are not limited to the cystic fibrosis transmembrane conductance regulator (CFTR) protein, and adenosine deaminase (ADA). In the case of CFTR, a parent nucleic acid sequence encoding the CFTR protein which may be utilized to produce the synthetic nucleic acid sequence is described, for example, in Riordan et al (1989, Science 245 1066-1073), and in the GenBank database under Accession No. HUMCFTRM.

The term "nucleic acid sequence" as used herein designates mRNA, RNA, cRNA, cDNA or DNA.

Regulatory nucleotide sequences which may be utilized to regulate expression of the synthetic nucleic acid sequence include, but are not limited to, a promoter, an enhancer, and a transcriptional terminator. Such regulatory sequences are well known to those of skill in the art.

Synthetic nucleic acid sequences according to the invention may be operably linked to one or more regulatory sequences in the form of an expression vector. By "vector" is meant a nucleic acid molecule, preferably a DNA molecule derived, for example, from a plasmid, bacteriophage, or mammalian or insect virus, into which a synthetic nucleic acid sequence may be inserted or cloned. A vector preferably contains one or more unique restriction sites and may be capable of autonomous replication in a defined host cell including the target cell or tissue or a precursor cell or precursor tissue thereof, or be integratable with the genome of the defined host such that the cloned sequence is reproducible. Thus, by "expression vector" is meant any autonomous element capable of directing the synthesis of a protein. Such expression vectors are well known by practitioners in the art.

The term "precursor cell" as used herein refers to a cell that gives rise to the target cell.

The invention also contemplates synthetic nucleic acid sub-sequences encoding desired portions of the protein. A nucleic acid sub-sequence encodes a domain of the protein having a function associated therewith and preferably encodes at least 10, 20, 50, 100, 150, or 500 contiguous amino acids of the protein.

The step of introducing the synthetic nucleic acid sequence into a target cell will differ depending on the intended use and or species, and may involve non-viral and viral vectors, cationic liposomes, retroviruses and adenoviruses such as, for example, described in Mulligan, R.C., (1993 Science 260 926-932). Such methods may include:
(i) Local application of the synthetic nucleic acid sequence by injection (Wolff et al., 1990, Science 247 1465-1468. surgical implantation, instillation or any other means. This method may also be used in combination with local application by injection, surgical implantation, instillation or any other means, of cells responsive to the protein encoded by the synthetic nucleic acid sequence so as to increase the effectiveness of that treatment. This method may also be used in combination with local application by injection, surgical implantation, instillation or any other means, of another factor or factors required for the activity of said protein.
(ii) General systemic delivery by injection of DNA, (Calabretta et al., 1993, Cancer Treat. Rev. 19 169-179,), or RNA, alone or in combination with liposomes (Zhu et al., 1993, Science 261 209-212,), viral capsids or nanoparticles (Bertling et al., 1991, Biotech. Appl. Biochem. 13 390-405,) or any other mediator of delivery. Improved targeting might be achieved by linking the synthetic nucleic acid sequence to a targeting molecule (the so-called "magic bullet" approach employing for example, an antibody), or by local application by injection, surgical implantation or any other means, of another factor or factors required for the activity of the protein produced from said synthetic nucleic acid sequence, or of cells responsive to said protein.
(iii) Injection or implantation or delivery by any means, of cells that have been modified *ex vivo* by transfection (for example, in the presence of calcium phosphate: Chen et al., 1987, Mole. Cell Biochem. 7 2745-2752, or of cationic lipids and polyamines: Rose et al., 1991, BioTech. 10 520-525,), infection, injection, electroporation (Shigekawa et al., 1988, BioTech. 6 742-751,) or any other way so as to increase the expression of said synthetic nucleic acid sequence in those cells. The modification may be mediated by plasmid, bacteriophage, cosmid, viral (such as adenoviral or retroviral; Mulligan, 1993, Science 260 926-932; Miller, 1992, Nature 357 455-460; Salmons et al., 1993, Hum. Gen. Ther. 4 129-141,) or other vectors, or other agents of modification such as liposomes (Zhu et al., 1993, Science 261 209-212,), viral capsids or nanoparticles (Bertling et al., 1991, Biotech. Appl. Biochem. 13 390-405,), or any other mediator of modification. The use of cells as a delivery vehicle for genes or gene products has been described by Barr et al., 1991, Science 254 1507-1512 and by Dhawan et al., 1991, Science 254 1509-1512. Treated cells may be delivered in combination with any nutrient, growth factor, matrix or other agent that will promote their survival in the treated subject.

A pharmaceutical composition comprising the synthetic nucleic sequences of the invention and a pharmaceutically acceptable carrier is also disclosed.

By "pharmaceutically-acceptable carrier" is meant a solid or liquid filler, diluent or encapsulating substance that may be safely used in systemic administration. Depending upon the particular route of administration, a variety of pharmaceutically acceptable carriers, well known in the art may be used. These carriers may be selected from a group including sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water.

Any suitable technique may be employed for determining expression of the protein from said synthetic nucleic acid sequence in a particular cell or tissue. For example, expression can be measured using an antibody specific for the protein of interest or portion thereof. Such antibodies and measurement techniques are well known to those skilled in the art.

### Applications

In one embodiment of the present invention, the target cell is suitably a differentiated cell. Advantageously, the protein which is desired to be selectively expressed in the differentiated cell is not expressible in a precursor cell thereof (such as an undifferentiated or less differentiated cell of the mammal) from a parent nucleic acid sequence at a level sufficient to effect a particular function associated with said protein. In this embodiment, the step of replacing at least one existing codon with a synonymous codon is characterized in that the synonymous codon corresponds to an iso-tRNA which, when compared to the iso-tRNA corresponding to the at least one existing codon, is in relatively higher abundance in the differentiated cell compared to the precursor cell. Accordingly, a synthetic nucleic acid sequence is produced having altered translational kinetics compared to the parent nucleic acid sequence wherein the protein is expressible in the differentiated cell at a level sufficient to effect a particular function associated with said protein, but wherein the protein is not expressible in the precursor cell at a level sufficient to effect said function.

As used herein, the term "function" refers to a biological, or therapeutic function.

The above embodiment may be utilized advantageously for somatic gene therapy where overexpression of a protein in undifferentiated cells such as stems cells has undesirable consequences including death or differentiation of the stem cells. In such a case, a suitable protein may include cystic fibrosis transmembrane conductance regulator (CFTR) protein, and adenosine deaminase (ADA).

The differentiated cell may comprise a cell of any lineage including a cell of epithelial, hemopoetic or neural origin. For example, the differentiated cell may be a mature differentiated keratinocyte.

### Targeting expression of a protein to progeny of a stem cell but not to the stem cell itself

The synthetic nucleic acid sequence produced above may be transfected directly into the differentiated cell for the desired function or alternatively, transfected into the precursor cell. For example, in the case of ADA deficiency, expression of ADA in stem cells may result in loss of stem phenotype which is undesirable. However, an advantageous therapy may reside in transducing autologous marrow stem cells with a synthetic nucleic acid sequence operably linked to one or more regulatory sequences, wherein existing codons of the wild type ADA gene have been replaced with synonymous codons each corresponding to an iso-tRNA expressed in relatively high abundance in differentiated lymphocytes compared to the marrow stem cells. The transduced stem cells may then be reinfused into the patient. This approach will result in transduced marrow stem cells which are not capable of expressing ADA themselves, but which are able to give rise to a renewable population of differentiated lymphocytes which are capable of expressing ADA at levels sufficient to permit a therapeutic effect. In this regard, a suitable cell source for this purpose may comprise stem cells isolated as CD34 positive cells from a patient's peripheral blood or marrow. For gene delivery, a suitable vector may include a retrovirus or Adeno associated virus.

Alternatively, in the case of inducing cell mediated immunity, dendritic cells are important antigen presenting cells (APC) but have a very limited life span for antigen presentation once activated of between 14 to 21 days. Consequently, dendritic cells provide relatively short-term immune stimulation that may not be optimal. However, in accordance with the present invention, a long-term immune stimulation may be provided by transducing autologous bone marrow-derived CD34 positive dendritic cell precursors with a synthetic nucleotide sequence encoding an antigen. such as the melanoma antigen MART-1, wherein the synthetic sequence is operably linked to one or more regulatory sequences, and wherein existing codons of a wild type nucleotide sequence encoding MART-1 have been replaced with synonymous codons each corresponding to an iso-tRNA expressed in relatively high abundance in dendritic cells compared to the dendritic cell precursors. The transduced dendritic cell precursors may then be reinfused into the patient. This approach will result in transduced dendritic cell precursors which are not capable of expressing MART-1 themselves, but which are able to give rise to a renewable population of dendritic cells which are capable of expressing MART-1 at levels sufficient to permit a lifelong intermittent restimulation of a cytotoxic T lymphocyte (CTL) response to the MART-1 antigen.

### Targeting expression of a protein to a stem cell but not to progeny of the stem cell

In an alternate embodiment, the target cell may be an undifferentiated cell wherein the protein is not expressible in said undifferentiated cell, from a parent nucleic acid sequence encoding the protein, at a level sufficient to effect a particular function associated with the protein. In such a case, at least one existing codon of the parent nucleic acid sequence is replaced with a synonymous codon corresponding to an iso-tRNA which, when compared to the iso-tRNA corresponding to the at least one existing codon, is in relatively higher abundance in the undifferentiated cell compared to a differentiated cell. This results in a synthetic nucleic acid sequence having altered translational kinetics compared to said parent nucleic acid sequence wherein the protein is expressible in the undifferentiated cell at a level sufficient to effect a particular function associated with the protein, but wherein the protein is not expressible in differentiated cells derived from the undifferentiated cell at a level sufficient to effect said function.

This alternate embodiment may, by way of example, be used to permit expression of a transcriptional regulatory protein which when expressed in a particular undifferentiated cell or stem cell facilitates differentiation of the stem cell along a particular cell lineage. It will be appreciated that in such a case, the regulatory protein is normally expressed from a gene in which the existing codons correspond to iso-tRNAs which are in relatively low abundance in the stem cell compared to other iso-tRNAs and that therefore the protein is not capable of being expressed at levels sufficient for commitment of the stem cell to differentiate along a particular cell lineage. It will also be apparent that such commitment to differentiate along a particular cell lineage may be utilized to prevent production of a particular lineage of cells such as cancer cells.

Alternatively, the method according to this embodiment may be used to express a transcriptional regulatory protein that is involved in the production of a therapeutic agent or agents. Such a protein may include, for example, NF-kappa-B transcription factor p65 subunit (NF-kappa-B p65) which is involved in the production of interleukin-2 (IL-2), interleukin-3 (IL-3) and granulocyte and macrophage colony stimulating factor (GMCSF). NF-kappa-B p65 is encoded naturally by a nucleotide sequence comprising a number of existing codons each corresponding to an iso-tRNA expressed in relatively low abundance in stem cells. Accordingly, such sequence may be used as the parent nucleic acid sequence according to this embodiment. A suitable nucleotide sequence encoding this protein is described, for example, in Lyle et al (1994, Gene 138 265-266) and in the EMBL database under Accession No HSNFKB65A.

A suitable undifferentiated cell which may be utilized in accordance with the present embodiment includes but is not limited to a stem cell, such as a CD34 positive hemopoetic stem cell.

The present embodiment may also be used advantageously for gene therapy where ongoing regulated expression of a transgene is desirable. For example, secure but reversible regulation of fertility is desirable in veterinary practice and in humans. Such regulation may be effected by transducing autologous breast ductal epithelial cells with a synthetic nucleic acid encoding a leutinising hormone (LH) antagonist or a leutinising hormone releasing hormone (LHRH) antagonist under the control of one or more regulatory sequences. The synthetic nucleic acid may be produced by replacing existing codons of a parent nucleic acid with synonymous codons corresponding to iso-tRNAs expressed in relatively high abundance in resting breast ductal epithelial cells compared to differentiated cells arising therefrom. Once the transduced cells are implanted back into the patient, expression may be switched off by oral administration of progestagen, forcing the differentiation of the majority of the stem cells and loss of expression of the antagonist. Once pregnancy is established, the suppression would be self sustaining by the naturally produced progestagen. The iso-tRNA composition of resting and oestrogen drived breast epithelial cells may be established by first obtaining resting cells from reduction mammoplasty, and determining the cellular tRNA composition in the presence and absence of oestrogen. The synthetic nucleic acid sequence may be introduced into autologous resting epithelial cells by cell electroporation *ex vivo*, and the transduced cells may be subsequently transplanted subcutaneously into the patient. Progestagen may be administered as required to reverse regulation of fertility.

### Targeting expression of a toxin to a tumor cell but not to any other cells of the mammal

Many toxins and drugs are available that can kill tumor cells. However, these toxins and drugs are generally toxic for all dividing cells. This problem may be nevertheless ameliorated by establishing the isoacceptor tRNA composition in a tumor clone, and constructing a synthetic toxin gene (*e.g.*, ricin gene) or a synthetic anti-proliferation gene (*e.g.*, the tumor supressor p53) using synonymous codons corresponding to iso-tRNAs expressed at relatively high abundance in the tumor clone compared to normal dividing cells of the mammal. The synthetic gene is then introduced into the patient by suitable means to selectively express the synthetic genes in tumor cells.

Alternatively, a chemotherapy enhancing product gene (i.e., a drug resistance gene e.g., the multi-drug resistance gene) using a codon pattern unlikely to be expressed in the tumor efficiently may be employed.

### Targeting gene therapy to control body fat

*Leptins are proteins known to control* satiety. By analogy with animal data, however, if too much leptin is administered to a patient, leptin-induced starvation might occur. Advantageously, a synthetic gene encoding leptin may be constructed including synonymous codons corresponding to iso-tRNAs expressed at relatively high levels in activated adipocytes compared to non-activated adipocytes. The synthetic gene may then be introduced into the patient by suitable means such that leptin is only expressed substantially in activated adipocytes as opposed to non-activated adipocytes. As body fat turnover diminishes under the influence of leptin reduced appetite, the metabolic activity of the adipocytes falls and the leptin production decreases correspondingly.

### Targeting expression of a protein to a stage of the cell cycle

In another embodiment of the invention, the target cell may be a non-cycling cell. In this case, the protein which is desired to be selectively expressed in the non-cycling cell is expressible in a cycling cell of the mammal from a parent nucleic acid sequence at a level sufficient to effect a particular function associated with the protein. The synonymous codons are selected such that each corresponds to an iso-tRNA which, when compared to the iso-tRNA corresponding to the at least one existing codon, is in higher abundance in the non-cycling cell compared to the cycling cell. Accordingly, a synthetic nucleic acid sequence is produced having altered translational kinetics compared to the parent nucleic acid sequence wherein the protein is expressible in the non-cycling cell at a level sufficient to effect a particular function associated with said protein, but wherein the protein is not expressible in the non-cycling cell to effect said function.

The term "non-cycling cell" as used herein refers to a cell that has withdrawn from the cell cycle and has entered the G0 state. In this state, it is well known that transcription of endogenous genes and protein translation are at substantially reduced levels compared to phases of the cell cycle, namely G1, S, G2 and M.

By "cycling cell" is meant a cell which is in one of the above phases of the cell cycle.

### Expressing a protein in a target cell or tissue by in vivo expression of iso-tRNAs in the target cell or tissue

In another aspect, the invention extends to a method wherein a protein may be selectively expressed in a target cell by introducing into the cell an auxiliary nucleic acid sequence capable of expressing therein one or more isoaccepting transfer RNAs which are not expressed in relatively high abundance in the cell but which are rate limiting for expression of the protein from a parent nucleic acid sequence to a level sufficient for effecting a function associated with the protein. In this embodiment, introduction of the auxiliary nucleic acid sequence in the cell changes the translational kinetics of the parent nucleic acid sequence such that said protein is expressed at a level sufficient to effect a function associated with the protein.

The step of introducing the auxiliary nucleic acid sequence into the target cell or a tissue comprising a plurality of these cells may be effected by any suitable means. For example, analogous methodologies for introduction of the synthetic nucleic acid sequence referred to above may be employed for delivery of the auxiliary nucleic acid sequence into said cycling cell.

### Assembly of virus particles in cells which do not normally permit assembly of virus particles

In yet another aspect, the invention extends to a method for producing a virus particle in a cycling eukaryotic cell. The virus particle will comprise at least one protein necessary for virus assembly, wherein the at least one protein is not expressed in the cell from a parent nucleic acid sequence at a level sufficient to permit virus assembly therein. This method is characterized by replacing at least one existing codon of the parent nucleic acid sequence with a synonymous codon to produce a synthetic nucleic acid sequence having altered translational kinetics compared to the parent nucleic acid sequence such that the at least one protein is expressible from the synthetic nucleic acid sequence in the cell at a level sufficient to permit virus assembly therein. The synthetic nucleic acid sequence so produced is operably linked to one or more regulatory nucleotide sequences and is then introduced into the cell or a precursor cell thereof. The at least one protein is expressed subsequently in the cell in the presence of other viral proteins required for assembly of the virus particle to thereby produce the virus particle.

Advantageously, the synonymous codon corresponds to an iso-tRNA expressed at relatively high level in the cell compared to the iso-tRNAs corresponding to the existing codons.

The cycling cell may be any cell in which the virus is capable of replication. Suitably, the cycling cell is a eukaryotic cell. Preferably, the cycling cell for production of the virus particle is a eukaryotic cell line capable of being grown *in vitro* such as, for example, CV-1 cells, COS cells, yeast or spodoptera cells.

Suitably, the at least one protein of the virus particle are viral capsid proteins. Preferably, the viral capsid proteins comprise L1 and/or L2 proteins of papillomavirus.

The other viral proteins required for assembly of the virus particle in the cell may be expressed from another nucleic acid sequence(s) which suitably contain the rest of the viral genome. In the case of the at least one protein comprising L1 and/or L2 of papillomavirus, said other nucleic acid sequence(s) preferably comprises the papillomavirus genome without the nucleotide sequences encoding L1 and/or L2.

In yet a further aspect of the invention, there is provided a method for producing a virus particle in a cycling cell, said virus particle comprising at least one protein necessary for assembly of said virus particle, wherein said at least one protein is not expressed in said cell from a parent nucleic acid sequence at a level sufficient to permit virus assembly therein, and wherein at least one existing codon of said parent nucleic acid sequence is rate limiting for the production said at least one protein to said level, said method including the step of introducing into said cell a nucleic acid sequence capable of expressing therein an isoaccepting transfer RNA specific for said at least one codon.

### EXAMPLE 1

### Expression of synthetic L1 and L2 protein in undifferentiated cells.

### Materials and Methods

### Codon replacements in the bovine PV (BPV) L1 and L2 genes

The DNA and amino acid sequences of the wild-type L1 (SEQ ID NOS:1,2)and L2 genes (SEQ ID NOS:5,6) are shown respectively in Figures 1A and 1B. To determine whether the presence of rare codons in wild-type L1 (SEQ ID NO:1) and L2 (SEQ ID NO:5) genes (Table 1) inhibited translation, we synthesized the L1 (SEQ ID NO:3) and L2 (SEQ ID NO:7) genes by using synonymous substitutions as shown. To construct the synthetic sequences, we synthesized 11 pairs of oligonucleotides for L1 and 10 pairs of oligonucleotides for L2. Each pair of oligonucleotides has restriction sites incorporated to facilitate subsequent cloning (Figures 1A and 1B). The degenerate oligonucleotides were used to amplify L1 and L2 sequences by PCR using a plasmid with BPV1 genome as the template. The amplified fragments were cut with appropriate enzymes and sequentially ligated to pUC18 vector, producing pUCHBL1 and pUCHBL2. The synthetic L1 (SEQ ID NO:3) and L2 (SEQ ID NO:7) sequences were sequenced and found to be error-free, and then sub-cloned into the mammalian expression vector pCDNA3 containing SV40 *ori* (Invitrogen), giving expression plasmids pCDNA/HBL1 and pCDNA/HBL2. To compare expression of L1 and L2 with that of the original sequences, the wild type L1 (SEQ ID NO:1) and L2 (SEQ ID NO:5) genes were cloned into the pCDNA3 vector, resulting in pCDNA/BPVL1wt and pCDNA/BPVL2wt.

### Immunofluorescence and Western blot staining

For immunoblotting assays, Cos-1 cells in 6-well plates were transfected with 2 µg L1 or L2 expression plasmids using lipofectamine (Gibco). 36 hrs after transfection, cells were washed with 0.15M phosphate buffered 0.9% NaCl (PBS) and lysed in SDS loading buffer. The cellular proteins were separated by 10% SDS PAGE and blotted onto nitrocellulose membrane. The L1 or L2 proteins were identified by electrochemiluminescence (Amersham, UK), using BPV1 L1 (DAKO) or L2-specific (17) antisera. For immunofluorescent staining, Cos-1 cells were grown on 8-chamber slides, transfected with plasmids, and fixed and permeabilised with 85% ethanol 36hr after transfection. The slides were blocked with 5% milk-PBS and probed with L1 or L2-specific antisera, followed by FITC-conjugated anti-rabbit IgG (Sigma). For GFP or PGFP plasmid transfected cells, the cell were fixed with 4% buffered formaldehyde and viewed by epi-fluorescence microscopy.

### Northern blotting

Cos cells transfected with various plasmids were used to extract cytoplasmic or total RNA using the QIAGEN RNeasy mini kit according to the supplier's handbook. Briefly, for cytoplasmic RNA purification, buffer RLN (50 mM Tris, pH 8.0, 140 mM NaCl, 1.5 mM MgCl₂ and 0.5% NP40) was directly added to monolayer cells and cells were lysed in 4 °C for 5 min. After the nuclei were removed by centrifugation, cytoplasmic RNAs were purified by column. For total RNA extraction, the monolayer cells were lysed using buffer RLT supplied by the kit and RNA was purified by spin column. The purified RNAs were separated by 1.5% agarose gel in the presence of formaldehyde. The RNAs were then blotted onto nylon membrane and probed with (a) 1:1 mixed 5'-end labelled L1 wt and HBL1 fragments; (b) 1:1 mixed 5'-end labelled L2 wt and HBL2 fragments; (c) 1:1 mixed 5'end labelled GFP and PGFP fragments or (d) randomly labelled PAGDH fragment. The blots were washed extensively at 65 °C and exposed to X-ray films for three days.

### Results

To test the hypothesis that the codon composition of the genes encoding the L1 and L2 capsid proteins of papillomavirus (PV) contributes to their preferential expression in differentiated epithelial cells, we produced synthetic BPV1 L1 (SEQ ID NO:3) and L2 (SEQ ID NO:7) genes, substituting codons preferentially used in mammalian genes for the codons frequently present in the wild type BPV1 L1 and L2 sequences which are rare in eukaryotic genes (Figures 1A, 1B).

For the L1 gene, a total of 202 base substitutions were made in 196 codons, without changing the encoded amino acid sequence (Figure 1A). This synthetic "humanized" BPV L1 gene (SEQ ID NO:3) was designated HBL1. In a similarly modified BPV1 L2 gene (SEQ ID NO:7) designated HBL2, 303 bases were changed to substitute 290 less frequently used codons with the corresponding preferentially used codons. Using the synthetic HBL1 (SEQ ID NO:3) and HBL2 (SEQ ID NO:7) genes, we constructed two eukaryotic expression plasmids based on pCDNA3, and designated pCDNA/HBL1 and pCDNA/HBL2. Similar expression plasmids, constructed with the wild type BPV1 L1 (SEQ ID NO:1) and BPV1 L2 (SEQ ID NO:5) genes, were designated pCDNA/BPVL1wt and pCDNA/BPVL2wt, respectively. In each of these plasmids the SV40 ori allowed replication in Cos-1 cells, and the L1 or L2 gene was driven by a strong constitutive CMV promoter.

To compare the expression of the synthetic humanized and the wild type BPV1 L1 or BPV1 L2 genes, we separately transfected Cos-1 cells with each of the L1 and L2 plasmids described above. Transfected cells were analyzed for expression of L1 (SEQ ID NO:2,4) or L2 (SEQ ID NO:6,8) protein by immunofluorescence 36 hr after transfection (Figures 2A and 3A). Cells transfected with the pCDNA3 expression plasmid containing the synthetic humanized L1 (SEQ ID NO:3) or L2 (SEQ ID NO:7) genes were observed to produce large amounts of the corresponding protein, while cells transfected with expression plasmids with the wild type L1 (SEQ ID NO:1) or L2 (SEQ ID NO:5) sequences produced no detectable L1 or L2 protein (Figures 2A and 3A, see nuclear staining of L1 and L2 proteins). To compare more accurately the expression of the different L1 and L2 constructs, L1 and L2 protein expression was assessed by immunoblot in Cos-1 cells transfected with the wild type or synthetic humanized BPV1 L1 or L2 pCDNA3 expression constructs (Figures 2B and 3B). Large amounts of immunoreactive L1 and L2 proteins were expressed from the synthetic humanized L1 (SEQ ID NO:3) and L2 (SEQ ID NO:7) sequences, but no L1 or L2 protein was expressed from the wild type L1 and L2 sequences (SEQ ID NO:1,5).

To establish whether the alterations to the primary sequence of the L1 and L2 mRNA which resulted from the codon alterations also affected steady state expression of the corresponding message, mRNA was prepared from Cos-1 cells transfected with the various capsid protein gene constructs. Using GAPDH as an internal standard it was established by Northern blot that two to three times more modified than wild type L1 mRNA, and similar levels of wild type and modified L2 mRNA were present in the cytoplasm of transfected cells (Figures 2C and 3C). The amount of L1 or L2 protein expressed per arbitrary unit of L1 or L2 mRNA was at least 100 fold higher for the humanized gene constructs than for the natural gene constructs.

### EXAMPLE 2

### Papillomavirus late protein translation in vitro

### Materials and Methods

### In vitro translation assay

One microgram of each plasmid was incubated with 20 µCi ³⁵S-methionine (Amersham) and 40 µL T7 coupled rabbit reticulocyte or wheat germ lysates (Promega). Translation was performed at 30 °C and stopped by adding SDS loading buffer. The L1 proteins were separated by 10% SDS PAGE and examined by autoradiography.

### Production of aminoacyl-tRNA

2.5 x 10⁻⁴ M tRNA (Boehringer) was added to a 20 µL reaction containing 10 mM Tris-acetate, pH.7.8, 44 mM KCl, 12 mM MgCl₂, 9 mM -mercaptoethanol, 38 mM ATP, 0.25 mM GTP and 7 µL rabbit reticulocyte extract. The reaction was carried out at 25 °C for 20 min, and 30 µL H₂O was added to the reaction to dilute the tRNAs to 1 x 10⁻⁴ M. The aminoacyl-tRNAs were then aliquoted and stored at -70 °C.

### Results

As the major limitation to expression of the wild type BPV L1 and L2 genes appeared to be translational in our system we wished to test whether this limitation reflected a limited availability of the appropriate tRNA species for gene translation. As transient expression of the synthetic genes within intact cells may be regulated by many factors, we tested our hypothesis in a cell free system using rabbit reticulocyte lysate (RRL) or wheat germ lysate to examine gene translation. Similar amounts of plasmids expressing the wild type or synthetic humanized BPV1 L1 gene were added to a T7-DNA polymerase coupled RRL transcription/translation system in the presence of ³⁵S-methionine. After 20 minutes, translated proteins were separated by SDS PAGE and visualized by autoradiography. Efficient translation of the modified L1 gene was observed (Figure 4, top panel, lane 2), while translation of the wild type BPV1 L1 sequence resulted in a weak 55 kDa L1 band (Figure 4, upper panel, lane 1). We reasoned that although the wild type sequence was not optimized for translation in RRL, some translation would occur as there would be no cellular mRNA species competing for the 'rare' codons present in the wild type L1 sequence. The above data suggest that the observed difference in efficiency of translation of the wild type and synthetic humanized L1 genes is a consequence of limited availability of the tRNAs required for translation of the rare codons present in the wild type gene. We therefore expected that addition of excess tRNA to the in vitro translation system would overcome the inhibition of translation of the wild type L1 gene. To address this question, 10⁻⁵ M aminoacyl-tRNAs from yeast were added into the RRL translation system, and L1 protein synthesis was assessed. Introduction of exogenous tRNAs resulted in a dramatic improvement in translation of the wild type L1 sequence, which now gave a yield of L1 protein comparable to that observed with the synthetic humanized L1 sequence (SEQ ID NO:3) (Figure 4, top panel). Enhancement of translation of the wild type L1 gene (SEQ ID NO:1) by aminoacyl-tRNA was dose-dependent, with an optimum efficiency at 10⁻⁵ M tRNA. As addition of exogenous tRNA improved the yield of L1 protein translated from the wild type L1 gene sequence (SEQ ID NO:1), we assessed the speed of translation of wild type and humanized L1 mRNA. Samples were collected from the translation mixture every 2 minutes, starting at the 8th minute. Translation of L1 (SEQ ID NO:2,4) from the wild type sequence (SEQ ID NO:1) was much slower than from the humanized L1 sequence (SEQ ID NO:3) (Figure 4 bottom panel), and the retardation of translation could be completely overcome by adding exogenous tRNA from commercially available yeast tRNA. Yeast tRNA was chosen in the above analysis because the codon usage in yeast is similar to that of papillomavirus (Table 1). Addition of exogenous tRNA did not significantly improve the translation of the humanized L1 gene (SEQ ID NO:3), indicating that this sequence was optimized with regard to codon usage for the rabbit reticulocyte translation machinery (Figure 4, bottom panel). In separate experiments we established that wt L1 translation could also be enhanced by liver tRNA (Figure 4), and by tRNAs extracted from bovine skin epidermis, which presumably constitutes a mixture of tRNAs from differentiated and undifferentiated cells (data not shown).

### EXAMPLE 3

### Translation of wild type L1 is efficient in wheat germ extract.

To further test our hypothesis that tRNA availability is a determinant of expression of the wild type BPV1 L1 gene (SEQ ID NO:1), we examined the translation of L1 in a cell type in which a quite different set of tRNAs would be available. In a wheat germ translation system, wild type L1 mRNA was translated as efficiently as humanized L1 mRNA, and addition of exogenous aminoacyl-tRNAs did not improve the translation efficiency of either wild type or humanized sequences (Figure 4 bottom panel). This indicated that in wheat germ there are sufficient of the tRNAs which are limiting for translation of wild type L1 sequence in RRL to allow efficient L1 translation.

### EXAMPLE 4

### Modified late genes can be expressed in undifferentiated cells from papillomavirus promoter(s)

While our data presented above indicates that translation is limiting for the production of BPV1 capsid proteins in our test system, these experiments were conducted in systems which are not truly representative of the viral late gene transcription from the BPV genome, in part because the genes were driven by a strong CMV promoter. We therefore wished to establish whether synthetic humanized BPV capsid protein mRNA would be translated more efficiently than the wild type mRNA, if transcribed from the natural BPV1 promoter. This would establish whether translation was indeed one of the limiting factors for expression of BPV1 late genes driven from the natural cryptic late gene promoter in an undifferentiated cell. The BPV genome was cleaved at nt 4450 and 6958 with BamHI/HindIII and the original L1 (nt 4186-5595) and L2 (5068-7095) ORFs were removed. The synthetic humanized L2 gene (SEQ ID NO:7), together with an SV40 ori sequence to allow plasmid replication in eukaryotic cells, were inserted into the BPV genome lacking L1/L2 ORF sequences. This plasmid (Figure 5A) was designated pCICR1. A similar plasmid was constructed with wild type (SEQ ID NO:5) rather than synthetic humanized L2 and designated pCICR2. Cos-1 cells were transfected with these plasmids and L2 protein expression examined by immunofluorescence of transfected cells. Synthetic humanized L2 (SEQ ID NO:7), driven by the natural BPV-1 promoter, was efficiently expressed, whereas the wild type L2 sequence (SEQ ID NO:5), driven from a similar construct, produced no immunoreactive L2 protein (SEQ ID NO:6,8) (Figure 5B). As undifferentiated cells supported the expression of the humanized L2 gene (SEQ ID NO:7) but not the wild type L2 (SEQ ID NO:5) expressed from the cryptic late BPV promoter, the results confirmed our earlier observations from experiments using the CMV promoter. However, the plasmids tested here contained SV40 ori, designed to replicate the DNA in Cos cells. The increased copy number of the BPV1 L2 plasmids or the transcriptional enhancing activity of the SV40 ori might explain in part the increased efficiency of expression of L2 in this experimental system when compared with infected skin. However, the marked difference in expression between the natural and humanized genes seen with a CMV promoter construct is still observed with the natural promoter.

### EXAMPLE 5

### Substitution of papillomavirus-preferred codons prevents translation but not transcription of a non-papillomavirus gene in undifferentiated cells.

### Materials and Methods

### Codon replacement in gfp gene

To construct a modified gfp gene (SEQ ID NO:11) using papillomavirus preferred codons (PGFP), 6 pairs of oligonucleotides were synthesized. Each pair of oligonucleotides has restriction sites incorporated and was used to amplify *gfp* using a humanized *gfp* gene (SEQ ID NO:9) (GIBCO) as template. The PCR fragments were ligated into the pUC18 vector to produce pUCPGFP. The PGFP gene was sequenced, and cloned into *Bam*HI site of the same mammalian expression vector, pCDNA3, under the CMV promoter. The DNA and deduced amino acid sequences of the humanized GFP gene are shown in Figures 1C. Mutations introduced into the wild type *gfp* gene (SEQ ID NO: 9) to produce the P*gfp* gene (SEQ ID NO:11) are indicated above the corresponding nucleotides of the wild-type sequence.

### Results

To further confirm that codon usage can alter gene expression in mammalian cells, we made a further variant on a synthetic gfp gene modified for optimal expression in eukaryotic cells (Zolotukhin, et al., 1996. J. Virol. 70:4646-4654). In our variant, codons optimized for expression in eukaryotic cells were substituted by those preferentially used in papillomavirus late genes. Of 240 codons in the humanized gfp gene (SEQ ID NO:9), which expresses high levels of fluorescent protein in cultured cells, 156 were changed to the corresponding papillomavirus late gene-preferred codons to produce a new *gfp* gene (SEQ ID NO:11) designated P*gfp*. Expression of P*gfp* (SEQ ID NO:11) in undifferentiated cells was compared with that of humanized *gfp* (SEQ ID NO:9). Cos-1 cells transfected with the humanized *gfp* (SEQ ID NO:9) produced a bright fluorescent signal after 24 hrs, while cells transfected with P*gfp* (SEQ ID NO:11) produced only a faint fluorescent signal (Figure 6A). To confirm that this difference reflected differing translational efficacy, *gfp* specific mRNA was tested in both transfections and found not to be significantly different (Figure 6B.). Thus, codon usage and corresponding tRNA availability apparently determines the observed restriction of expression of PV late genes, and modification of codon usage in other genes similarly prevents their expression in undifferentiated cells.

### EXAMPLE 6

PGFP with papillomavirus-preferred codons is efficiently expressed *in vivo* in differentiated mouse keratinocytes.

### Materials and Methods

### Delivery of plasmid DNA into mouse skin by gene gun

Fifty microgram of DNA was coated onto 25 µg gold micro-carriers by calcium precipitation, following the manufacturer's instructions (Bio-Rad). C57/bl mouse skin was bombarded with gold particles coated with DNA plasmid at a pressure of 600 psi. Serial sections were taken from the skin and examined for distribution of the particles, confirming that a pressure of 600 psi could deliver particles throughout the epidermis.

### Results

Mice were shot with gold beads carrying PGFP DNA plasmid and, 24 hrs later, skin samples were cut from the site of DNA delivery and examined for expression of GFP protein (SEQ ID NO:10,12). Fluorescence was detected mostly in upper keratinocyte layers, representing the differentiated epithelium, and was not seen in undifferentiated basal cells. In contrast, skin sections shot with the humanized GFP plasmid showed fluorescence in cells randomly distributed throughout the whole epidermis (Figure 7). Although GFP-positive cells were rare in both PGFP-(SEQ ID NO:11) and GFP-inoculated (SEQ ID NO:9) mouse skin, fluorescence was observed only in differentiated strata in the PGFP sample (SEQ ID NO:11), whereas fluorescence was observed throughout the epidermis in GFP-inoculated (SEQ ID NO:9) mouse skin. This result confirmed that the use of papillomavirus-preferred codons resulted in the protein being expressed in an epithelial differentiation-dependent manner.

### EXAMPLE 7

### Microinjection of yeast tRNA and wild type L1 gene into cultured cells

To test if yeast tRNA could facilitate expression of wild type BPV-1 L1 (SEQ ID NO:1) (as yeast uses a similar set of codons to those observed in papillomavirus for its own genes), 2 pL of mixtures containing tRNA (2 mg/mL) (purified yeast tRNA (Boehringer Mannheim) or bovine liver tRNA - control) and BPV L1 DNA (2 µg/mL) can be injected into CV-1 cells (Lu and Campisi, 1992, Proc. Natl. Acad. Sci. U. S. A. 89 3889-3893). The injected cells can then be cultured for 48 hrs at 37°C and examined for expression of L1 gene by standard immunofluoresence methods using BPV L1-specific antibody and quantified by FACS analysis (Qi et al 1996, Virology 216 35-45).

### EXAMPLE 8

### Establishment of a cell line which can continuously produce HPV virus particles

To produce infectious PV, various methods have been tried including the epithelial raft culture system (Dollard et al 1992, Genes Dev 6 1131-1142), and cell lines containing BPV-1 episomal DNA, and infected by BPV-1 L1/L2 recombinant vaccinia (Zhou et al 1993, J. Gen. Virol. 74 763-768) or transfected by SFV RNA (Roden et al 1996, J. Virol. 70 5875-5883). The yield of particles is in each case low. In a reduction to practice of our discovery, synthetic BPV L1 (SEQ ID NO:3) and L2 genes (SEQ ID NO:7) (as described in Example 1) can be used to produce infectious BPV in a cell line containing BPV-1 episomal DNA. Fibroblast cell lines (CON/BPV) containing BPV-1 episomal DNA (Zhou et al 1993, J. Gen. Virol. 74 763-768) can be used for transfection of the synthetic BPV-1 L1 (SEQ ID NO:3) and L2 genes (SEQ ID NO:7) under control of CMV promoter. BPV particles may then be purified from the cell lysate and the purified particles examined for the presence of BPV-1 genome. Standard methods such as transfection with lipofectamine (BRL) and G418 selection of transfected cells can be utilized to generate suitable transfectants expressing humanized L1 (SEQ ID NO:3) and L2 (SEQ ID NO:7) in the background of BPV-1 episomal DNA. Examination of L1 and L2 protein expression can be performed using rabbit anti-BPV L1 or rabbit anti-BPV L2 polyclonal antibodies. BPV particles can then be purified using our published methods (Zhou et al 1995, Virology 214 167-176) and can be characterized by electron microscopy and DNA blotting. The infectivity of BPV particles isolated from the cultured cells may be tested in focus formation assays using C127 fibroblasts.

### EXAMPLE 9

### Method for extracting and measuring tRNA from tissues

Tissue(100g) is homogenized in a Waring Blender with 150 mL of phenol (Mallinckrodt, Analytical Reagent, 88%) saturated with water (15:3) and 150 mL of 1.0 M NaCl, 0.005 M EDTA in 0.1 M Tris-chloride buffer, pH 7.5. The homogenate was spun for ten minutes at top speed in the International clinical centrifuge and the upper layer was carefully decanted off. To this aqueous layer, three volumes of 95% ethanol were added. The resultant precipitate was spun down at top speed in the International clinical centrifuge and resuspended in 250 mL of 0.1 M Tris/chloride buffer, pH 7.5. This solution was added (flow rate of 15-20 drops per minute) to a column (2 x 10 cm) of 2 g of DEAE-cellulose previously equilibrated with cold 0.1 M Tris-chloride buffer pH 7.5. The column was then washed with 1 L of Tris-chloride buffer, pH 7.5 and the RNA eluted with 1.0 M NaCl in 0.1 M Tris-chloride buffer, pH 7.5. The first 10 mL of NaCl solution were discarded as "hold-up." Sufficient salt solution (60-80 mL) was then collected until the optical density of the effluent was less than three at 260 nm. This solution was extracted twice with an equal volume of phenol saturated with water and twice with ether. To the aqueous solution containing the RNA, three volumes of 95% ethanol were added and the solution wag allowed to stand overnight in the cold. The precipitate was spun down and washed first with 80% and then twice with 95% ethanol and dried in a vacuum. Approximately 60 mg of soluble RNA were obtained from a 100-g lot of rat liver.

### Quantitating tRNAs

The following nylon membranes are used: Biodine A and B (PALL). For the preparation of dot blots, the tRNA samples (from 1 pg to 5 ng) are denatured at 60 °C for 15 min in 1-5 µL of 15% formaldehyde. 10x SSC (SSC is NaCl 0.3 M, tri-sodium citrate 0.03 M). The samples are spotted in 1 µL aliquots onto the membranes that have been soaked for 15 min in deionized water and slightly dried between two sheets of 3MM Whatman paper prior to the application of the samples. The tRNAs are fixed covalently (in the membranes by ultraviolet-irradiation (10 mm using an ultraviolet lamp at 254 nm and 100 W strength at a distance of 20 cm) and the membranes are baked for 2-3 h at 80 °C.

A 5' end labelled synthetic deoxyribo-oligonucleotide complementary to the A54-A73 sequence of the tRNA is used as a probe for the hybridization experiments. Labelling of the oligonucleotide is performed by direct phosphorylation of the 5' OH' ended probe.

For hybridisation experiments, the UV-irradiated membranes are first preincubated for 5 h at 50 C in 50% deionized formamide, 5 × SSC, 1% SDS, 0.04% Ficoll 0.04% polyvinylpyrrolidone and 250 µL/mL of sonicated salmon sperm DNA using 5 mL of buffer for 100 cm² of membrane. Hybridization is finally performed overnight at 50 °C in the above solution (2.5 mL/100 cm²) where the labeled probe has been added. After hybridization, the membranes are washed twice in 2 × SSC, 0.1% SDS for 5 min at room temperature, twice in 2 × SSC, 1% SDS for 30 mm at 60 °C and finally in 0.1 × SSC. 0.1% SDS for 30 min at room temperature. To detect the hybridized probes the membranes are exposed for 16 h to Fuji XR film at 70 °C with an intensifying screen.

### Sequence of tRNA probes

The sequences of the tRNA probes are as follows:
Ala^{GCA} 5'-TAAGGACTGTAAGACTT (SEQ ID NO:13)
Arg^{CGA}: 5'-CGAGCCAGCCAGGAGTC (SEQ ID NO:14)
Asn^{AAC}: 5'-CTAGATTGGCAGGAATT (SEQ ID NO:15)
Asp^{GAC}: 5'-TAAGATATATAGATTAT (SEQ ID NO:16)
Csy^{TGC}: 5'-AAGTCTTAGTAGAGATT (SEQ ID NO:17)
Gluc^{GAA}: 5'-TATTTCTACACAGCATT (SEQ ID NO:18)
Gln^{CAA}: 5'-CTAGGACAATAGGAATT (SEQ ID NO:19)
Gly^{GGA}: 5'-TACTCTCTTCTGGGTTT (SEQ ID NO:20)
His^{CAC}: 5'-TGCCGTGACTCGGATTC (SEQ ID NO:21)
Ile^{ATC}: 5'-TAGAAATAAGAGGGCTT (SEQ ID NO:22)
Leu^{CTA}: 5'-TACTTTTATTTGGATTT (SEQ ID NO:23)
Leu^{CTT}: 5'-TATTAGGGAGAGGATTT (SEQ ID NO:24)
Lys^{AAA} : 5'-TCACTATGGAGATTTTA (SEQ ID NO:25)
Lys^{AAG}: 5'-CGCCCAACGTGGGGCTC (SEQ ID NO:26)
Met^{elong} 5'-TAGTACGGGAAGGATTT (SEQ ID NO:27)
Phe^{TTC}: 5'-TGTTTATGGGATACAAT (SEQ ID NO:28)
Pro^{CCA}: 5'-TCAAGAAGAAGGAGCTA (SEQ ID NO:29)
Pro^{CCI}: 5'-GGGCTCGTCCGGGATTT (SEQ ID NO:30)
Ser^{AGC}: 5'-ATAAGAAAGGAAGATCG (SEQ ID NO:31)
Thr^{ACA}: 5'-TGTCTTGAGAAGAGAAG (SEQ ID NO:32)
Tyr^{TAC}: 5'-TGGTAAAAAGAGGATTT (SEQ ID NO:33)
val^{GTA}: 5'-TCAGAGTGTTCATTGGT (SEQ ID NO:34)

### EXAMPLE 10

### Comparison of the relative abundance of tRNA species in undifferentiated and differentiated keratinocytes

### Materials and Methods

### Isolation of epidermal cells

2-day old mice were killed and their skins removed. The skins were digested with 0.25% trypsin PBS at 4 °C overnight. The epidermis was separated from the dermis using forceps and minced with scissors in 10% FCS DMEM medium. The cell suspension was first filtered through a 1 mm and then a 0.2 mm nylon net. The cell suspension was then pelleted and washed twice with PBS.

### Density gradient centrifugation

The keratinocytes were resuspended in 30% Percoll and separated by centrifugation through a discontinuous Percoll gradient (1.085, 1.075 and 1.050 g/mL) at 1200 x g at room temperature for 25 min. The cells were then washed with PBS and used to extract tRNA.

### tRNA purification

The cells were lysed in 5 mL of lysis buffer (0.2 M NaOH, 1% SDS) for 10 min at room temperature. The lysate was neutralized with 5 mL of 3.0 M potassium acetate (pH 5.5). After centrifugation, the supernatant was diluted with 3 volumes of 100 mM Tris (pH 7.5) and added to a DEAE column equilibrated with 100 mM Tris (pH 7.5). An equal volume of isopropanol was added to the aqueous solution containing tRNA, and the solution was allowed to stand overnight at 4 °C. The tRNA was spun down and washed with 75% ethanol, then dissolved in RNase-free water.

### tRNA blotting

10 ng of each tRNA sample in 1 µL was denatured in 60°C for 15 min in 4 µL formaldehyde and 5 µL 20 x SSC. The samples were spotted in 1 µL aliquots onto charged nylon membrane (Amersham), and the tRNAs were fixed with UV and probed with ³²P-oligonucleotides.

### Results

Comparison of the abundance of the tRNA species in undifferentiated and differentiated keratinocytes showed that the levels of some tRNA populations changed dramatically. For example, the levels of tRNAs specific for Ala^{GCA}, Leu^{CTT}, Leu^{CTA} were increased in differentiated cells while tRNAs for Arg^{CGA}, Pro^{CCI}, Asn^{AAC} were more abundant in undifferentiated keratinocytes (see Table 2).

### GENERAL DISCUSSION

In the present specification the inventors have confirmed that one determinant of the efficiency of translation of a gene in mammalian cells is its codon composition. This observation has commonly been made when genes from prokaryotic organisms have been expressed in eukaryotic cells (Smith, D. W., 1996, Biotechnol. Prog. 12:417-422). The present inventors have also presented evidence that mRNA encoding the capsid genes of papillomavirus are not effectively translated in cultured eukaryotic cells, apparently because tRNA availability is rate limiting for translation, and that the block to PV late gene translation in eukaryotic cells in culture can be overcome by altering the codon usage of the late genes to match the consensus for mammalian genes, or alternatively by providing exogenous tRNAs. Alterations to mRNA secondary structure or protein binding (Sokolowski, et al., 1998, J. Virol. 72:1504-1515) as a consequence of the changes to the primary sequence of the PV capsid genes might contribute to the observed differences in efficiency of translation of the natural and modified PV capsid gene mRNAs in cultured cells. However, the enhancement of translation of the natural but not the modified mRNA that was observed after addition of tRNA in a mammalian *in vitro* translation system, which was not observed in a plant translation system, strengthens the argument that tRNA availability is rate limiting for translation of the natural gene in mammalian cells. A shortage of critical tRNAs could result in slowed elongation of the nascent peptide or premature termination of translation (Oba, et al., 1991, Biochimie 73:1109-1112). Slowed elongation appears to be the major consequence for the PV late gene. Analysis of codon usage in the PV genome shows that PV late genes use many codons that mammalian cells rarely use. For example, PV frequently uses UUA for leucine, CGU for arginine, ACA for threonine, and AUA for isoleucine, whereas these codons are significantly less often used in mammalian genes. In contrast, papillomavirus late genes can be expressed efficiently in yeast (Jansen, et al., 1995, Vaccine 13:1509-1514) (Sasagawa, et al., 1995, Virology 206:126-135) and the codon composition of yeast and papillomavirus genes are similar (Table 1). An apparent exception is that PV L1 genes can be efficiently expressed in insect cells (Kirnbauer, et al., 1992, Proc. Natl. Acad. Sci. USA 89:12180-12184) using recombinant baculovirus, or in various undifferentiated mammalian cells using recombinant vaccinia (Zhou, et al., 1991, Virology 185:251-257). As infection with vaccinia or baculovirus down regulates cellular protein synthesis, the efficient expression of the L1 capsid proteins under these circumstances may occur because less cellular mRNA is available in a virus infected cell to compete with the L1 mRNA for the rarer tRNAs.

Codon composition could be a more general determinant of gene expression within different stages of differentiation of the same tissue. Although the genetic code is essentially universal, different organisms show differences in codon composition of their genes, while the codon composition of genes tends to be relatively similar for all genes within each organism, and matched to the population of iso-tRNAs for that organism (Ikemura, T., 1981, J. Mol. Biol. 146:1-21). However, populations of tRNAs in differentiating and neoplastic cells are different (Kanduc, D., 1997, Arch. Biochem. Biophys. 342:1-6; Yang, and Comb, 1968, J. Mol. Biol. 31:138-142; Yang, and Novelli, 1968, Biochem. Biophys. Res. Commun. 31: 534-539) and the tRNA populations also vary in cells growing under different growth conditions (Doi, et al., 1968, J. Biol. Chem. 243:945-951). Accordingly, the inventors believe that codon composition and tRNA availability together provide a primitive mechanism for spatial and/or temporal regulation of gene expression. It is well recognized that the G+C content of many dsDNA viruses, a crude marker for viral gene codon composition, is markedly different from the G+C content of the DNA of the cells they infect (Strauss, et al., 1995, "Virus Evolution" in Virology (eds. Fields, B. N., et al.), Lipipincott-Raven, Philadelphia, pp 153-171). Viruses may therefore have evolved to take advantage of codon composition to regulate their own program of gene expression, perhaps to avoid expression of lethal quantities of viral proteins in undifferentiated cells where the virus utilizes the cellular machinery to replicate its genome.

As the inventors' observations represent an apparently novel mechanism of regulation of gene translation within a single tissue, it is relevant to consider how this relates to previously proposed hypotheses for the restriction of expression of PV late genes to differentiated epithelium. A number of explanations have been proposed for the observation that PV late genes are only effectively expressed in differentiated epithelium. Reduced late gene transcription may reflect dependence of transcription from the late promoter on transcription factors expressed only in differentiated epithelium, or may alternatively be due to suppression of late promoter transcription by viral (Stubenrauch, et al., 1996, J. Virol. 70:119-126) or cellular gene products expressed in undifferentiated cells. The "late" promoters of HPV31b and of HPV5 (Haller, et al., 1995, Virology 214:245-255; Hummel, et al., 1992, J. Virol. 66:6070-6080) are described as differentiation dependent, although the search for relevant transcription control factors in differentiated keratinocytes by conventional footprinting and DNA binding studies has to date been unrewarding. Our data show that capsid proteins are not translated from PV L1 and L2 mRNAs in cells transfected with CMV promoter-based expression vectors (Fig. 2), suggesting that in addition to any transcriptional controls that may exist that there is a post-transcriptional block to capsid protein synthesis in undifferentiated cells. Sequences resembling 5' splice donor sites exist within L1 or L2 mRNA or within flanking untranslated message which are inhibitory to transcription of genes with which they are associated (Kennedy, et al., 1991, J. Virol. 65:2093-2097) (Furth, et al., 1994, Mol. Cell. Biol. 14:5278-5289). Other AU rich sequences in L1 or L2 mRNA promote mRNA degradation (Sokolowski, et al., 1997, Oncogene 15:2303-2319). These mechanisms inhibiting L1 and L2 expression in undifferentiated cells have yet to be shown to be inactive in differentiated epithelium, to explain the successful translation of late genes in this tissue.

Because inhibitory RNA sequences within the L1 coding sequence could have been rendered non-functional by the systematic codon substitution employed in the experiments described herein and the untranslated inhibitory sequences were not included in the inventors' test system, the respective roles of inhibitory sequences and codon mismatch in suppression of PV late gene expression in cultured mammalian cells cannot be determined. However, regulatory sequences promoting RNA degradation or inhibiting translation are presumed to act through interaction with nuclear or cytoplasmic proteins (Sokolowski, et al., 1998, J. Virol. 72:1504-1515), and inefficient translation of native sequence L1 mRNA was observed in a cell free translation system from anucleate cells, demonstrating that codon composition of the PV late genes must play some role in regulation of PV late gene translation.

Further evidence supporting the hypothesis that codon composition is an important determinant of PV capsid gene expression was gathered from an analysis of the 84 PV L1 sequences currently available in Genebank. The codon composition of the L1 genes, and particularly the frequency of usage of the rarer codons, was essentially the same across all the published sequences (data not shown) as would be predicted by the similar G+C content of the papillomavirus genomes. The PV L1 gene is relatively conserved at the amino acid level, showing 60 - 80% amino acid homology between PV genotypes, as might be expected by the constraints on capsid protein function. There are, however, no obvious constraining influences on the codon composition of the PV late genes beyond those of the inventors' hypothesis, as the late gene region does not code for other genes, either in other reading frames or on the complementary DNA strand, and has no known cis acting regulatory functions. If codon composition of the capsid genes were not important for PV function, a considerable heterogeneity of codon usage might therefore be expected, given the evolutionary diversity of PVs (Chan, et al. 1995, J. Virol. 69:3074-3083).

Taken together, the data and evidence outlined herein makes a strong case that codon usage is a significant determinant of expression of PV late genes in undifferentiated and differentiated epithelial cells, and that this observation is generalizable. The relative role of message instability and codon mismatch in determining expression in differentiated tissues will require comparisons of transcriptional activity and translation of the L1 or L2 genes driven from strong constitutive promoters in differentiated and undifferentiated epithelium. Such work should now be feasible using either transgenic technology or keratinocyte raft cultures.

Although mechanisms of transcriptional regulation of PV L1 or L2 gene expression in the superficial layer of differentiated epithelium have been proposed (Zeltner et al., 1994, J. Virol. 68:3620; Brown, et al., 1995, Virology 214:259; Stoler et al., 1992, Hum. Pathol. 23:117; Hummel et al., 1995, J. Virol. 69:3381; Haller et al., 1995, Virology 214:245; Barksdale and Baker, 1993, J. Virol. 67:5605), measurable PV late gene mRNA is not always associated with production of late proteins (Zeltner *et al.,* 1994, *supra*; Ozbun and Meyers, 1997, J. Virol. 71:5161), and the data presented here suggest that translation regulation may play a major part in controlling PV late gene expression. This observation has implications as herein described for the regulation of expression of genes related to the specialised functions of any differentiated tissue, and also for targeting of expression of therapeutic genes to such tissue while avoiding the potentially deleterious consequences of expression of the exogenous gene in a self renewing stem cell population.

### TABLE LEGENDS

### TABLE 1

The codon usage data for human, cow yeast and wheat proteins are derived from published results (18) . The BPV1 data are from the sequences in the Genbank database.

### TABLE 2

Each iso-acceptor tRNA with anticodon shown as superscript are shown on top row. The "+" indicates the abundance of tRNA wherein each "+" indicates about 10 fold increase.

### TABLES

**TABLE 1**

| *Frequency (per one* thousand) *of codon* usage for individual *organisms.* | | | | | | |
|---|---|---|---|---|---|---|
| **Amino acids** | **Codons** | **Human** | **Cow** | **Yeast** | **Wheat** | **BPVL1/ L2** |
| ARG | CGA | 5.4 | 5.5 | 2.3 | 2.3 | 7.2 |
| | CGC | 11.3 | 12.2 | 2.0 | 7.5 | 4.1 |
| | CGG | 10.4 | 11.2 | 1.1 | 4.6 | 5.1 |
| | CGU | 4.7 | 3.7 | 7.5 | 1.1 | 10.4 |
| | AGA | 9.9 | 9.9 | 24.0 | 4.1 | 14.4 |
| | AGG | 11.1 | 11.4 | 7.5 | 7.1 | 9.3 |
| LEU | CUA | 6.2 | 4.9 | 11.8 | 12.1 | 18.6 |
| | CUC | 19.9 | 21.2 | 4.1 | 18.6 | 6.2 |
| | CUG | 42.5 | 46.6 | 8.3 | 15.5 | 15.5 |
| | CUC | 10.7 | 10.6 | 9.6 | 6.5 | 20.7 |
| | UUA | 5.3 | 4.0 | 24.5 | 1.8 | 14.5 |
| | UUG | 11.0 | 9.6 | 32.1 | 15.3 | 15.5 |
| SER | UCA | 9.3 | 7.6 | 15.6 | 14.6 | 16.6 |
| | UCC | 17.7 | 17.6 | 14.4 | 10.1 | 11.4 |
| | UCG | 4.2 | 4.5 | 6.5 | 9.6 | 6.2 |
| | UCU | 13.2 | 11.2 | 24.6 | 14.8 | 15.5 |
| | AGC | 18.7 | 18.7 | 7.1 | 12.8 | 12.4 |
| | AGU | 9.4 | 8.6 | 11.7 | 12.9 | 21.7 |
| THR | ACA | 14.4 | 11.4 | 15.6 | 4.6 | 37.3 |
| | ACC | 23.0 | 21.1 | 13.9 | 15.9 | 19.7 |
| | ACG | 6.7 | 7.8 | 6.7 | 4.5 | 4.1 |
| | ACU | 12.7 | 9.6 | 22.0 | 11.8 | 28.0 |
| PRO | CCA | 14.6 | 12.0 | 21.4 | 71.2 | 22.8 |
| | CCC | 20.0 | 19.2 | 5.9 | 11.1 | 15.5 |
| | CCG | 6.5 | 7.9 | 4.1 | 19.4 | 0.0 |
| | CCU | 15.5 | 14.6 | 12.8 | 10.3 | 33.1 |
| ALA | GCA | 14.0 | 13.1 | 15.3 | 11.2 | 33.1 |
| | GCC | 29.1 | 35.8 | 15.5 | 19.5 | 17.6 |
| | GCG | 7.2 | 9.3 | 5.1 | 13.8 | 4.1 |
| | GCU | 19.6 | 19.1 | 28.3 | 9.6 | 13.5 |
| GLY | GGA | 17.1 | 16.2 | 8.9 | 25.9 | 22.8 |
| | GGC | 25.4 | 28.1 | 8.9 | 28.0 | 12.4 |
| | GGG | 17.3 | 19.2 | 5.1 | 28.5 | 22.8 |
| | GGU | 11.2 | 11.8 | 34.9 | 9.6 | 18.6 |
| VAL | GUA | 5.9 | 5.1 | 10.0 | 4.4 | 15.5 |
| | GUC | 16.3 | 18.4 | 14.9 | 14.8 | 6.2 |
| | GUG | 30.9 | 32.9 | 9.5 | 12.9 | 23.8 |
| | GUU | 10.4 | 9.9 | 26.6 | 11.6 | 16.6 |
| LYS | AAA | 22.2 | 21.6 | 37.7 | 4.5 | 37.2 |
| | AAG | 34.9 | 37.1 | 35.2 | 17.4 | 13.5 |
| ASN | AAC | 22.6 | 22.4 | 25.8 | 14.2 | 10.3 |
| | AAU | 16.6 | 12.5 | 31.4 | 6.7 | 24.8 |
| GLN | CAA | 11.1 | 9.7 | 29.8 | 171.8 | 22.8 |
| | CAG | 33.6 | 34.4 | 10.4 | 79.4 | 17.6 |
| HIS | CAC | 14.2 | 14.0 | 8.2 | 8.2 | 6.2 |
| | CAU | 9.3 | 7.5 | 12.3 | 7.1 | 13.4 |
| GLU | GAA | 26.8 | 24.4 | 48.9 | 7.8 | 36.2 |
| | GAG | 41.4 | 45.4 | 16.9 | 19.7 | 21.7 |
| ASP | GAC | 29.0 | 31.5 | 22.3 | 13.0 | 18.6 |
| | GAU | 21.7 | 19.2 | 37.0 | 4.0 | 33.1 |
| TYR | UAC | 18.8 | 20.3 | 16.5 | 24.5 | 17.6 |
| | UAU | 12.5 | 10.5 | 16.5 | 12.5 | 18.6 |
| CYS | UGC | 14.5 | 13.9 | 3.7 | 14.8 | 5.2 |
| | UGU | 9.9 | 9.4 | 7.6 | 4.9 | 5.2 |
| PHE | UUC | 22.6 | 25.5 | 20.0 | 14.1 | 7.2 |
| | UUU | 15.8 | 17.0 | 23.2 | 15.0 | 23.8 |
| ILE | AUA | 5.8 | 5.2 | 12.8 | 5.4 | 22.7 |
| | AUC | 24.3 | 25.8 | 18.4 | 19.7 | 8.2 |
| | AUU | 14.9 | 13.1 | 31.1 | 10.7 | 20.7 |

**TABLE 2**

| *tRNA population changes as KC starts to differentiate.* | | | | | | | | | |
|---|---|---|---|---|---|---|---|---|---|
| tRNA | Arg^{CGA} | Ala^{GCA} | His^{CAC} | Leu^{CTT} | Leu^{CTA} | Lys^{AAG} | Lys^{AAA} | Met^{Ini} | Pro^{CCI} |
| Supra | + | +++ | + | +++ | +++ | ++ | + | + | + |
| Basal | +++ | + | ++ | + | + | + | + | ++ | +++ |
| | | | | | | | | | |
| tRNA | Val^{GTA} | Val^{GTI} | His^{CAC} | Asn^{AAC} | Thr^{ACA} | Met^{Elo} | Gly^{GGA} | | |
| Supra | ++ | + | ++ | + | + | + | + | | |
| Basal | + | + | + | +++ | + | ++ | + | | |

## Claims

1. A method of constructing a synthetic polynucleotide that encodes a polypeptide, the method comprising:
- selecting a first codon of a parent polynucleotide for replacement with a synonymous codon, wherein the synonymous codon is selected on the basis that it exhibits a higher translational efficiency in a first cell of a mammal than in a second cell of the mammal; and
- replacing the first codon with the synonymous codon to construct the synthetic polynucleotide.

2. The method of claim 1, wherein the first codon and the synonymous codon are selected by:
- comparing translational efficiencies of individual codons in the first cell relative to the second cell; and
- selecting the first codon and the synonymous codon based on the comparison.

3. The method of claim 2, wherein the translational efficiency of an individual codon is compared by measuring the abundance of an iso-tRNA corresponding to the individual codon in the first cell relative to the second cell.

4. The method of claim 3, wherein the synonymous codon corresponds to an iso-tRNA which is in higher abundance in the first cell relative to the second cell.

5. The method of claim 3, wherein selecting the first codon and the synonymous codon comprises:
- measuring abundance of different iso-tRNAs in the first cell relative to the second cell; and
- selecting the first codon and the synonymous codon based on the measurement, wherein the synonymous codon corresponds to an iso-tRNA which is in higher abundance in the first cell than in the second cell.

6. The method of claim 3, wherein the synonymous codon corresponds to an iso-tRNA that is present in the first cell at a level which is at least 110% of the level of the iso-tRNA that is present in the second cell.

7. The method of claim 1, wherein the synonymous codon is selected from the group consisting of (1) a codon used at relatively high frequency by genes of the first cell, (2) a codon used at relatively high frequency by genes of the mammal, (3) a codon used at relatively low frequency by genes of the second cell, and (4) a codon used at relatively low frequency by genes of an organism other than the mammal.

8. The method of claim 1, wherein the first codon and the synonymous codon are selected such that the polypeptide is expressed from the synthetic polynucleotide in the first cell at a level which is at least 110% of the level at which the polypeptide is expressed from the parent polynucleotide in the first cell.

9. The method of claim 1, wherein the second cell is a precursor cell of the first cell.

10. The method of claim 1, wherein the second cell is a cell derived from the first cell.

11. The method of claim 1, wherein the polypeptide is not substantially expressible in the second cell.

12. The method of claim 1, wherein the first cell is of the same type as the second cell, but is at a different stage of differentiation.

13. The method of claim 1, wherein the first cell is of the same type as the second cell, but is at a different stage of the cell cycle.

14. A method of selectively expressing a polypeptide in a first cell of a mammal, the method comprising:
- selecting a first codon of a parent polynucleotide encoding the polypeptide for replacement with a synonymous codon, wherein the synonymous codon is selected on the basis that it exhibits a higher translational efficiency in the first cell than in a second cell of the mammal;
- replacing the first codon with the synonymous codon to construct a synthetic polynucleotide; and
introducing the synthetic polynucleotide into a cell selected from the group consisting of the first cell and a precursor of the first cell, the synthetic polynucleotide being operably linked to a regulatory polynucleotide,
whereby the polypeptide is selectively expressed in the first cell.

15. The method of claim 14, wherein the synonymous codon has a higher translational efficiency in the first cell than in the second cell.

16. A method of expressing a polypeptide from a first polynucleotide in a mammalian cell, the method comprising:
- selecting an iso-tRNA on the basis that it is normally in lower abundance in the cell than other iso-tRNAs and corresponds to a codon of the first polynucleotide;
- introducing into the cell a second polynucleotide that encodes the selected iso-tRNA and that is operably linked to a regulatory polynucleotide; and
- expressing the second polynucleotide in the cell, whereby the polypeptide is expressed in the cell.

17. A method of expressing a polypeptide from a first polynucleotide in a mammalian cell, the method comprising
- selecting an iso-tRNA on the basis that it is normally in lower abundance in the cell than other iso-tRNAs and corresponds to a codon of the first polynucleotide;
- introducing into a precursor of the cell a second polynucleotide that encodes the selected iso-tRNA and that is operably linked to a regulatory polynucleotide, wherein the precursor is exposed to conditions sufficient to produce the cell; and
- expressing the second polynucleotide in the cell, whereby the polypeptide is expressed in the cell.

18. A method of producing a virus particle in a cycling eukaryotic cell, wherein the virus particle comprises a polypeptide necessary for assembly of the virus particle, and wherein the polypeptide is expressed in the cell from a parent polynucleotide, but not at a level sufficient to permit virus assembly therein, the method comprising:
- providing a comparison of translational efficiencies of individual codons in the cell;
- selecting from the comparison a first codon of the parent polynucleotide for replacement with a synonymous codon, wherein the synonymous codon is selected on the basis that it exhibits a higher translational efficiency in the cell than the first codon; and
- replacing the first codon with the synonymous codon to construct a synthetic polynucleotide having enhanced translational kinetics compared to the parent polynucleotide, such that the polypeptide is expressible from the synthetic polynucleotide in the cell at a level sufficient to permit virus assembly therein; and
- introducing into the cell the synthetic polynucleotide operably linked to a regulatory polynucleotide,
whereby the polypeptide is expressed and the virus particle is produced in the cell.

19. The method of claim 18, wherein the synonymous codon has a higher translational efficiency in the cell than the first codon.

20. A method of producing a virus particle in a cycling cell, wherein the virus particle comprises at least one polypeptide necessary for assembly of the virus particle, wherein the polypeptide is expressed in the cell from a first polynucleotide, but not at a level sufficient to permit virus assembly therein, the method comprising:
- providing a comparison of translational efficiencies of individual codons in the cell;
- selecting from the comparison a first codon of the first polynucleotide that exhibits a lower translational efficiency in the cell than a synonymous codon;
- introducing into the cell a second polynucleotide encoding an iso-tRNA specific for the first codon; and
- expressing the second polynucleotide in the cell,
whereby the virus particle is produced in the cycling cell.

21. A method of producing a virus particle in a cycling eukaryotic cell, wherein the virus particle comprises a polypeptide necessary for assembly of the virus particle, and wherein the polypeptide is expressed in the cell from a parent polynucleotide, but not at a level sufficient to permit virus assembly therein, the method comprising:
- providing a comparison of translational efficiencies of individual codons in the cell;
- selecting from the comparison a first codon of the parent polynucleotide for replacement with a synonymous codon, wherein the synonymous codon is selected on the basis that it exhibits a higher translational efficiency in the cell than the first codon; and
- replacing the first codon with the synonymous codon to construct a synthetic polynucleotide having increased translational kinetics compared to the parent polynucleotide, such that the polypeptide is expressible from the synthetic polynucleotide in the cell at a level sufficient to permit virus assembly therein;
- introducing into a precursor of the cell the synthetic polynucleotide operably linked to a regulatory polynucleotide; and
- exposing the precursor to conditions sufficient to produce the cell, wherein the cell comprises the polypeptide necessary for assembly of the virus particle,
whereby the polypeptide is expressed and the virus particle is produced in the cell.

22. A method of constructing a synthetic polynucleotide that encodes a polypeptide, the method comprising:
- selecting a first codon of a parent polynucleotide for replacement with a synonymous codon, wherein the synonymous codon is selected on the basis that it exhibits a lower translational efficiency in a first cell of a mammal than in a second cell of the mammal; and
- replacing the first codon with the synonymous codon to construct the synthetic polynucleotide.

23. The method of claim 22, wherein the first codon and the synonymous codon are selected by:
- comparing translational efficiencies of individual codons in the first cell relative to the second cell; and
- selecting the first codon and the synonymous codon based on the comparison.

24. The method of claim 23, wherein the translational efficiency of an individual codon is compared by measuring the abundance of an iso-tRNA corresponding to the individual codon in the first cell relative to the second cell.

25. The method of claim 24, wherein the synonymous codon corresponds to an iso-tRNA which is in lower abundance in the first cell relative to the second cell.

26. The method of claim 23, wherein selecting the first codon and the synonymous codon comprises:
- measuring abundance of different iso-tRNAs in the first cell relative to the second cell; and
- selecting the first codon and the synonymous codon based on the measurement, wherein the synonymous codon corresponds to an iso-tRNA which is in lower abundance in the first cell than in the second cell.

27. A method of constructing a synthetic polynucleotide that encodes a polypeptide, the method comprising:
- providing a comparison of translational efficiencies of individual codons in a cell of a mammal;
- selecting from the comparison a first codon of the parent polynucleotide for replacement with a synonymous codon, wherein the synonymous codon is selected on the basis that it exhibits a higher translational efficiency in the cell than the first codon; and
- replacing the first codon with the synonymous codon to construct the synthetic polynucleotide.

28. The method of claim 27, wherein the synonymous codon corresponds to an iso-tRNA which is in higher abundance in the cell relative to the iso-tRNA corresponding to the first codon.

29. The method of claim 27, wherein the first codon and the synonymous codon are selected such that the polypeptide is expressed from the synthetic polynucleotide in the cell at a level which is at least 110% of the level at which the polypeptide is expressed from the parent polynucleotide in the cell.

30. The method of claim 27, wherein the comparison is provided by comparing translational efficiencies of individual codons in the cell.

31. The method of claim 27, wherein the translational efficiencies are compared by measuring abundance of different iso-tRNAs in the cell.

32. A method of constructing a synthetic polynucleotide encoding a polypeptide, the method comprising:
- providing a comparison of translational efficiencies of individual codons in a cell of a mammal;
- selecting from the comparison a first codon of the parent polynucleotide for replacement with a synonymous codon, wherein the synonymous codon is selected on the basis that it exhibits a lower translational efficiency in the cell than the first codon; and
- replacing the first codon with the synonymous codon to construct the synthetic polynucleotide.

33. The method of claim 31, wherein the synonymous codon corresponds to an iso-tRNA which is in lower abundance in the cell relative to the iso-tRNA corresponding to the first codon.

34. The method of claim 31, wherein the comparison is provided by comparing translational efficiencies of individual codons in the cell.

35. The method of claim 34, wherein the translational efficiencies are compared by measuring abundance of different iso-tRNAs in the cell.

## Patentansprüche

1. Verfahren zur Konstruktion eines synthetischen Polynucleotids, das ein Polypeptid codiert, wobei das Verfahren umfasst:
- Auswählen eines ersten Codons eines Eltern-Polynucleotids zum Ersetzen mit einem synonymen Codon, wobei das synonyme Codon anhand der Eigenschaft ausgewählt ist, dass es in einer ersten Zelle eines Säugers eine höhere Translationseffizienz aufweist als in einer zweiten Zelle des Säugers, und
- Ersetzen des ersten Codons mit dem synonymen Codon zur Konstruktion des synthetischen Polynucleotids.

2. Verfahren nach Anspruch 1, wobei das erste Codon und das synonyme Codon ausgewählt werden durch:
- Vergleichen von Translationseffizienzen einzelner Codons in der ersten Zelle im Vergleich zu der zweiten Zelle und
- Auswählen des ersten Codons und des synonymen Codons basierend auf dem Vergleich.

3. Verfahren nach Anspruch 2, wobei die Translationseffizienz eines einzelnen Codons durch Messen der Menge einer iso-tRNA, die dem einzelnen Codon entspricht, in der ersten Zelle im Verhältnis zu der zweiten Zelle verglichen wird.

4. Verfahren nach Anspruch 3, wobei das synonyme Codon einer iso-tRNA entspricht, die in der ersten Zelle im Verhältnis zu der zweiten Zelle in größerer Menge vorliegt.

5. Verfahren nach Anspruch 3, wobei das Auswählen des ersten Codons und des synonymen Codons Folgendes umfasst:
- Messen der Menge von unterschiedlichen iso-tRNAs in der ersten Zelle im Vergleich zu der zweiten Zelle und
- Auswählen des ersten Codons und des synonymen Codons basierend auf der Messung, wobei das synonyme Codon einer iso-tRNA entspricht, die in größerer Menge in der ersten Zelle als in der zweiten Zelle vorliegt.

6. Verfahren nach Anspruch 3, wobei das synonyme Codon einer iso-tRNA entspricht, die in der ersten Zelle in einer Menge vorhanden ist, die wenigstens 110 % der Menge der iso-tRNA, die in der zweiten Zelle vorhanden ist, entspricht.

7. Verfahren nach Anspruch 1, wobei das synonyme Codon ausgewählt ist aus der Gruppe, bestehend aus (1) einem Codon, das mit relativ großer Häufigkeit durch Gene der ersten Zelle verwendet wird, (2) einem Codon, das mit relativ großer Häufigkeit durch Gene des Säugers verwendet wird, (3) einem Codon, das mit relativ geringer Häufigkeit durch Gene der zweiten Zelle verwendet wird, und (4) einem Codon, das mit relativ geringer Häufigkeit durch Gene eines von dem Säuger verschiedenen Organismus verwendet wird.

8. Verfahren nach Anspruch 1, wobei das erste Codon und das synonyme Codon so ausgewählt sind, dass das Polypeptid von dem synthetischen Polynucleotid in der ersten Zelle in einer Menge exprimiert wird, die wenigstens 110 % der Menge, mit welcher das Polypeptid von dem Eltern-Polynucleotid in der ersten Zelle exprimiert wird, entspricht.

9. Verfahren nach Anspruch 1, wobei die zweite Zelle eine Vorläuferzelle der ersten Zelle ist.

10. Verfahren nach Anspruch 1, wobei die zweite Zelle eine von der ersten Zelle abgeleitete Zelle ist.

11. Verfahren nach Anspruch 1, wobei das Polypeptid in der zweiten Zelle nicht wesentlich exprimierbar ist.

12. Verfahren nach Anspruch 1, wobei die erste Zelle vom gleichen Typ ist wie die zweite Zelle, aber in einem anderen Stadium der Differenzierung.

13. Verfahren nach Anspruch 1, wobei die erste Zelle vom gleichen Typ ist wie die zweite Zelle, aber in einem anderen Stadium des Zellzyklus.

14. Verfahren zum selektiven Exprimieren eines Polypeptids in einer ersten Zelle eines Säugers, wobei das Verfahren umfasst:
- Auswählen eines ersten Codons eines Eltern-Polynucleotids, das das Polypeptid zum Ersetzen mit einem synonymen Codon codiert, wobei das synonyme Codon anhand der Eigenschaft ausgewählt ist, dass es in der ersten Zelle eine höhere Translationseffizienz als in einer zweiten Zelle des Säugers aufweist,
- Ersetzen des ersten Codons mit dem synonymen Codon, um ein synthetisches Polynucleotid zu konstruieren, und
- Einbringen des synthetischen Polynucleotids in eine Zelle, die ausgewählt ist aus der Gruppe, bestehend aus der ersten Zelle und einem Vorläufer der ersten Zelle, wobei das synthetische Polypeptid funktionell an ein regulatorisches Polynucleotid geknüpft ist,
wobei das Polypeptid selektiv in der ersten Zelle exprimiert wird.

15. Verfahren nach Anspruch 14, wobei das synonyme Codon in der ersten Zelle eine höhere Translationseffizienz als in der zweiten Zelle aufweist.

16. Verfahren zum Exprimieren eines Polypeptids von einem ersten Polynucleotid in einer Säugerzelle, wobei das Verfahren Folgendes umfasst:
- Auswählen einer iso-tRNA anhand der Eigenschaft, dass sie normalerweise in geringerer Menge in der Zelle vorhanden ist als andere iso-tRNAs und einem Codon des ersten Polynucleotids entspricht,
- Einbringen eines zweiten Polynucleotids, das die ausgewählte iso-tRNA codiert und funktionell an ein regulatorisches Polynucleotid geknüpft ist, in die Zelle und
- Exprimieren des zweiten Polynucleotids in der Zelle, wobei das Polypeptid in der Zelle exprimiert wird.

17. Verfahren zum Exprimieren eines Polypeptids von einem ersten Polynucleotid in einer Säugerzelle, wobei das Verfahren Folgendes umfasst:
- Auswählen einer iso-tRNA anhand der Eigenschaft, dass sie normalerweise in geringerer Menge in der Zelle vorhanden ist als andere iso-tRNAs und einem Codon des ersten Polynucleotids entspricht,
- Einbringen eines zweiten Polynucleotids, das die ausgewählte iso-tRNA codiert und funktionell an ein regulatorisches Polynucleotid geknüpft ist, in einen Vorläufer der Zelle, wobei der Vorläufer Bedingungen ausgesetzt wird, die für die Erzeugung der Zelle ausreichen, und
- Exprimieren des zweiten Polynucleotids in der Zelle, wobei das Polypeptid in der Zelle exprimiert wird.

18. Verfahren zur Erzeugung eines Viruspartikels in einer zyklisierenden Eukaryontenzelle, wobei das Virusteilchen ein Polypeptid umfasst, das für den Zusammenbau des Virusteilchens notwendig ist, und wobei das Polypeptid in der Zelle von einem Eltern-Polynucleotid exprimiert wird, aber nicht in ausreichender Menge, um einen Viruszusammenbau darin zu ermöglichen, wobei das Verfahren Folgendes umfasst:
- Bereitstellen eines Vergleichs von Translationseffizienzen einzelner Codons in der Zelle,
- Auswählen eines ersten Codons des Eltern-Polynucleotids aus dem Vergleich zum Ersetzen mit einem synonymen Codon, wobei das synonyme Codon anhand der Eigenschaft ausgewählt wird, dass es eine höhere Translationseffizienz in der Zelle aufweist als das erste Codon, und
- Ersetzen des ersten Codons mit dem synonymen Codon zur Konstruktion eines synthetischen Polynucleotids, das verbesserte Translationskinetiken im Vergleich zu dem Eltern-Polynucleotid aufweist, sodass das Polypeptid von dem synthetischen Polynucleotid in der Zelle in einer Menge exprimierbar ist, die einen Viruszusammenbau darin ermöglicht, und
- Einbringen des synthetischen Polynucleotids, das funktionell an ein regulatorisches Polynucleotid geknüpft ist, in die Zelle,
wobei das Polypeptid exprimiert wird und das Virusteilchen in der Zelle erzeugt wird.

19. Verfahren nach Anspruch 18, wobei das synonyme Codon eine höhere Translationseffizienz in der Zelle aufweist als das erste Codon.

20. Verfahren zur Erzeugung eines Virusteilchens in einer zyklisierenden Zelle, wobei das Virusteilchen wenigstens ein Polypeptid umfasst, das für einen Zusammenbau des Virusteilchens notwendig ist, wobei das Polypeptid in der Zelle von einem ersten Polynucleotid exprimiert wird, aber nicht in einer Menge, die ausreichend ist, um einen Viruszusammenbau darin zu ermöglichen, wobei das Verfahren Folgendes umfasst:
- Bereitstellen eines Vergleichs von Translationseffizienzen einzelner Codons in der Zelle,
- Auswählen eines ersten Codons des ersten Polynucleotids aus dem Vergleich, das eine geringere Translationseffizienz in der Zelle aufweist als ein synonymes Codon,
- Einbringen eines zweiten Polynucleotids, das eine für das erste Codon spezifische iso-tRNA codiert, in die Zelle und
- Exprimieren des zweiten Polynucleotids in der Zelle, wobei das Virusteilchen in der zyklisierenden Zelle erzeugt wird.

21. Verfahren zur Erzeugung eines Virusteilchens in einer zyklisierenden Eukaryontenzelle, wobei das Virusteilchen ein Polypeptid umfasst, das notwendig für einen Zusammenbau des Virusteilchens ist, und wobei das Polypeptid in der Zelle von einem Eltern-Polynucleotid exprimiert wird, aber nicht in einer Menge, die ausreichend ist, um einen Viruszusammenbau darin zu ermöglichen, wobei das Verfahren Folgendes umfasst:
- Bereitstellen eines Vergleichs von Translationseffizienzen einzelner Codons in der Zelle,
- Auswählen eines ersten Codons des Eltern-Polynucleotids zum Ersetzen mit einem synonymen Codon aus dem Vergleich, wobei das synonyme Codon anhand der Eigenschaft ausgewählt wird, dass es eine höhere Translationseffizienz in der Zelle aufweist als das erste Codon, und
- Ersetzen des ersten Codons mit dem synonymen Codon zur Konstruktion eines synthetischen Polynucleotids, das gesteigerte Translationskinetiken im Vergleich zu dem Eltern-Polynucleotid aufweist, sodass das Polypeptid von dem synthetischen Polynucleotid in der Zelle in einer Menge exprimierbar ist, die ausreicht, um einen Viruszusammenbau darin zu ermöglichen,
- Einbringen des synthetischen Polynucleotids, das funktional an ein regulatorisches Polynucleotid geknüpft ist, in einen Vorläufer der Zelle und
- den Vorläufer Bedingungen aussetzen, die zur Erzeugung der Zelle ausreichen, wobei die Zelle das Polypeptid, das für einen Zusammenbau des Virusteilchens notwendig ist, umfasst,
wobei das Polypeptid exprimiert wird und das Virusteilchen in der Zelle erzeugt wird.

22. Verfahren zur Konstruktion eines synthetischen Polynucleotids, das ein Polypeptid codiert, wobei das Verfahren Folgendes umfasst:
- Auswählen eines ersten Codons eines Eltern-Polynucleotids zum Ersetzen mit einem synonymen Codon, wobei das synonyme Codon anhand der Eigenschaft ausgewählt wird, dass es in einer ersten Zelle eines Säugers eine geringere Translationseffizienz als in einer zweiten Zelle des Säugers aufweist, und
- Ersetzen des ersten Codons mit dem synonymen Codon zur Konstruktion des synthetischen Polynucleotids.

23. Verfahren nach Anspruch 22, wobei das erste Codon und das synonyme Codon ausgewählt werden durch:
- Vergleichen von Translationseffizienzen einzelner Codons in der ersten Zelle im Verhältnis zu der zweiten Zelle und
- Auswählen des ersten Codons und des synonymen Codons basierend auf dem Vergleich.

24. Verfahren nach Anspruch 23, wobei die Translationseffizienz eines einzelnen Codons durch Messen der Häufigkeit einer iso-tRNA, die dem einzelnen Codon entspricht, in der ersten Zelle im Verhältnis zu der zweiten Zelle verglichen wird.

25. Verfahren nach Anspruch 24, wobei das synonyme Codon einer iso-tRNA entspricht, die in geringerer Häufigkeit in der ersten Zelle im Verhältnis zu der zweiten Zelle vorhanden ist.

26. Verfahren nach Anspruch 23, wobei das Auswählen des ersten Codons und des synonymen Codons Folgendes umfasst:
- Messen von Häufigkeiten unterschiedlicher iso-tRNAs in der ersten Zelle im Verhältnis zu der zweiten Zelle und
- Auswählen des ersten Codons und des synonymen Codons basierend auf der Messung, wobei das synonyme Codon einer iso-tRNA entspricht, die in der ersten Zelle in geringerer Häufigkeit als in der zweiten Zelle vorhanden ist.

27. Verfahren zur Konstruktion eines synthetischen Polynucleotids, das ein Polypeptid codiert, wobei das Verfahren Folgendes umfasst:
- Bereitstellen eines Vergleichs von Translationseffizienzen einzelner Codons in einer Zelle eines Säugers,
- Auswählen eines ersten Codons des Eltern-Nucleotids aus dem Vergleich zum Ersetzen mit einem synonymen Codon, wobei das synonyme Codon anhand der Eigenschaft ausgewählt wird, dass es eine höhere Translationseffizienz in der Zelle aufweist als das erste Codon, und
- Ersetzen des ersten Codons mit dem synonyme Codon zum Konstruieren des synthetischen Polynucleotids.

28. Verfahren nach Anspruch 27, wobei das synonyme Codon einer iso-tRNA entspricht, die in größerer Häufigkeit in der Zelle vorhanden ist im Verhältnis zu der iso-tRNA, die dem ersten Codon entspricht.

29. Verfahren nach Anspruch 27, wobei das erste Codon und das synonyme Codon so ausgewählt werden, dass das Polypeptid in der Zelle von dem synthetischen Polynucleotid in einer Menge exprimiert wird, die wenigstens 110 % der Menge entspricht, in welcher das Polypeptid von dem Eltern-Polynucleotid in der Zelle exprimiert wird.

30. Verfahren nach Anspruch 27, wobei der Vergleich durch Vergleichen von Translationseffizienzen einzelner Codons in der Zelle bereitgestellt wird.

31. Verfahren nach Anspruch 27, wobei die Translationseffizienzen durch Messen der Häufigkeit von unterschiedlichen iso-tRNAs in der Zelle verglichen werden.

32. Verfahren zum Konstruieren eines synthetischen Polynucleotids, das ein Polypeptid codiert, wobei das Verfahren Folgendes umfasst:
- Bereitstellen eines Vergleichs von Translationseffizienzen einzelner Codons in einer Zelle eines Säugers,
- Auswählen eines ersten Codons des Eltern-Polynucleotids zum Ersetzen mit einem synonymen Codon aus dem Vergleich, wobei das synonyme Codon anhand der Eigenschaft ausgewählt wird, dass es eine geringere Translationseffizienz in der Zelle aufweist als das erste Codon, und
- Ersetzen des ersten Codons mit dem synonymen Codon zur Konstruktion des synthetischen Polynucleotids.

33. Verfahren nach Anspruch 31, wobei das synonyme Codon einer iso-tRNA entspricht, die in geringerer Häufigkeit in der Zelle vorhanden ist im Verhältnis zu der iso-tRNA, die dem ersten Codon entspricht.

34. Verfahren nach Anspruch 31, wobei der Vergleich durch Vergleichen von Translationseffizienzen einzelner Codons in der Zelle bereitgestellt wird.

35. Verfahren nach Anspruch 34, wobei die Translationseffizienzen durch Messen von Häufigkeiten unterschiedlicher iso-tRNAs in der Zelle verglichen werden.

## Revendications

1. Procédé de construction d'un polynucléotide synthétique qui code pour un polypeptide, le procédé comprenant :
- la sélection d'un premier codon d'un polynucléotide parent pour remplacement par un codon synonyme, le codon synonyme étant choisi en se basant sur le fait qu'il présente une efficacité traductionnelle supérieure dans une première cellule d'un mammifère par rapport à une deuxième cellule du mammifère ; et
- le remplacement du premier codon par le codon synonyme pour construire le polynucléotide synthétique.

2. Procédé selon la revendication 1, dans lequel le premier codon et le codon synonyme sont choisis en :
- comparant les efficacités traductionnelles des codons individuels dans la première cellule par rapport à la deuxième cellule ;
et
- choisissant le premier codon et le codon synonyme sur la base de la comparaison.

3. Procédé selon la revendication 2, dans lequel l'efficacité traductionnelle d'un codon individuel est comparée en mesurant l'abondance d'un iso-ARNt correspondant au codon individuel dans la première cellule par rapport à la deuxième cellule.

4. Procédé selon la revendication 3, dans lequel le codon synonyme correspond à un iso-ARNt qui est plus abondant dans la première cellule par rapport à la deuxième cellule.

5. Procédé selon la revendication 3, dans lequel la sélection du premier codon et du codon synonyme comprend :
- la mesure de l'abondance de différents iso-ARNt dans la première cellule par rapport à la deuxième cellule ; et
- la sélection du premier codon et du codon synonyme sur la base de la mesure, le codon synonyme correspondant à un iso-ARNt qui est plus abondant dans la première cellule que dans la deuxième cellule.

6. Procédé selon la revendication 3, dans lequel le codon synonyme correspond à un iso-ARNt qui est présent dans la première cellule à un niveau qui est d'au moins 110 % du niveau de l'iso-ARNt qui est présent dans la deuxième cellule.

7. Procédé selon la revendication 1, dans lequel le codon synonyme est choisi dans le groupe constitué de (1) un codon utilisé à une fréquence relativement élevée par des gènes de la première cellule, (2) un codon utilisé à une fréquence relativement élevée par des gènes du mammifère, (3) un codon utilisé à une fréquence relativement faible par des gènes de la deuxième cellule, et (4) un codon utilisé à une fréquence relativement faible par des gènes d'un organisme autre que le mammifère.

8. Procédé selon la revendication 1, dans lequel le premier codon et le codon synonyme sont choisis de telle sorte que le polypeptide soit exprimé à partir du polynucléotide synthétique dans la première cellule à un niveau qui est d'au moins 110 % du niveau auquel le polypeptide est exprimé à partir du polynucléotide parent dans la première cellule.

9. Procédé selon la revendication 1, dans lequel la deuxième cellule est une cellule précurseur de la première cellule.

10. Procédé selon la revendication 1, dans lequel la deuxième cellule est une cellule dérivée de la première cellule.

11. Procédé selon la revendication 1, dans lequel le polypeptide n'est pas substantiellement exprimable dans la deuxième cellule.

12. Procédé selon la revendication 1, dans lequel la première cellule est du même type que la deuxième cellule mais est à un stade de différentiation différent.

13. Procédé selon la revendication 1, dans lequel la première cellule est du même type que la deuxième cellule mais est à un stade différent du cycle cellulaire.

14. Procédé d'expression sélective d'un polypeptide dans une première cellule d'un mammifère, le procédé comprenant :
- la sélection d'un premier codon d'un polynucléotide parent codant pour le polypeptide pour remplacement par un codon synonyme, le codon synonyme étant choisi en se basant sur le fait qu'il présente une efficacité traductionnelle supérieure dans une première cellule d'un mammifère par rapport à une deuxième cellule du mammifère ; et
- le remplacement du premier codon par le codon synonyme pour construire un polynucléotide synthétique ; et
- l'introduction du polynucléotide synthétique dans une cellule choisie dans le groupe constitué de la première cellule et l'un précurseur de la première cellule, le polynucléotide synthétique étant lié fonctionnellement à un polynucléotide régulateur, le polypeptide étant exprimé sélectivement dans la première cellule.

15. Procédé selon la revendication 14, dans lequel le codon synonyme a une efficacité traductionnelle supérieure dans la première cellule par rapport à la deuxième cellule.

16. Procédé d'expression d'un polypeptide d'un premier polynucléotide dans une cellule de mammifère, le procédé comprenant :
- la sélection d'un iso-ARNt en se basant sur le fait qu'il est normalement moins abondant dans la cellule que les autres iso-ARNt et qu'il correspond à un codon du premier polynucléotide ;
- l'introduction dans la cellule d'un deuxième polynucléotide qui code pour l'iso-ARNt choisi et qui est lié fonctionnellement à un polynucléotide régulateur ; et
- l'expression du deuxième polynucléotide dans la cellule, le polypeptide étant exprimé dans la cellule.

17. Procédé d'expression d'un polypeptide d'un premier polynucléotide dans une cellule de mammifère, le procédé comprenant
- la sélection d'un iso-ARNt en se basant sur le fait qu'il est normalement moins abondant dans la cellule que les autres iso-ARNt et correspond à un codon du premier polynucléotide ;
- l'introduction dans un précurseur de la cellule d'un deuxième polynucléotide qui code pour l'iso-ARNt choisi et qui est lié fonctionnellement à un polynucléotide régulateur, le précurseur étant exposé à des conditions suffisantes pour produire la cellule ; et
- l'expression du deuxième polynucléotide dans la cellule, le polypeptide étant exprimé dans la cellule.

18. Procédé de production d'une particule virale dans une cellule eucaryote de cyclage, la particule virale comprenant un polypeptide nécessaire pour l'assemblage de la particule virale et dans lequel le polypeptide est exprimé dans la cellule à partir d'un polynucléotide parent mais non à un niveau suffisant pour permettre l'assemblage du virus dans celui-ci, le procédé comprenant :
- la fourniture d'une comparaison des efficacités traductionnelles des codons individuels dans la cellule ;
- la sélection de la comparaison d'un premier codon du polynucléotide parent pour remplacement par un codon synonyme, le codon synonyme étant choisi en se basant sur le fait qu'il présente une efficacité traductionnelle supérieure dans la cellule par rapport au premier codon ; et
- le remplacement du premier codon par le codon synonyme pour construire un polynucléotide synthétique ayant une cinétique traductionnelle améliorée par rapport au polynucléotide parent de telle sorte que le polypeptide puisse être exprimé à partir du polynucléotide synthétique dans la cellule à un niveau suffisant pour permettre l'assemblage du virus dans celui-ci ; et
- l'introduction dans la cellule du polynucléotide synthétique lié fonctionnellement à un polynucléotide régulateur,
le polypeptide étant exprimé et la particule virale étant produite dans la cellule.

19. Procédé selon la revendication 18, dans lequel le codon synonyme a une efficacité traductionnelle supérieure dans la cellule par rapport au premier codon.

20. Procédé de production d'une particule virale dans une cellule en cours de cycle, dans lequel la particule virale comprend au moins un polypeptide nécessaire pour l'assemblage de la particule virale, le polypeptide étant exprimé dans la cellule à partir d'un premier polynucléotide mais non à un niveau suffisant pour permettre un assemblage viral dans celui-ci, le procédé comprenant :
- la fourniture d'une comparaison des efficacités traductionnelle des codons individuels dans la cellule ;
- la sélection de la comparaison d'un premier codon du premier polynucloétide qui présente une efficacité traductionnelle inférieure dans la cellule par rapport à un codon synonyme ;
- l'introduction dans la cellule d'un deuxième polynucléotide codant pour un iso-ARNt spécifique du premier codon ; et
- l'expression du deuxième polynucléotide dans la cellule, la particule virale étant produite dans la cellule en cours de cycle.

21. Procédé de production d'une particule virale dans une cellule eucaryote en cours de cycle, la particule virale comprenant un polypeptide nécessaire pour l'assemblage de la particule virale et dans lequel le polypeptide est exprimé dans la cellule à partir d'un polynucléotide parent mais non à un niveau suffisant pour permettre l'assemblage viral dans celui-ci, le procédé comprenant :
- la fourniture d'une comparaison des efficacités traductionnelles des codons individuels dans la cellule ;
- la sélection de la comparaison d'un premier codon du polynucléotide parent pour remplacement par un codon synonyme, le codon synonyme étant choisi en se basant sur le fait qu'il présente une efficacité traductionnelle supérieure dans la cellule par rapport au premier codon : et
- le remplacement du premier codon par le codon synonyme pour construire un polynucléotide synthétique ayant une cinétique traductionnelle améliorée par rapport au polynucléotide parent de telle sorte que le polypeptide puisse être exprimé à partir du polynycléotide synthétique dans la cellule à un niveau suffisant pour permettre un assemblage viral dans celui-ci ;
- l'introduction dans un précurseur de la cellule du polynucléotide synthétique lié fonctionnellement à un polynucléotide régulateur ; et
- l'exposition du précurseur à des conditions suffisantes pour produire la cellule, la cellule comprenant le polypeptide nécessaire pour l'assemblage de la particule virale, le polypeptide étant exprimé et la particule virale étant produite dans la cellule.

22. Procédé de construction d'un polynucléotide synthétique qui code pour un polypeptide, le procédé comprenant :
- la sélection d'un premier codon d'un polynucléotide parent pour remplacement par un codon synonyme, le codon synonyme étant choisi en se basant sur le fait qu'il présente une efficacité traductionnelle inférieure dans une première cellule d'un mammifère par rapport à une deuxième cellule du mammifère ; et
- le remplacement du premier codon par le codon synonyme pour construire le polynucléotide synthétique.

23. Procédé selon la revendication 22, dans lequel le premier codon est le codon synonyme sont choisis par :
- comparaison des efficacités traductionnelles des codons individuels dans la première cellule par rapport à la deuxième cellule ;
et
- sélection du premier codon et du codon synonyme sur la base de la comparaison.

24. Procédé selon la revendication 23, dans lequel l'efficacité traductionnelle d'un codon individuel est comparée en mesurant l'abondance d'un iso-ARNt correspondant au codon individuel dans la première cellule par rapport à la deuxième cellule.

25. Procédé selon la revendication 24, dans lequel le codon synonyme correspond à un iso-ARNt qui est moins abondant dans la première cellule que dans la deuxième cellule.

26. Procédé selon la revendication 23, dans lequel la sélection du premier codon et du codon synonyme comprend :
- la mesure de l'abondance de différents iso-ARNt dans la première cellule par rapport à la deuxième cellule ; et
- la sélection du premier codon et du codon synonyme sur la base de la mesure, le codon synonyme correspondant à un iso-ARNt qui est moins abondant dans la première cellule que dans la deuxième cellule.

27. Procédé de construction d'un polynucléotide synthétique qui code pour un polypeptide, le procédé comprenant :
- la fourniture d'une comparaison des efficacités traductionnelles des codons individuels dans une cellule d'un mammifère ;
- la sélection de la comparaison d'un premier codon du polynucléotide parent pour remplacement par un codon synonyme, le codon synonyme étant choisi en se basant sur le fait qu'il présente une efficacité traductionnelle supérieure dans la cellule par rapport au premier codon ; et
- le remplacement du premier codon par le codon synonyme pour construire le polynucléotide synthétique.

28. Procédé selon la revendication 27, dans lequel le codon synonyme correspond à un iso-ARNt qui est plus abondant dans la cellule que l'iso-ARNt correspondant au premier codon.

29. Procédé selon la revendication 27, dans lequel le premier codon et le codon synonyme sont choisis de telle sorte que le polypeptide est exprimé à partir du polynucléotide synthétique dans la cellule à un niveau qui est d'au moins 110 % du niveau auquel le polypeptide est exprimé à partir du polynucléotide parent dans la cellule.

30. Procédé selon la revendication 27, dans lequel la comparaison est assurée en comparant les efficacités traductionnelles des codons individuels dans la cellule.

31. Procédé selon la revendication 27, dans lequel les efficacités traductionnelles sont comparées en mesurant l'abondance d'iso-ARNt différents dans la cellule.

32. Procédé de construction d'un polynucléotide synthétique codant pour un polypeptide, le procédé comprenant :
- la fourniture d'une comparaison des efficacités traductionnelles de codons individuels dans une cellule d'un mammifère ;
- la sélection de la comparaison d'un premier codon du polynucléotide parent pour remplacement par un codon synonyme, le codon synonyme étant choisi en se basant sur le fait qu'il présente une efficacité traductionnelle inférieure dans la cellule par rapport au premier codon ; et
- le remplacement du premier codon par le codon synonyme pour construire le polynucléotide synthétique.

33. Procédé selon la revendication 31, dans lequel le codon synonyme correspond à un iso-ARNt qui est moins abondant dans la cellule que l'iso-ARNt correspondant au premier codon.

34. Procédé selon la revendication 31, dans lequel la comparaison est assurée en comparant les efficacités traductionnelles des codons individuels dans la cellule.

35. Procédé selon la revendication 34, dans lequel les efficacités traductionnelles sont comparées en mesurant l'abondance de différents iso-ARNt différents dans la cellule.
